# EUROPEAN PATENT APPLICATION

(11) **EP 4 317 157 A1**
(43) Date of publication of application: **07.02.2024**
(21) Application number: 22774324.2
(22) Date of filing: 24.03.2022
(51) Int. Cl.: C07D 471/04, A61K 31/519, A61P 35/00

(54) **6-CARBAMATE SUBSTITUTED HETEROARYL RING DERIVATIVES**

(30) Priority: 26.03.2021 CN 202110327907; 20.01.2022 CN 202210068496
(71) Applicant: Medshine Discovery Inc., Nanjing, Jiangsu 210032 (CN)
(72) Inventor: WU, Lingyun, Shanghai 200131 (CN); DAI, Tianzi, Shanghai 200131 (CN); ZHAO, Lele, Shanghai 200131 (CN); SUN, Jianjun, Shanghai 200131 (CN); QIAN, Yi, Shanghai 200131 (CN); LI, Jian, Shanghai 200131 (CN); CHEN, Shuhui, Shanghai 200131 (CN)
(74) Representative: Dehns
(86) International application number: PCT/CN2022/082862
(87) International publication number: WO 2022/199670

(57) **Abstract**

Disclosed are 6-carbamate substituted heteroaryl ring derivatives and a preparation method therefor. The present application particularly relates to a compound as shown in formula (II) and a pharmaceutically acceptable salt thereof.

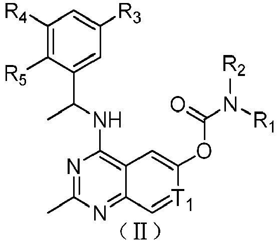

## Description

The present application claims the right of the following priorities:
CN202110327907.9, March 26, 2021;
CN202210068496.0, January 20, 2022.

### TECHNICAL FIELD

The present disclosure relates to a new class of 6-carbamate-substituted heteroaromatic ring derivatives and a preparation method therefor, in particular to a compound of formula (II) and a pharmaceutically acceptable salt thereof.

### BACKGROUND

RAS protein is a guanine nucleoside-binding protein with guanosine triphosphatase (GTPase) activity, mainly including three subtypes, KRAS, NRAS, and HRAS. As a binary molecular switch for GDP/GTP cycling control, RAS protein can cycle between an active GTP-bound state (GTP-RAS) and an inactive GDP-bound state (GDP-RAS). This cycle has important regulatory functions in cells and is closely related to cell proliferation, survival, metabolism, migration, immunity, and growth.

Studies have shown that RAS gene mutations exist in about 15% of tumor cases. Oncogenic RAS mutations can simultaneously inhibit intrinsic GTPase activity and GAP-activated GTPase activity, so that the RAS cycle is always in the "on" state of RAS-GTP, resulting in continuous activation of downstream signaling pathways to cause cancer. Here, about 85% of RAS mutations are concentrated in KRAS (Andrew et al., Cancer Cell, 2014, 25:272-281). Therefore, inhibiting the mutant of KRAS and abnormal activation of downstream pathway has become one of the popular targets in cancer treatment.

SOS1 (Son of Sevenless 1) is a class of GEFs that regulate the GDP/GTP cycle of RAS protein. After the cell surface receptor is activated and binds to intracellular Grb2, Grb2 recruits SOS1 to the cell membrane, and then SOS1 catalyzes RAS-GDP/GTP exchange, thereby activating downstream signaling pathways. The small molecule SOS1 inhibitor bound to the catalytic site can block the binding of SOS1 and RAS protein, thereby effectively reducing the abnormal activation of the downstream signaling pathway of RAS in cancer cells and playing a role in the treatment of cancer. At present, for SOS 1 small molecule inhibitors, only BI-1701963 (WO2018115380, WO2019122129) developed by Boehringer Ingelheim, has entered phase I clinical trials. The SOS1 inhibitor developed by Bayer (WO2018172250, WO2019201848) is still in the preclinical research stage. In recent years, studies have suggested that drugs in the RAS pathway are prone to drug resistance in clinical application, and part of the reason for drug resistance is that the inhibition of ERK phosphorylation will activate the upstream RAS pathway by negative feedback. This negative feedback regulation mechanism is closely related to SOS1. Therefore, the development of SOS1 small molecule inhibitors has broad application prospects.

AMG-510 is a potent, orally bioavailable, and selective KRAS G12C covalent inhibitor developed by Amgen for the treatment of locally advanced or metastatic non-small cell lung cancer harboring a KRAS G12C mutation. Its structure is as follows:

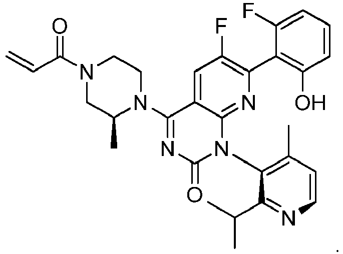

### CONTENT OF THE PRESENT INVENTION

The present disclosure provides a compound of formula (II) or a pharmaceutically acceptable salt thereof, 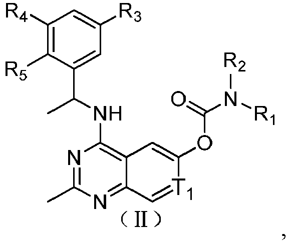 wherein
R₁ and R₂ are each independently selected from H, C₁₋₆ alkyl, C₃₋₁₂ cycloalkyl, 3- to 12-membered heterocycloalkyl, C₆₋₁₀ aryl, and 5- to 10-membered heteroaryl, wherein the C₁₋₆ alkyl, C₃₋₁₂ cycloalkyl, 3- to 12-membered heterocycloalkyl, C₆₋₁₀ aryl, and 5- to 10-membered heteroaryl are each independently and optionally substituted by 1, 2, 3, or 4 Rₐ;
or, R₁, R₂ together with the nitrogen atom to which they are attached form a 3- to 12-membered heterocycloalkyl ring, wherein the 3- to 12-membered heterocycloalkyl ring is optionally substituted by 1, 2, 3, or 4 R_{b};
R₃ is selected from H, F, Cl, Br, I, -OH, -NH₂, -CN, C₁₋₆ alkyl, C₁₋₆ alkoxy, and C₁₋₆ alkylamino, wherein the C₁₋₆ alkyl, C₁₋₆ alkoxy, and C₁₋₆ alkylamino are each independently and optionally substituted by 1, 2, 3, or 4 R_{c};
R₄ is selected from H, F, Cl, Br, I, -OH, -NH₂, -CN, C₁₋₆ alkyl, C₁₋₆ alkoxy, C₁₋₆ alkylamino, -C(=O)-O-C₁₋₆ alkyl, C₃₋₁₀ cycloalkyl, and 3- to 10-membered heterocycloalkyl, wherein the C₁₋₆ alkyl, C₁₋₆ alkoxy, C₁₋₆ alkylamino, -C(=O)-O-C₁₋₆ alkyl, C₃₋₁₀ cycloalkyl, and 3- to 10-membered heterocycloalkyl are each independently and optionally substituted by 1, 2, 3, or 4 R_{c};
R₅ is selected from H, F, Cl, Br, I, -OH, -NH₂, -CN, C₁₋₆ alkyl, C₁₋₆ alkoxy, and C₁₋₆ alkylamino, wherein the C₁₋₆ alkyl, C₁₋₆ alkoxy, and C₁₋₆ alkylamino are each independently and optionally substituted by 1, 2, 3, or 4 R_{c};
or, R₄, R₅ together with the carbon atom to which they are attached form 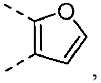 wherein 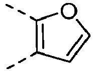 is optionally substituted by 1, 2, 3, or 4 R_{d};
T₁ is selected from CR₆ and N;
R₆ is selected from -OCH₃, -CN, and -S(=O)₂-CH₃;
each Rₐ is independently selected from F, Cl, Br, I, -OH, -NH₂, -CN, =O, C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl, wherein the C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl are each independently and optionally substituted by 1, 2, 3, or 4 R;
each R_{b} is independently selected from F, Cl, Br, I, -OH, -NH₂, -CN, =O, C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl, wherein the C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl are each independently and optionally substituted by 1, 2, 3, or 4 R;
each R_{c} is independently selected from F, Cl, Br, I, -OH, -NH₂, -CN, -COOH, =O, - C(=O)H, -C(=O)-NH₂, C₁₋₃ alkyl, C₁₋₃ alkoxy, and 5- to 6-membered heterocycloalkyl, wherein the C₁₋₃ alkyl, C₁₋₃ alkoxy, and 5- to 6-membered heterocycloalkyl are each independently and optionally substituted by 1, 2, 3, or 4 R;
each R_{d} is independently selected from F, Cl, Br, I, -OH, -NH₂, -CN, -COOH, =O, - C(=O)H, -C(=O)-NH₂, and C₁₋₃ alkyl;
each R is independently selected from F, Cl, Br, I, -OH, -NH₂, -CN, -COOH, =O, - C(=O)H, -C(=O)-NH₂, and C₁₋₃ alkyl;
"hetero" in the 5- to 6-membered heterocycloalkyl, 3- to 10-membered heterocycloalkyl, 3- to 12-membered heterocycloalkyl, 5- to 10-membered heteroaryl, and -O-5- to 10-membered heteroaryl represents 1, 2, 3, or 4 heteroatoms or heteroatom groups each independently selected from -O-, -NH-, -S-, and -N-.

The present disclosure provides a compound of formula (I-2) or a pharmaceutically acceptable salt thereof, 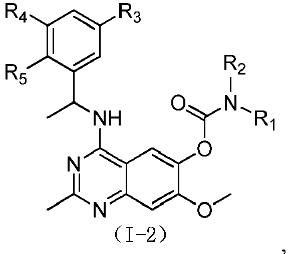 wherein
R₁, R₂ together with the nitrogen atom to which they are attached form a 6- to 10-membered heterocycloalkyl ring, wherein the 6- to 10-membered heterocycloalkyl ring is optionally substituted by 1, 2, 3, or 4 R_{b};
R₃ is selected from H, F, Cl, Br, I, -OH, and -NH₂;
R₄ is selected from H, F, Cl, Br, I, -OH, -NH₂, -CN, and C₁₋₄ alkyl, wherein the C₁₋₄ alkyl is optionally substituted by 1, 2, 3, or 4 R_{c};
R₅ is selected from H, F, Cl, Br, I, and C₁₋₃ alkyl, wherein the C₁₋₃ alkyl is optionally substituted by 1, 2, 3, or 4 R_{c};
or, R₄, R₅ together with the carbon atom to which they are attached form 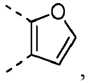 wherein 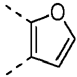 is optionally substituted by 1, 2, 3, or 4 R_{d};
each R_{b} is independently selected from F, Cl, Br, I, -OH, -NH₂, -CN, =O, C₁₋₃ alkyl, C₁₋₃ alkylamino, and 4- to 5-membered heterocycloalkyl, wherein the C₁₋₃ alkyl, C₁₋₃ alkylamino, and 4- to 5-membered heterocycloalkyl are each independently and optionally substituted by 1, 2, 3, or 4 R;
each R_{c} is independently selected from F, Cl, Br, I, -OH, -NH₂, -CN, -COOH, -C(=O)H, -C(=O)-NH₂, and 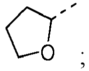
each R_{d} is independently selected from F, Cl, Br, I, -OH, -NH₂, -CN, -COOH, - C(=O)H, and -C(=O)-NH₂;
"hetero" in the 6- to 10-membered heterocycloalkyl and 4- to 5-membered heterocycloalkyl represents 1, 2, 3, or 4 heteroatoms or heteroatom groups each independently selected from -O-, -NH-, -S-, and -N-.

In some embodiments of the present disclosure, the compound has a structure of formula (II-1): 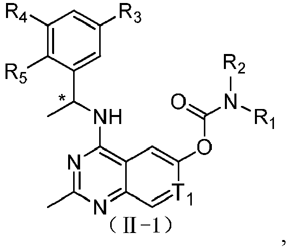 wherein
T₁, R₁, R₂, R₃, R₄, and R₅ are as defined in the present disclosure;
the carbon atom with "*" is a chiral carbon atom, which exists in an (*R*) or (*S*) single enantiomer form or an (*R*) or (*S*) single enantiomer-rich form.

In some embodiments of the present disclosure, each R is independently selected from F, Cl, Br, and =O, and other variables are as defined in the present disclosure.

In some embodiments of the present disclosure, each Rₐ is independently selected from F, Cl, Br, -OH, -NH₂, -CN, C₁₋₃ alkyl, C₁₋₃ alkoxy, C₁₋₃ alkylamino, and -C(=O)-O-C₁₋₃ alkyl, wherein the C₁₋₃ alkyl, C₁₋₃ alkoxy, C₁₋₃ alkylamino, and -C(=O)-O-C₁₋₃ alkyl are each independently and optionally substituted by 1, 2, 3, or 4 R, and R and other variables are as defined in the present disclosure.

In some embodiments of the present disclosure, each Rₐ is independently selected from F, Cl, Br, -OH, -NH₂, -CN, -CH₃, and -OCH₃, wherein the -CH₃ and -OCH₃ are each independently and optionally substituted by 1, 2, 3, or 4 R, and R and other variables are as defined in the present disclosure.

In some embodiments of the present disclosure, the R₁ and R₂ are each independently selected from H and C₁₋₃ alkyl, wherein the C₁₋₃ alkyl is optionally substituted by 1, 2, 3, or 4 Rₐ, and Rₐ and other variables are as defined in the present disclosure.

In some embodiments of the present disclosure, each R_{b} is independently selected from F, Cl, Br, I, -OH, -NH₂, -CN, =O, C₁₋₃ alkyl, C₁₋₃ alkylamino, and 3- to 6-membered heterocycloalkyl, wherein the C₁₋₃ alkyl, C₁₋₃ alkylamino, and 3- to 6-membered heterocycloalkyl are each independently and optionally substituted by 1, 2, 3, or 4 R, and R and other variables are as defined in the present disclosure.

In some embodiments of the present disclosure, each R_{b} is independently selected from F, Cl, Br, I, -OH, -NH₂, -CN, =O, C₁₋₃ alkyl, C₁₋₃ alkylamino, and 4-membered heterocycloalkyl, wherein the C₁₋₃ alkyl, C₁₋₃ alkylamino, and 4-membered heterocycloalkyl are each independently and optionally substituted by 1, 2, 3, or 4 R, and R and other variables are as defined in the present disclosure.

In some embodiments of the present disclosure, each R_{b} is independently selected from F, Cl, Br, -NH₂, -CH₃, -CH₂-CH₃, 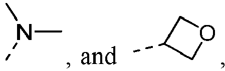 wherein the -CH₃, -CH₂-CH₃, 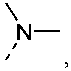 and 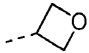 are each independently and optionally substituted by 1, 2, 3, or 4 R, and R and other variables are as defined in the present disclosure.

In some embodiments of the present disclosure, each R_{b} is independently selected from F, Cl, Br, -NH₂, -CH₃, -CH₂-CH₃, 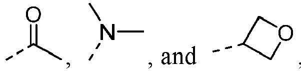 , and other variables are as defined in the present disclosure.

In some embodiments of the present disclosure, each R_{c} is independently selected from F, Cl, Br, -OH, -OCH₃, and 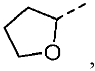 and other variables are as defined in the present disclosure.

In some embodiments of the present disclosure, each R_{c} is independently selected from F, Cl, Br, I, -OH, -NH₂, -CN, -COOH, =O, -C(=O)H, -C(=O)-NH₂, C₁₋₃ alkyl, C₁₋₃ alkoxy, and 5-membered heterocycloalkyl, wherein the C₁₋₃ alkyl, C₁₋₃ alkoxy, and 5-membered heterocycloalkyl are each independently and optionally substituted by 1, 2, 3, or 4 R, and R and other variables are as defined in the present disclosure.

In some embodiments of the present disclosure, each R_{c} is independently selected from F, -OH, and 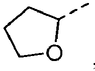 , and other variables are as defined in the present disclosure.

In some embodiments of the present disclosure, each R_{d} is independently selected from F, Cl, and Br, and other variables are as defined in the present disclosure.

In some embodiments of the present disclosure, each R_{d} is independently selected from F, and other variables are as defined in the present disclosure.

In some embodiments of the present disclosure, the R₃ is selected from H, F, Cl, Br, and -NH₂, and other variables are as defined in the present disclosure.

In some embodiments of the present disclosure, the R₃ is selected from H and -NH₂, and other variables are as defined in the present disclosure.

In some embodiments of the present disclosure, the R₄ is selected from H, -CN, and C₁₋₄ alkyl, wherein the C₁₋₄ alkyl is optionally substituted by 1, 2, 3, or 4 R_{c}, and R_{c} and other variables are as defined in the present disclosure.

In some embodiments of the present disclosure, the R₄ is selected from H, F, Cl, Br, - CN, -CH₃, -CH₂CH₃, -CH(CH₃)₂, and -CH₂CH(CH₃)₂, wherein the -CH₃, -CH₂CH₃, - CH(CH₃)₂, and -CH₂CH(CH₃)₂ are each independently and optionally substituted by 1, 2, 3, or 4 R_{c}, and R_{c} and other variables are as defined in the present disclosure.

In some embodiments of the present disclosure, the R₄ is selected from H, F, Cl, Br, - CN, 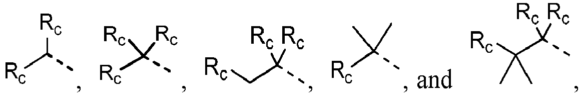 and R_{c} and other variables are as defined in the present disclosure.

In some embodiments of the present disclosure, the R₄ is selected from H, F, Cl, Br, - CN, 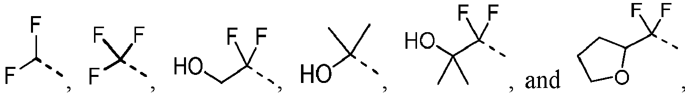 , and other variables are as defined in the present disclosure.

In some embodiments of the present disclosure, the R₅ is selected from H, F, Cl, Br, and -CH₃, and other variables are as defined in the present disclosure.

In some embodiments of the present disclosure, the R₅ is selected from H, F, and -CH₃, and other variables are as defined in the present disclosure.

In some embodiments of the present disclosure, the R₆ is selected from -OCH₃, and other variables are as defined in the present disclosure.

In some embodiments of the present disclosure, the m is selected from 0, 1, and 2, and other variables are as defined in the present disclosure.

In some embodiments of the present disclosure, the R₁, R₂ together with the nitrogen atom to which they are attached form a 5- to 11-membered heterocycloalkyl ring, wherein the 5- to 11-membered heterocycloalkyl ring is optionally substituted by 1, 2, 3, or 4 R_{b}, and R_{b} and other variables are as defined in the present disclosure.

In some embodiments of the present disclosure, the structural moiety 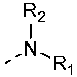 is selected from 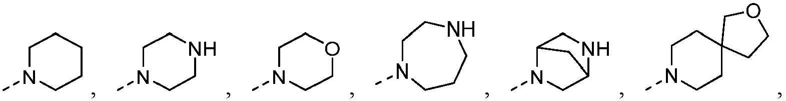 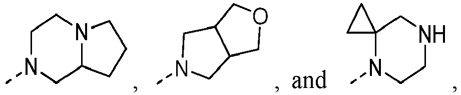 wherein the 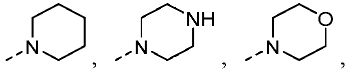 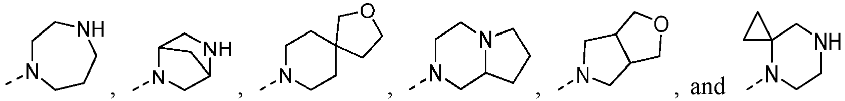 are each independently and optionally substituted by 1, 2, 3, or 4 R_{b}, and R_{b} and other variables are as defined in the present disclosure.

In some embodiments of the present disclosure, the structural moiety 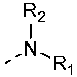 is selected from 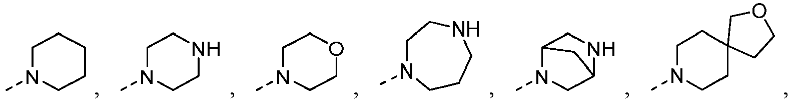 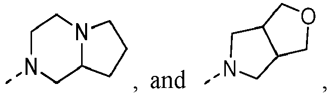 wherein the 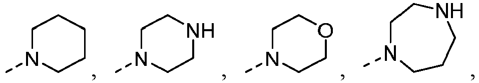 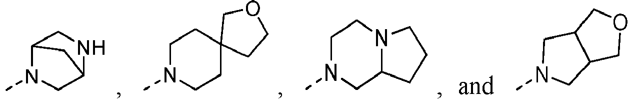 are each independently and optionally substituted by 1, 2, 3, or 4 R_{b}, and R_{b} and other variables are as defined in the present disclosure.

In some embodiments of the present disclosure, the structural moiety 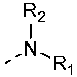 is selected from 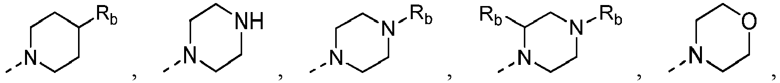 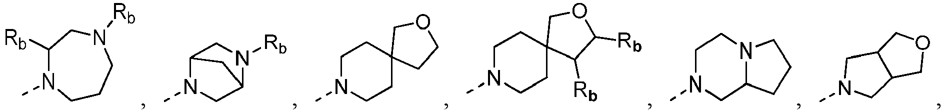 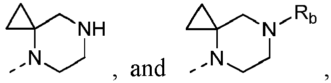 and R_{b} and other variables are as defined in the present disclosure.

In some embodiments of the present disclosure, the structural moiety 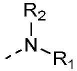 is selected from 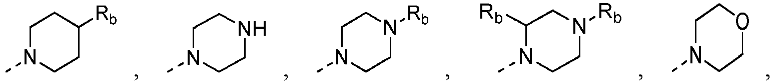 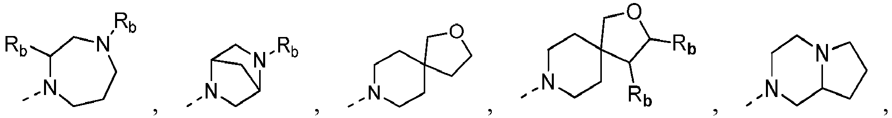 and 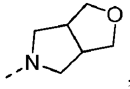 , and R_{b} and other variables are as defined in the present disclosure.

In some embodiments of the present disclosure, the structural moiety 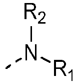 is selected from 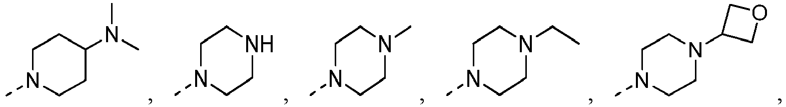 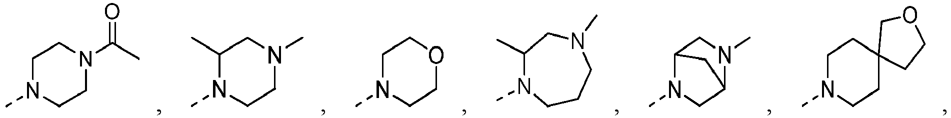 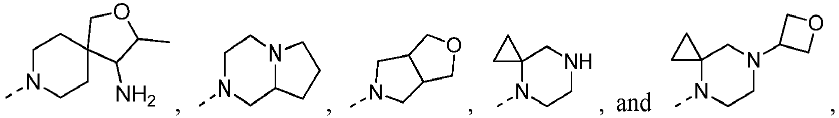 and other variables are as defined in the present disclosure.

In some embodiments of the present disclosure, the structural moiety 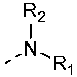 is selected from 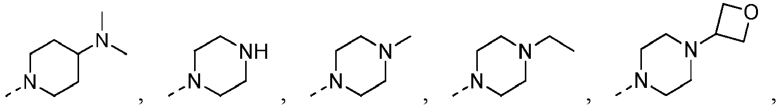 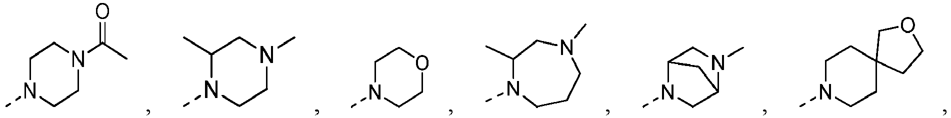 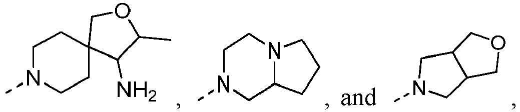 and other variables are as defined in the present disclosure.

In some embodiments of the present disclosure, the structural moiety 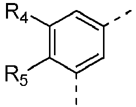 is selected from 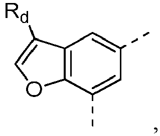 and other variables are as defined in the present disclosure.

In some embodiments of the present disclosure, the structural moiety 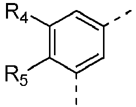 is selected from 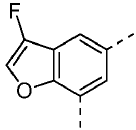 , and other variables are as defined in the present disclosure.

In some embodiments of the present disclosure, the compound has a structure of formula (II-2) or (II-3): 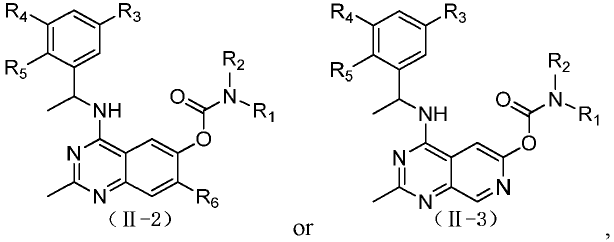 wherein R₁, R₂, R₃, R₄, R₅, and R₆ are as defined in the present disclosure.

In some embodiments of the present disclosure, the compound has a structure of formula (I-2): 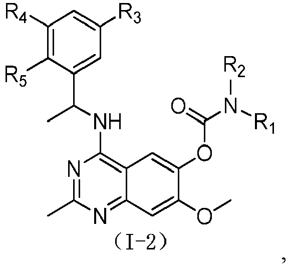 wherein R₁, R₂, R₃, R₄, and R₅ are as defined in the present disclosure.

In some embodiments of the present disclosure, the compound has a structure of formula (I-3): 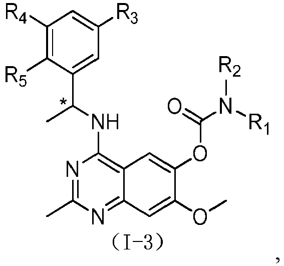
wherein R₁, R₂, R₃, R₄, and R₅ are as defined in the present disclosure;
the carbon atom with "*" is a chiral carbon atom, which exists in an (R) or (5) single enantiomer form or an (*R*) or (*S*) single enantiomer-rich form.

In some embodiments of the present disclosure, the compound has a structure of formula (I-4), (I-5), or (I-6): 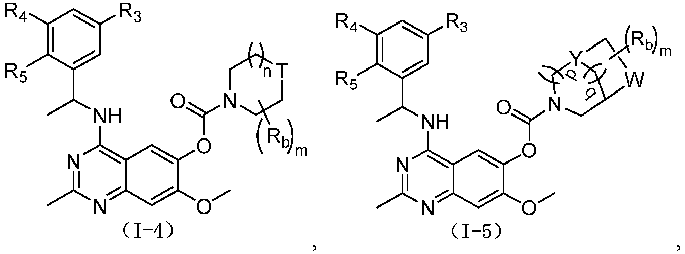 or 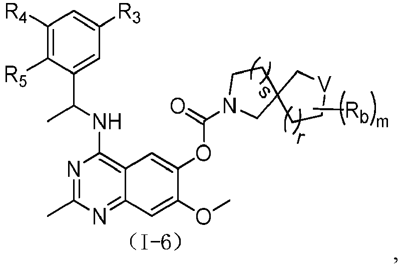 wherein
T and V are each independently selected from CH₂, NH, and O;
m is selected from 0, 1, 2, 3, and 4;
n, p, q, r, and s are each independently selected from 0, 1, and 2;
and p + q ≤ 3;
W is selected from NH, -CH₂-CH₂-, and -O-CH₂-;
Y is selected from N and CH;
R₃, R₄, R₅, and R_{b} are as defined in the present disclosure.

In some embodiments of the present disclosure, the compound has a structure of formula (III): 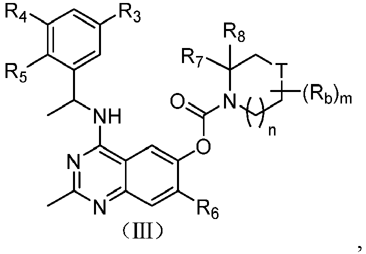 wherein
T is selected from CH₂, NH, and O;
R_{b} is selected from F, Cl, Br, I, -OH, -NH₂, -CN, =O, C₁₋₃ alkyl, C₁₋₃ alkylamino, and 3- to 6-membered heterocycloalkyl, wherein the C₁₋₃ alkyl, C₁₋₃ alkylamino, and 3- to 6-membered heterocycloalkyl are each independently and optionally substituted by 1, 2, 3, or 4 R;
R₆ is selected from -OCH₃, -CN, and -S(=O)₂-CH₃;
R₇ and R₈ are each independently selected from H, F, Cl, Br, I, -OH, -NH₂, -CN, =O, and C₁₋₃ alkyl;
or, R₇, R₈ together with the carbon atom to which they are attached form a C₃₋₄ cycloalkyl ring;
m is selected from 0, 1, 2, 3, and 4;
n is selected from 0, 1 and 2;
R, R₃, R₄, and R₅ are as defined in the present disclosure.

In some embodiments of the present disclosure, the compound has a structure of formula (III-1): 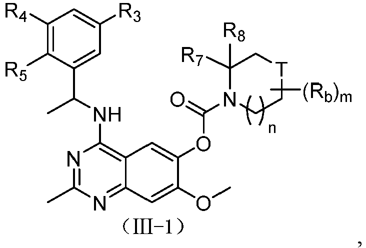 wherein
T is selected from CH₂, NH, and O;
R_{b} is selected from F, Cl, Br, I, -OH, -NH₂, -CN, =O, C₁₋₃ alkyl, C₁₋₃ alkylamino, and 3- to 6-membered heterocycloalkyl, wherein the C₁₋₃ alkyl, C₁₋₃ alkylamino, and 3- to 6-membered heterocycloalkyl are each independently and optionally substituted by 1, 2, 3, or 4 R;
R₇ and R₈ are each independently selected from H, F, Cl, Br, I, -OH, -NH₂, -CN, =O, and C₁₋₃ alkyl;
or, R₇, R₈ together with the carbon atom to which they are attached form a C₃₋₄ cycloalkyl ring;
m is selected from 0, 1, 2, 3, and 4;
n is selected from 0, 1 and 2;
R, R₃, R₄, and R₅ are as defined in the present disclosure.

In some embodiments of the present disclosure, the compound has a structure of formula (III-2) or (III-3): 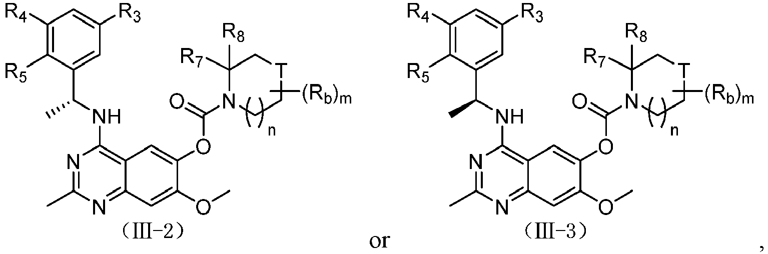 wherein
T, m, n, R_{b}, R₃, R₄, R₅, R₇, and R₈ are as defined in the present disclosure.

In some embodiments of the present disclosure, the compound has a structure of formula (III-4): 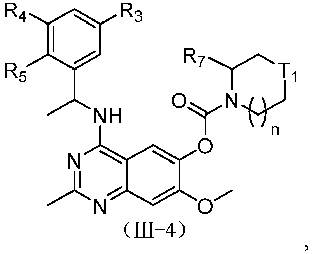 wherein
T₁ is selected from CHR_{b}, NH, NR_{b}, and O;
R_{b} is selected from F, Cl, Br, I, -OH, -NH₂, -CN, =O, C₁₋₃ alkyl, C₁₋₃ alkylamino, and 4- to 5-membered heterocycloalkyl, wherein the C₁₋₃ alkyl, C₁₋₃ alkylamino, and 4- to 5-membered heterocycloalkyl are each independently and optionally substituted by 1, 2, 3, or 4 R;
R₇ is selected from H, F, Cl, Br, I, -OH, -NH₂, -CN, =O, and C₁₋₃ alkyl;
n is selected from 1 and 2;
R, R₃, R₄, and R₅ are as defined in the present disclosure.

In some embodiments of the present disclosure, the compound has a structure of formula (III-5) or (III-6): 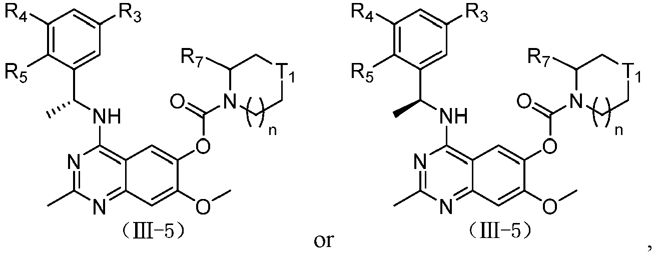 wherein T₁, n, R₃, R₄, R₅, and R₇ are as defined in the present disclosure.

In some embodiments of the present disclosure, the R₇ and R₈ are each independently selected from H, F, Cl, Br, I, -OH, -NH₂, -CN, -CH₃, and -CH₂-CH₃, and other variables are as defined in the present disclosure.

In some embodiments of the present disclosure, the R₇ and R₈ are each independently selected from H and -CH₃, and other variables are as defined in the present disclosure.

In some embodiments of the present disclosure, the R₇ is selected from H, F, Cl, Br, I, -OH, -NH₂, -CN, -CH₃, and -CH₂-CH₃, and other variables are as defined in the present disclosure.

In some embodiments of the present disclosure, the R₇ is selected from H and -CH₃, and other variables are as defined in the present disclosure.

In some embodiments of the present disclosure, the R₈ is selected from H, and other variables are as defined in the present disclosure.

In some embodiments of the present disclosure, the R₇, R₈ together with the carbon atom they are attached form a cyclopropyl ring.

In some embodiments of the present disclosure, the compound or the pharmaceutically acceptable salt thereof is provided, wherein the compound has a structure of formula (I-7), (I-8), (I-9), or (III-1A): 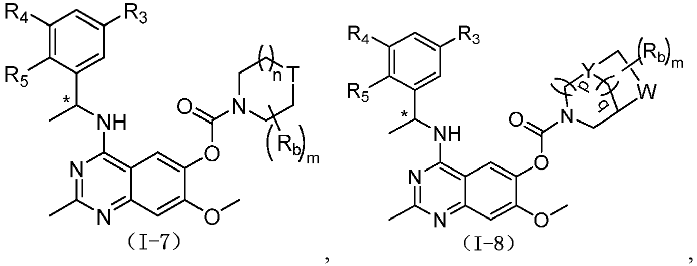 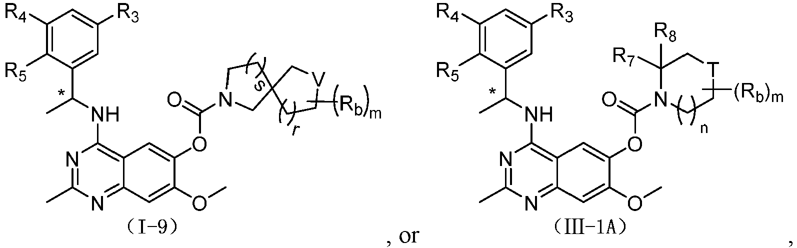
wherein T, V, W, Y, m, n, p, q, r, s, R₃, R₄, R₅, R_{b}, R₇, and R₈ are as defined in the present disclosure;
the carbon atom with "*" is a chiral carbon atom, which exists in an (*R*) or (*S*) single enantiomer form or an (*R*) or (*S*) single enantiomer-rich form.

In some embodiments of the present disclosure, the compound has a structure of formula (I-10), (I-11), or (I-12): 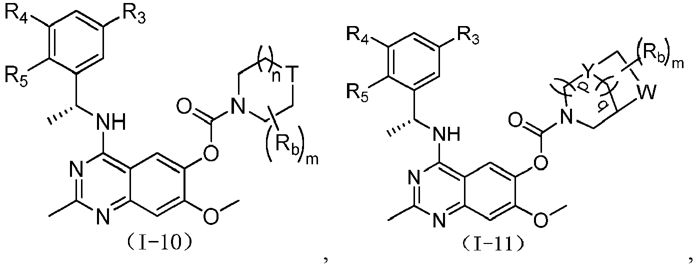 or 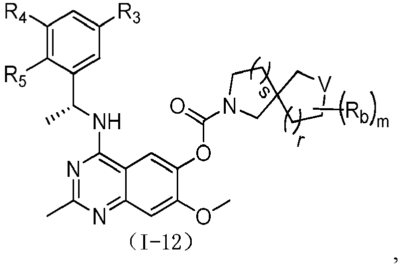 wherein T, V, W, Y, m, n, p, q, r, s, R₃, R₄, R₅, and R_{b} are as defined in the present disclosure.

There are still some embodiments of the present disclosure which are obtained by any combination of the above variables.

The present disclosure also provides a compound of the following formula or a pharmaceutically acceptable salt thereof, 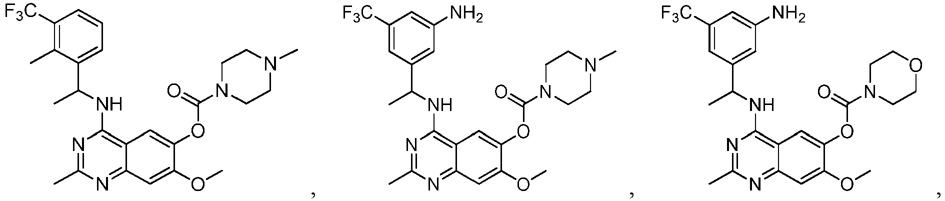 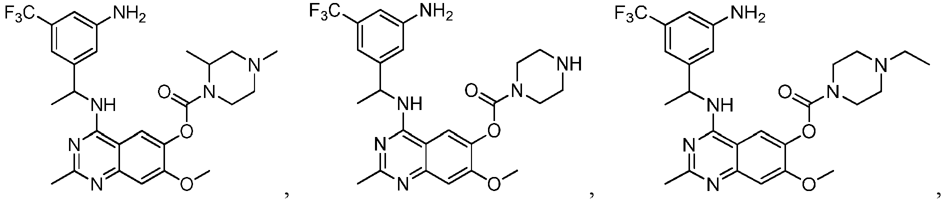 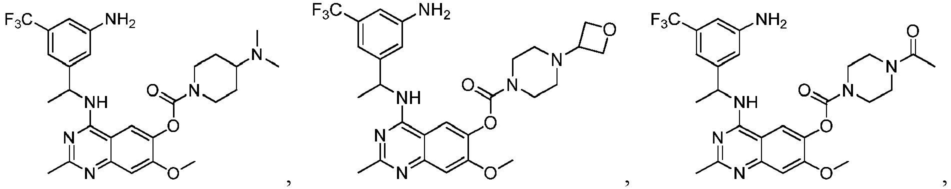 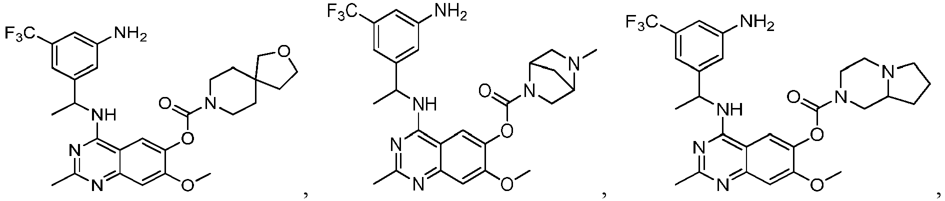 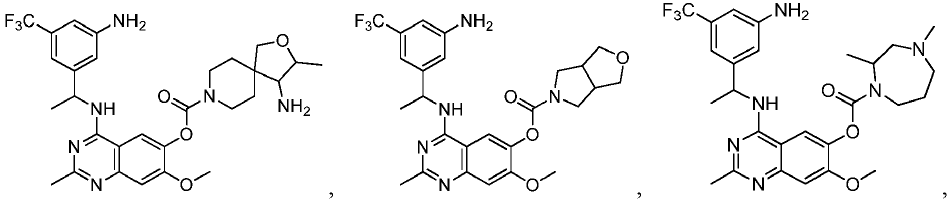 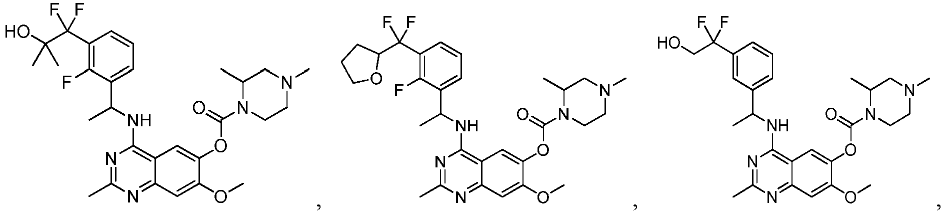 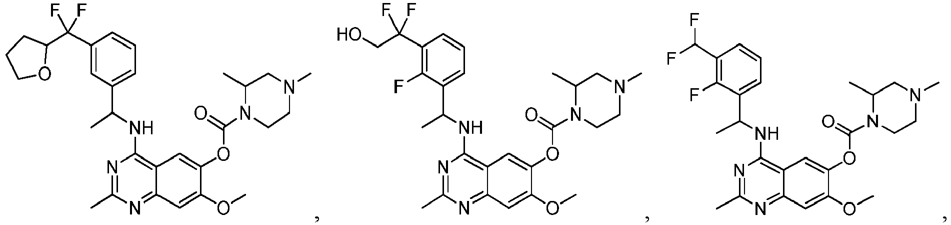 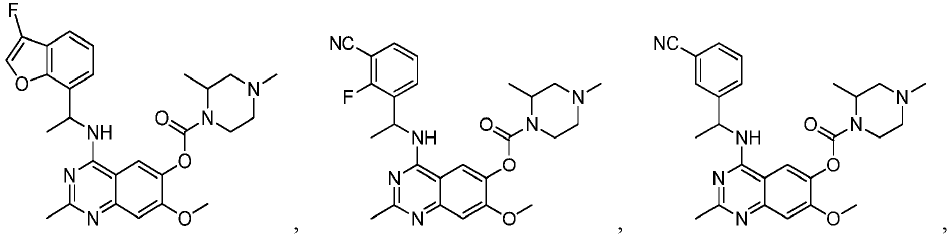 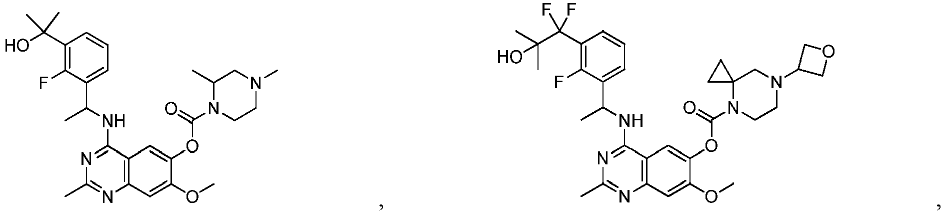 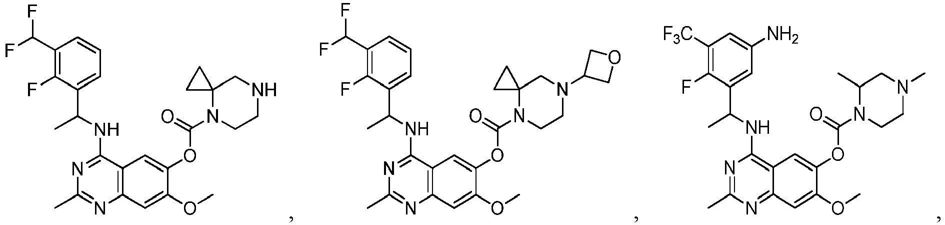 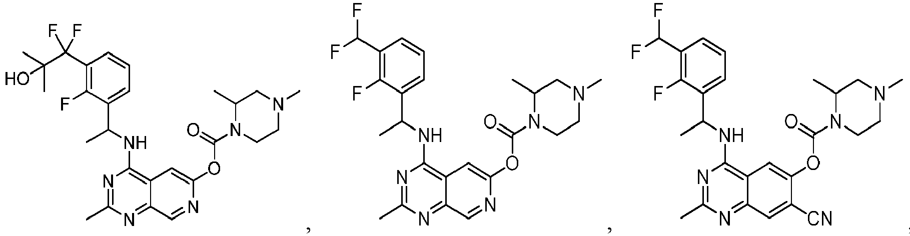 , or

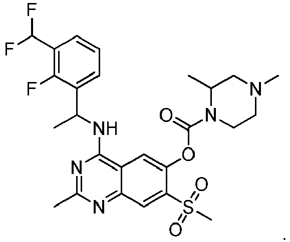

The present disclosure also provides a compound of the following formula or a pharmaceutically acceptable salt thereof,

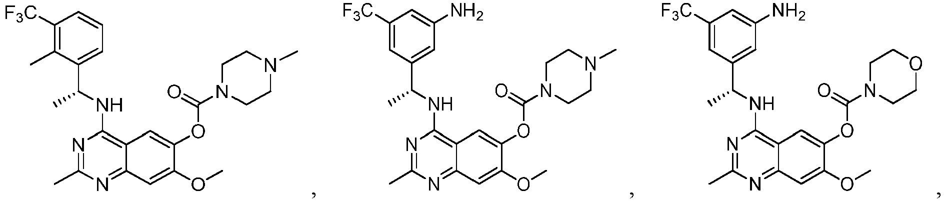

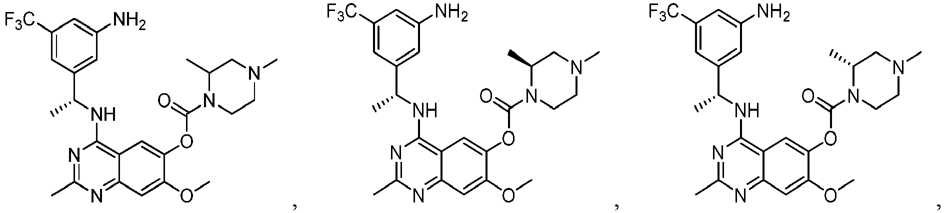

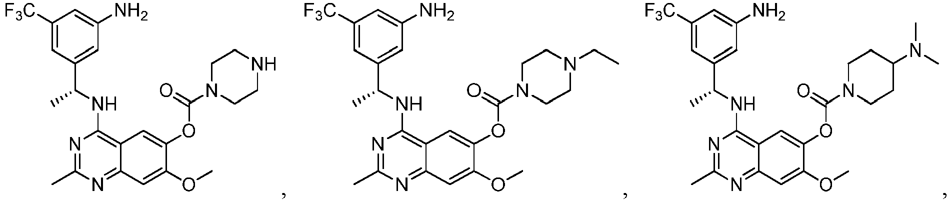

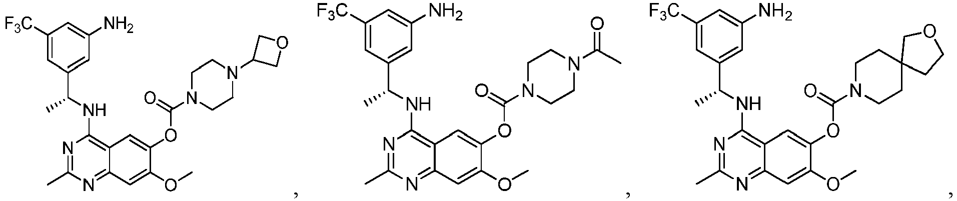

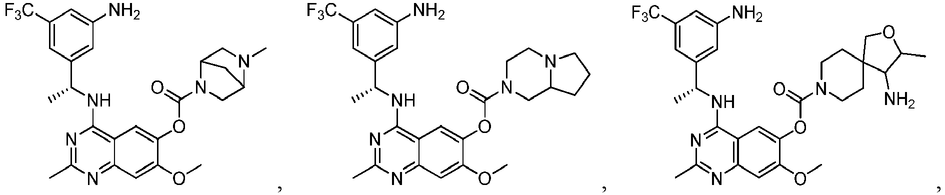

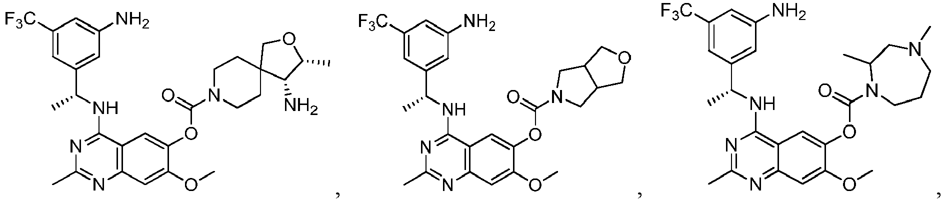

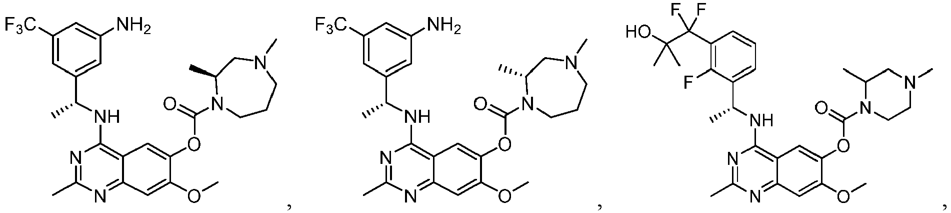

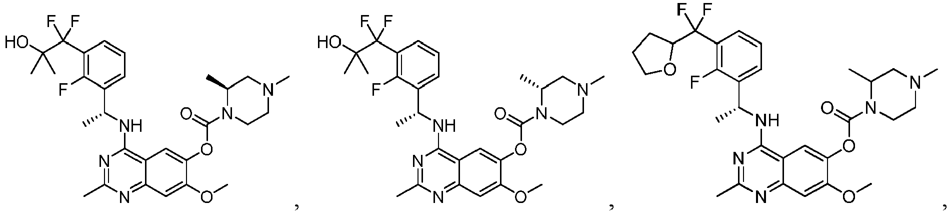

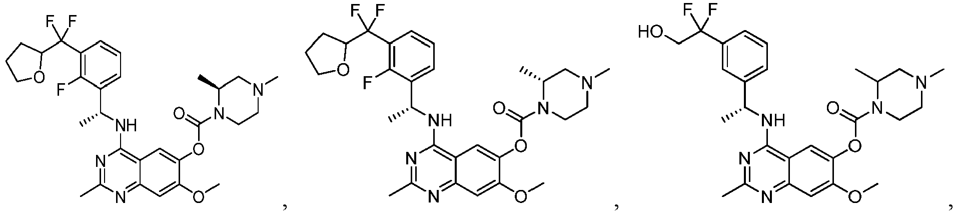

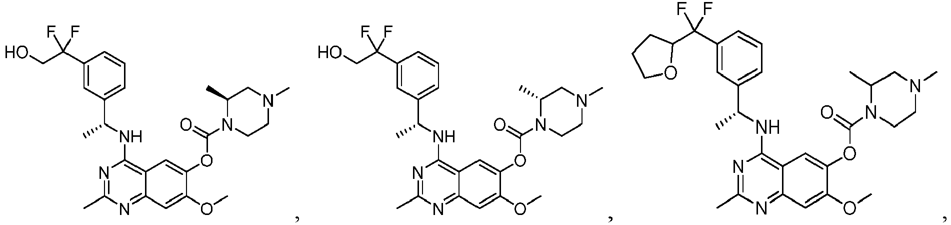

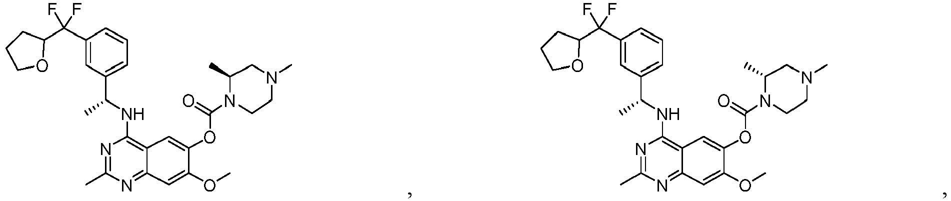

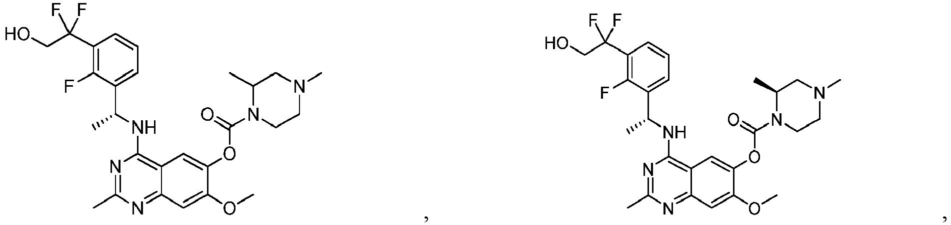

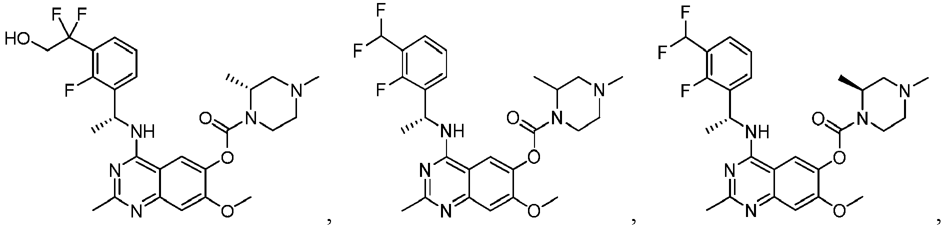

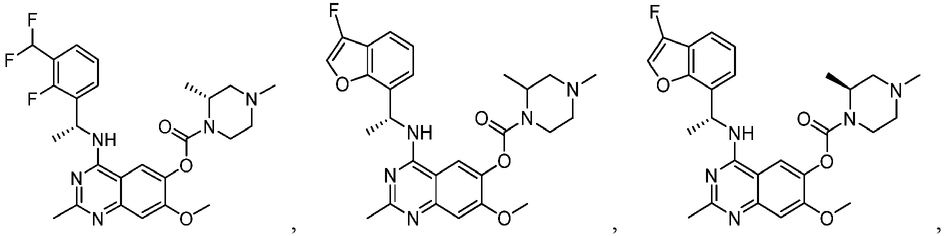

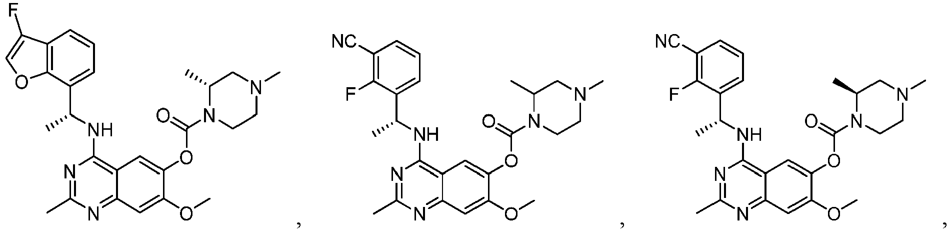

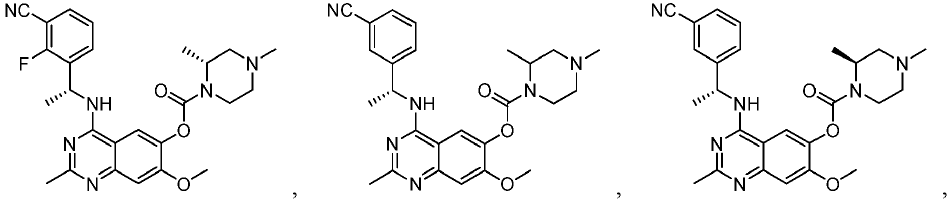

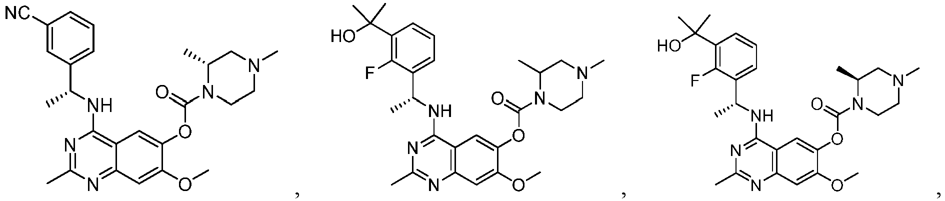

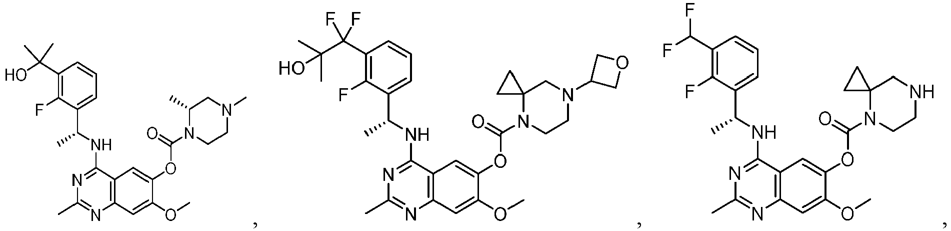

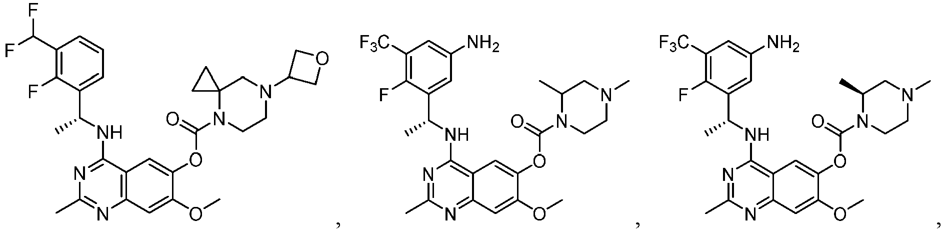

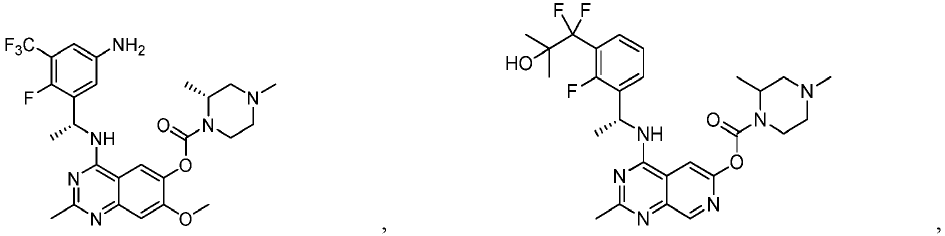

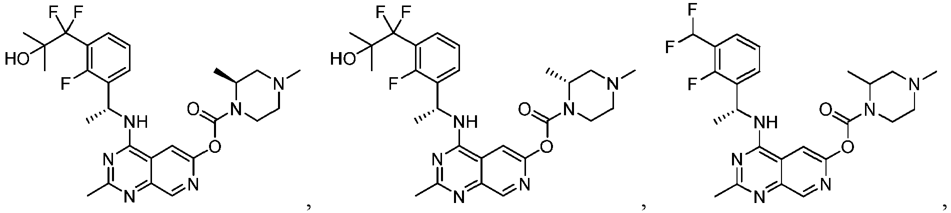

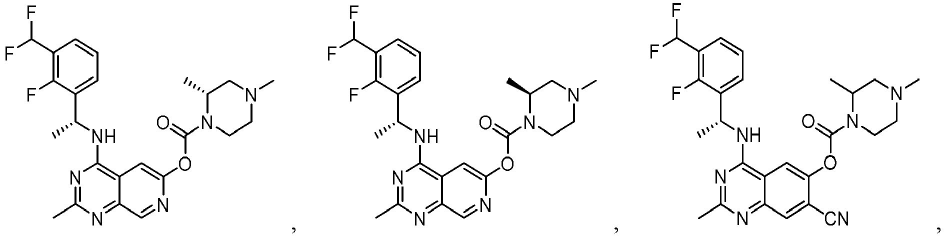

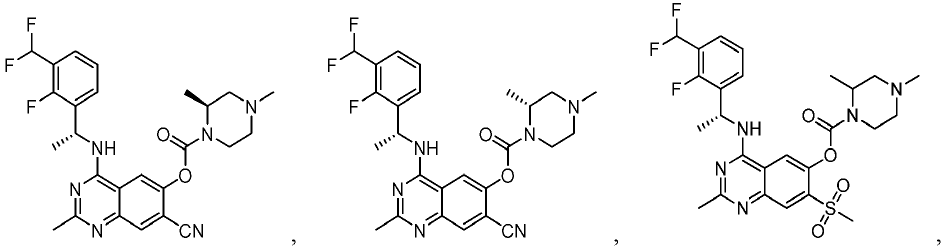

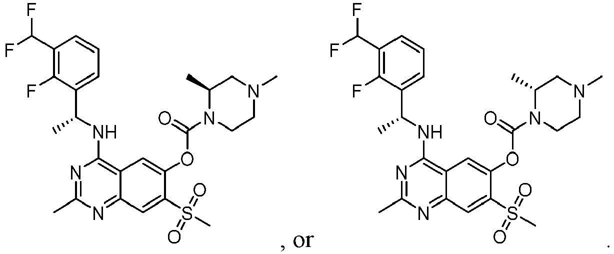

The present disclosure also provides the compound or the pharmaceutically acceptable salt thereof, wherein the pharmaceutically acceptable salt is hydrochloride.

The present disclosure also provides a use of the compound or the pharmaceutically acceptable salt thereof in the manufacture of a medicament for treating KRAS mutant solid tumor diseases.

### Technical effect

The compound of the present disclosure has good KRAS(G12C)-SOS1 binding inhibitory activity, as well as significant inhibitory activity against DLD-1 cells and KRAS(G12C) mutant H358 cells, and good pharmacokinetic properties, thereby obtaining excellent tumor growth inhibitory activity.

### Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( 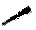 ) and a wedged dashed bond ( 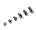 ), and the relative configuration of a stereogenic center is represented by a straight solid bond ( 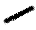 ) and a straight dashed bond ( 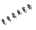 ), a wave line ( 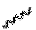 ) is used to represent a wedged solid bond ( 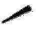 ) or a wedged dashed bond ( 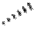 ), or the wave line ( 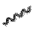 ) is used to represent a straight solid bond ( 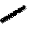 ) and a straight dashed bond ( 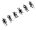 ).

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom that constitutes the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 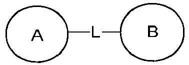 is -M-W-, then -M-W- can link ring A and ring B to form 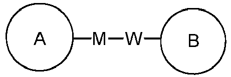 in the direction same as left-to-right reading order, and form 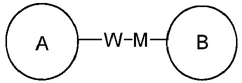 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of chemical bond linking thereto so as to meet the corresponding valence. The chemical bond between the site and other groups can be represented by a straight solid bond 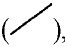 , a straight dashed bond 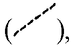 or a wavy line 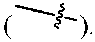 For example, the straight solid bond in -OCH₃ means that it is linked to other groups through the oxygen atom in the group; the straight dashed bonds in 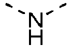 means that it is linked to other groups through the two ends of nitrogen atom in the group; the wave lines in 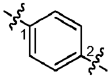 means that the phenyl group is linked to other groups through carbon atoms at position 1 and position 2. 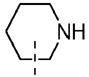 means that it can be linked to other groups through any linkable sites on the piperidinyl by one chemical bond, including at least four types of linkage, including 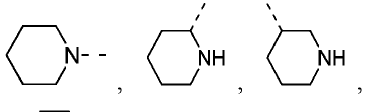 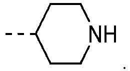 Even though the H atom is drawn on the -N-, 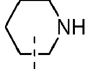 still includes the linkage of 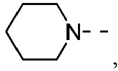 merely when one chemical bond was connected, the H of this site will be reduced by one to the corresponding monovalent piperidinyl.

When the chemical bond of a substituent intersects with the chemical bond between two atoms in the ring, it means that the substituent can form a bond with any atom in the ring. When the atom connected to a substituent is not specified, the substituent can form a bond with any atom. If the atom to which the substituent is connected is in a bicyclic or tricyclic system, it means that the substituent can form a bond with any atom of any ring in the system. Combinations of substituents and/or variables are allowable only if such combinations result in stable compounds. For example, the structural moiety 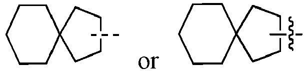 means that it can be substituted at any position on the cyclohexyl or cyclopentyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, C₅ alkyl, etc.; it can be monovalent (such as methyl), divalent (such as methylene), or multivalent (such as methine). Examples of C₁₋₆ alkyl include, but are not limited to, methyl (Me), ethyl (Et), propyl (including n-propyl and isopropyl), butyl (including *n*-butyl, isobutyl, *s*-butyl, and *t*-butyl), pentyl (including *n*-pentyl, isopentyl, and neopentyl), hexyl, etc.

Unless otherwise specified, the term "C₁₋₄ alkyl" refers to a linear or branched saturated hydrocarbon group consisting of 1 to 4 carbon atoms. The C₁₋₄ alkyl includes C₁₋₂, C₁₋₃, C₂₋₃ alkyl, etc.; it can be monovalent (such as methyl), divalent (such as methylene), or multivalent (such as methine). Examples of C₁₋₄ alkyl include, but are not limited to, methyl (Me), ethyl (Et), propyl (including *n*-propyl and isopropyl), butyl (including *n*-butyl, isobutyl, *s*-butyl, and *t*-butyl), etc.

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

Unless otherwise specified, the term "C₁₋₆ alkoxy" refers to an alkyl group containing 1 to 6 carbon atoms that are connected to the rest of the molecule through an oxygen atom. The C₁₋₆ alkoxy includes C₁₋₄, C₁₋₃, C₁₋₂, C₂₋₆, C₂₋₄, C₆, C₅, C₄, C₃ alkoxy, etc. Examples of C₁₋₆ alkoxy include, but are not limited to, methoxy, ethoxy, propoxy (including n-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, C₂ alkoxy, etc. Examples of C₁₋₃ alkoxy include, but are not limited to, methoxy, ethoxy, propoxy (including n-propoxy and isopropoxy), etc.

Unless otherwise specified, the term "C₁₋₆ alkylamino" refers to an alkyl group containing 1 to 6 carbon atoms that are connected to the rest of the molecule through an amino group. The C₁₋₆ alkylamino includes C₁₋₄, C₁₋₃, C₁₋₂, C₂₋₆, C₂₋₄, C₆, C₅, C₄, C₃, C₂ alkylamino, etc. Examples of C₁₋₆ alkylamino include, but are not limited to, -NHCH₃, -N(CH₃)₂, - NHCH₂CH₃, -N(CH₃)CH₂CH₃, -N(CH₂CH₃)(CH₂CH₃), -NHCH₂CH₂CH₃, -NHCH₂(CH₃)₂, - NHCH₂CH₂CH₂CH₃, etc.

Unless otherwise specified, the term "C₁₋₃ alkylamino" refers to an alkyl group containing 1 to 3 carbon atoms that are connected to the rest of the molecule through an amino group. The C₁₋₃ alkylamino includes C₁₋₂, C₃, C₂ alkylamino, etc. Examples of C₁₋₃ alkylamino include, but are not limited to, -NHCH₃, -N(CH₃)₂, -NHCH₂CH₃, -N(CH₃)CH₂CH₃, -NHCH₂CH₂CH₃, -NHCH₂(CH₃)₂, etc.

Unless otherwise specified, the heterocycloalkyl and heteroaryl refer to specific groups containing heteroatoms or heteroatom groups. The heteroatoms or heteroatom groups include, but are not limited to, N, O, S, NH, substituted or protected -N(H)-, -S(=O)-, -S(=O)₂-, -C(=O)-, -C(=S)-, C(=O)O-, -C(=O)N(H)-, -C(=NH)-, -S(=O)₂N(H)-, and -S(=O)N(H)-, wherein nitrogen and sulfur atoms are optionally oxidized and the nitrogen heteroatom is optionally quaternized. For the ring system, heteroatoms or heteroatom groups can be located within or outside the ring system (such as cyclopropylsulfonyl, cyclopropylacyl), wherein the so-called heterocycloalkyl and heteroaryl are connected to the rest of the molecule through carbon atoms, that is, the heteroatom can be located at any position of the group (except the position where the group is attached to the rest of the molecule); the so-called heterocycloalkyl and heteroaryl are connected to the rest of the molecule through heteroatoms, that is, the heteroatoms are located at the position where the group is attached to the rest of the molecule; the so-called heterocycloalkyl and heteroaryl are connected to the rest of the molecule through heteroatoms, wherein the heteroatoms can be located at any position of the group (including the position where the group is attached to the rest of the molecule).

Unless otherwise specified, "C₃₋₁₂ cycloalkyl" refers to a saturated cyclic hydrocarbon group consisting of 3 to 12 carbon atoms, which includes monocyclic, bicyclic, and tricyclic systems, wherein the bicyclic and tricyclic systems include a spiro ring, a fused ring, and a bridged ring. The C₃₋₁₂ cycloalkyl includes C₃₋₁₀, C₃₋₁₀, C₃₋₆, C₃₋₅, C₄₋₁₀, C₄₋₈, C₄₋₆, C₄₋₅, C₅₋₈, C₅₋₆ cycloalkyl, etc.; it can be monovalent, divalent, or multivalent. Examples of C₃₋₁₂ cycloalkyl include, but are not limited to, cyclopropyl, cyclobutyl, cyclopentyl, cyclohexyl, cycloheptyl, norbornyl, [2.2.2]bicyclooctyl, [4.4.0]bicyclodecyl, etc.

Unless otherwise specified, "C₃₋₁₀ cycloalkyl" refers to a saturated cyclic hydrocarbon group consisting of 3 to 10 carbon atoms, which includes monocyclic, bicyclic, and tricyclic systems, wherein the bicyclic and tricyclic systems include a spiro ring, a fused ring, and a bridged ring. The C₃₋₁₀ cycloalkyl includes C₃₋₈, C₃₋₆, C₃₋₅, C₄₋₁₀, C₄₋₈, C₄₋₆, C₄₋₅, C₅₋₈, C₅₋₆ cycloalkyl, etc.; it can be monovalent, divalent, or multivalent. Examples of C₃₋₁₀ cycloalkyl include, but are not limited to, cyclopropyl, cyclobutyl, cyclopentyl, cyclohexyl, cycloheptyl, norbornyl, [2.2.2]bicyclooctyl, [4.4.0]bicyclodecyl, etc.

Unless otherwise specified, "C₃₋₄ cycloalkyl" refers to a saturated cyclic hydrocarbon group consisting of 3 to 4 carbon atoms, which is a monocyclic system, and the C₃₋₅ cycloalkyl includes C₃, C₄ cycloalkyl, etc.; it can be monovalent, divalent, or multivalent. Examples of C₃₋₄ cycloalkyl include, but are not limited to, cyclopropyl, cyclobutyl, etc.

Unless otherwise specified, the term "3- to 12-membered heterocycloalkyl" by itself or in combination with other terms refers to a saturated cyclic group consisting of 3 to 12 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(O)ₚ, p is 1 or 2). The 3- to 12-membered heterocycloalkyl includes monocyclic, bicyclic, and tricyclic systems, wherein the bicyclic and tricyclic systems include a spiro ring, a fused ring, and a bridged ring. In addition, with regard to the "3- to 12-membered heterocycloalkyl", a heteroatom may occupy the connection position of the heterocycloalkyl with the rest of the molecule. The 3- to 12-membered heterocycloalkyl includes 3- to 10-membered, 3- to 8-membered, 3- to 6-membered, 3- to 5-membered, 4- to 6-membered, 5- to 6-membered, 4-membered, 5-membered, 6-membered heterocycloalkyl, etc. Examples of 3- to 12-membered heterocycloalkyl include, but are not limited to, aziridinyl, oxiranyl, thiiranyl, azetidinyl, oxetanyl, thietanyl, pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, etc.

Unless otherwise specified, the term "3- to 10-membered heterocycloalkyl" by itself or in combination with other terms refers to a saturated cyclic group consisting of 3 to 10 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(O)ₚ, p is 1 or 2). The 3- to 10-membered heterocycloalkyl includes monocyclic, bicyclic, and tricyclic systems, wherein the bicyclic and tricyclic systems include a spiro ring, a fused ring, and a bridged ring. In addition, with regard to the "3- to 10-membered heterocycloalkyl", a heteroatom may occupy the connection position of the heterocycloalkyl with the rest of the molecule. The 3- to 10-membered heterocycloalkyl includes 3- to 8-membered, 3- to 6-membered, 3- to 5-membered, 4- to 6-membered, 5- to 6-membered, 4-membered, 5-membered, 6-membered heterocycloalkyl, etc. Examples of 3- to 10-membered heterocycloalkyl include, but are not limited to, aziridinyl, oxiranyl, thiiranyl, azetidinyl, oxetanyl, thietanyl, pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, etc.

Unless otherwise specified, the term "5- to 11-membered heterocycloalkyl" by itself or in combination with other terms refers to a saturated cyclic group consisting of 5 to 11 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(O)ₚ, p is 1 or 2). The 5- to 11-membered heterocycloalkyl includes monocyclic, bicyclic, and tricyclic systems, wherein the bicyclic and tricyclic systems include a spiro ring, a fused ring, and a bridged ring. In addition, with regard to the "5- to 11-membered heterocycloalkyl", a heteroatom may occupy the connection position of the heterocycloalkyl with the rest of the molecule. The 5- to 11-membered heterocycloalkyl includes 5- to 6-membered, 5- to 7-membered, 5- to 8-membered, 5- to 9-membered, 5- to 10-membered, 5- to 11-membered, 6-to 7-membered, 6- to 8-membered, 6- to 9-membered, 6- to 10-membered, 6- to 11-membered, 5-membered, 6-membered, 7-membered heterocycloalkyl, etc. Examples of 5- to 11-membered heterocycloalkyl include, but are not limited to, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, etc.

Unless otherwise specified, the term "6- to 10-membered heterocycloalkyl" by itself or in combination with other terms refers to a saturated cyclic group consisting of 6 to 10 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(O)ₚ, p is 1 or 2). The 6- to 10-membered heterocycloalkyl includes monocyclic, bicyclic, and tricyclic systems, wherein the bicyclic and tricyclic systems include a spiro ring, a fused ring, and a bridged ring. In addition, with regard to the "6- to 10-membered heterocycloalkyl", a heteroatom may occupy the connection position of the heterocycloalkyl with the rest of the molecule. The 6- to 10-membered heterocycloalkyl includes 6-membered, 6- to 7-membered, 6- to 8-membered, 6- to 9-membered, 5- to 10-membered, 7-membered, 7- to 8-membered, 7-to 9-membered, 7- to 10-membered, 8-membered, 8- to 9-membered, 8- to 10-membered, 9-membered, 10-membered heterocycloalkyl, etc. Examples of 6- to 10-membered heterocycloalkyl include, but are not limited to, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, etc.

Unless otherwise specified, the term "3- to 6-membered heterocycloalkyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(O)ₚ, p is 1 or 2). It includes monocyclic and bicyclic systems, wherein the bicyclic systems include a spiro ring, a fused ring, and a bridged ring. In addition, with regard to the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, 6-membered heterocycloalkyl, etc. Examples of 3- to 6-membered heterocycloalkyl include, but are not limited to, aziridinyl, oxiranyl, thiiranyl, azetidinyl, oxetanyl, thietanyl, pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, etc.

Unless otherwise specified, the term "5- to 6-membered heterocycloalkyl" by itself or in combination with other terms refers to a saturated cyclic group consisting of 5 to 6 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(=O)ₚ, p is 1 or 2). It includes monocyclic and bicyclic systems, wherein the bicyclic systems include a spiro ring, a fused ring, and a bridged ring. In addition, with regard to the "5- to 6-membered heterocycloalkyl", a heteroatom may occupy the connection position of the heterocycloalkyl with the rest of the molecule. The 5- to 6-membered heterocycloalkyl includes 5-membered and 6-membered heterocycloalkyl. Examples of 5- to 6-membered heterocycloalkyl include, but are not limited to, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, etc.

Unless otherwise specified, the term "4- to 5-membered heterocycloalkyl" by itself or in combination with other terms refers to a saturated cyclic group consisting of 4 to 5 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(=O)ₚ, p is 1 or 2). In addition, with regard to the "4- to 5-membered heterocycloalkyl", a heteroatom may occupy the connection position of the heterocycloalkyl with the rest of the molecule. The 4- to 5-membered heterocycloalkyl includes 4-membered and 5-membered heterocycloalkyl. Examples of 4- to 5-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), etc.

Unless otherwise specified, the term "4-membered heterocycloalkyl" by itself or in combination with other terms refers to a saturated cyclic group consisting of 4 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e., C(=O), NO, and S(=O)ₚ, p is 1 or 2). In addition, with regard to the "4-membered heterocycloalkyl", a heteroatom may occupy the connection position of the heterocycloalkyl with the rest of the molecule. Examples of 4-membered heterocycloalkyl include, but are not limited to, azetidinyl, oxetanyl, thietanyl, etc.

Unless otherwise specified, the terms "C₆₋₁₀ aromatic ring" and "C₆₋₁₀ aryl" in the present disclosure can be used interchangeably, and the term "C₆₋₁₀ aromatic ring" or "C₆₋₁₀ aryl" refers to a cyclic hydrocarbon group consisting of 6 to 10 carbon atoms with a conjugated π-electron system, which can be a monocyclic, condensed bicyclic, or condensed tricyclic system, wherein each ring is aromatic. It can be monovalent, divalent, or multivalent, and the C₆₋₁₀ aryl includes C₆₋₉, C₉, C₁₀, C₆ aryl, etc. Examples of C₆₋₁₀ aryl include, but are not limited to, phenyl, naphthyl (including 1-naphthyl, 2-naphthyl, etc.).

Unless otherwise specified, the terms "5- to 10-membered heteroaryl ring" and "5- to 10-membered heteroaryl" in the present disclosure can be used interchangeably, and the term "5- to 10-membered heteroaryl" refers to a cyclic group consisting of 5 to 10 ring atoms with a conjugated π-electron system, wherein 1, 2, 3, or 4 ring atoms are heteroatoms independently selected from O, S, and N, and the rest are carbon atoms. It can be a monocyclic, condensed bicyclic, or condensed tricyclic system, wherein each ring is aromatic. Here, the nitrogen atom is optionally quaternized, and nitrogen and sulfur heteroatoms can be optionally oxidized (i.e., NO and S(O)ₚ, wherein p is 1 or 2). The 5- to 10-membered heteroaryl can be linked to the rest of the molecule through a heteroatom or a carbon atom. The 5- to 10-membered heteroaryl includes 5- to 8-membered, 5- to 7-membered, 5- to 6-membered, 5-membered, and 6-membered heteroaryl, etc. Examples of the 5- to 10-membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrrolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H-*1,2,3-triazolyl, 2*H*-1,2,3-triazolyl, 1*H*-1,2,4-triazolyl, 4*H*-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furyl (including 2-furyl, 3-furyl, etc.), thienyl (including 2-thienyl, 3-thienyl, etc.), pyridyl (including 2-pyridyl, 3-pyridyl, 4-pyridyl, etc.), pyrazinyl, pyrimidyl (including 2-pyrimidyl, 4-pyrimidyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolyl (including 1-isoquinolyl, 5-isoquinolinyl, etc.), quinoxalinyl (including 2-quinoxalinyl, 5-quinoxalinyl, etc.), or quinolinyl (including 3-quinolinyl, 6-quinolinyl, etc.).

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring.

The term "leaving group" refers to a functional group or atom which can be replaced by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonate; acyloxy, such as acetoxy, trifluoroacetoxy.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for blocking the side reaction on hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97).

The solvents used in the present disclosure are commercially available.

The present disclosure adopts the following abbreviations: Alloc stands for allyloxycarbonyl; SEM stands for trimethylsilylethoxymethyl; OTs stands for 4-toluenesulfonyl; Boc stands for tert-butoxycarbonyl; DCM stands for dichloromethane; DIEA stands for *N,N-*diisopropylethylamine; MeI stands for iodomethane; PE stands for petroleum ether; EA stands for ethyl acetate; THF stands for tetrahydrofuran; EtOH stands for ethanol; MeOH stands for methanol; Boc₂O stands for di-tert-butyl dicarbonate; NH₄Cl stands for ammonium chloride; T₃P stands for propylphosphonic anhydride; Pd/C stands for palladium/carbon catalyst; TMSN₃ stands for azidotrimethylsilane; NCS stands for N-chlorosuccinimide; HBr stands for hydrobromic acid; AcOH stands for acetic acid; HATU stands for 2-(7-azabenzotriazol-1-yl)-*N,N,N',N'*-tetramethyluronium hexafluorophosphate; DBU stands for 1,8-diazabicyclo[5.4.0]undec-7-ene; FA stands for formic acid; ACN stands for acetonitrile; TLC stands for thin-layer chromatography; HPLC stands for high pressure liquid chromatography; LCMS stands for liquid chromatography-mass spectrometry. DMSO stands for dimethyl sulfoxide; DMSO-*d₆* stands for deuterated dimethyl sulfoxide; CD₃OD stands for deuterated methanol; CDCl₃ stands for deuterated chloroform; D₂O stands for deuterium oxide.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure. It will be apparent to those skilled in the art that various variations and improvements can be made to specific embodiments of the present disclosure without departing from the spirit and scope of the present disclosure.

### Intermediate A

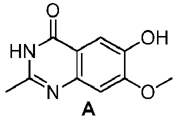

### Synthetic route:

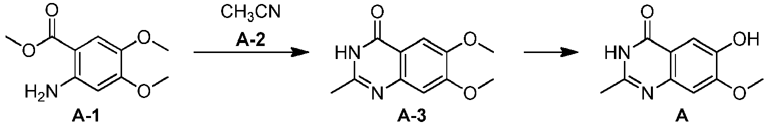

### Step 1

Compound **A-1** (10.0 g, 47.3 mmol) was dissolved in **A-2** acetonitrile (24 mL), and dry hydrogen chloride gas was introduced thereto at 25°C for 0.5 hours. The reaction mixture was stirred at 90°C for 3 hours, cooled to 25°C, and filtered. The filter cake was collected and dissolved in water (100 mL), and the resulting mixture was neutralized with a 10% sodium bicarbonate (100 mL) solution, and filtered. The filter cake was washed with ice water (100 mL) and dried to obtain compound **A-3.** ¹H NMR (400 MHz, CD₃OD) δ 7.55 (s, 1H), 7.07 (s, 1H), 3.98 (s, 3H), 3.95 (s, 3H), 2.45 (s, 3H).

### Step 2

Compound **A-3** (3.00 g, 13.6 mmol) was dissolved in methanesulfonic acid (15 mL), and added with *DL*-methionine (2.44 g, 16.4 mmol). The reaction mixture was reacted at 110°C for 12 hours, quenched with water (90 mL) and sodium hydroxide (2 mol/L, 150 mL), and filtered. The filter cake was collected and dried under vacuum to obtain intermediate A. ¹H NMR (400 MHz, DMSO-d₆) δ 7.33 (s, 1H), 7.02 (s, 1H), 3.88 (s, 3H), 2.88 (s, 3H).

### Intermediate B

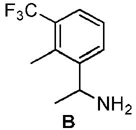

### Synthetic route:

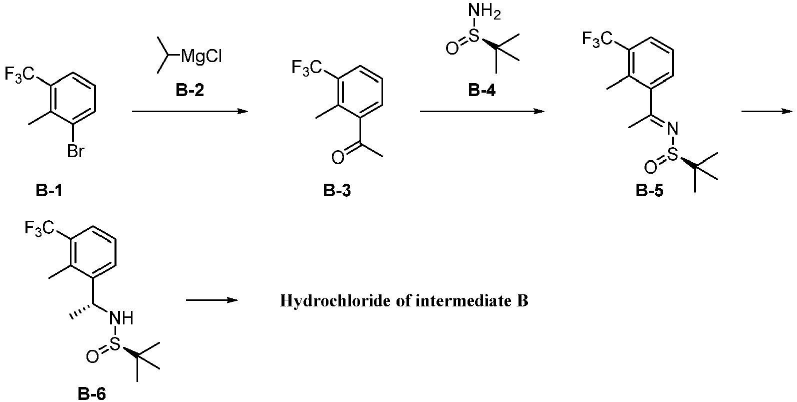

### Step 1

To a solution of compound **B-1** (20.0 g, 83.7 mmol) in tetrahydrofuran (50 mL) was added dropwise a solution of compound **B-2** (1.3 M, 96.5 mL) in tetrahydrofuran under nitrogen atmosphere, and the reaction mixture was stirred at 70°C for 3 hours under nitrogen atmosphere. The reaction system was added with a solution of acetic anhydride (12.8 g, 125 mmol) in tetrahydrofuran (50 mL) at 0°C, and the reaction mixture was stirred at 40°C for 1 hour. The reaction mixture was added with water (100 mL), extracted with ethyl acetate (100 mL × 3), washed with saturated brine (200 mL × 1), dried over anhydrous sodium sulfate, filtered, concentrated under reduced pressure, and the residue was purified by silica gel column chromatography (3/1, petroleum ether/ethyl acetate, Rf = 0.63) to obtain compound **B-3**. ¹H NMR (400 MHz, CDCl₃) δ 7.74 (d, *J*=8.0 Hz, 2H), 7.66 (d, *J*=8.0 Hz, 2H), 7.37 (t, *J*=8.0 Hz, 1H), 2.60 (s, 3H), 2.54 (s, 3H).

### Step 2

Compound **B-3** (8.00 g, 39.6 mmol) was dissolved in tetrahydrofuran (150 mL), then **B-4** (7.19 g, 59.4 mmol) and tetraethyl titanate (22.6 g, 98.9 mmol) were added thereto, and the reaction mixture was stirred at 70°C for 4 hours. The reaction mixture was added with water (300 mL), extracted with ethyl acetate (150 mL × 3), washed with saturated brine (200 mL × 1), dried over anhydrous sodium sulfate, filtered, concentrated under reduced pressure, and the residue was purified by silica gel column chromatography (2/1, petroleum ether/ethyl acetate, Rf = 0.41) to obtain compound **B-5**. MS-ESI calculated for [M+H]⁺ 306, found 306.

### Step 3

Compound **B-5** (6.50 g, 21.3 mmol) was dissolved in tetrahydrofuran (100 mL) and water (2 mL) at -78°C. Sodium borohydride (1.45 g, 38.3 mmol) was added thereto, and the reaction mixture was stirred at 25°C for 5 hours. The reaction mixture was added with water (100 mL), extracted with ethyl acetate (100 mL × 3), washed with saturated brine (200 mL × 1), dried over anhydrous sodium sulfate, filtered, concentrated under reduced pressure, and the residue was purified by silica gel column chromatography (2/1, petroleum ether/ethyl acetate, Rf = 0.21) to obtain compound **B-6.** MS-ESI calculated for [M+H]⁺ 308, found 308.

### Step 4

Compound **B-6** (3.60 g, 11.7 mmol) was dissolved in ethyl acetate (10 mL), and a solution of hydrogen chloride in ethyl acetate (4 M, 15.0 mL) was added thereto. The reaction mixture was stirred and reacted at 25°C for 6 hours. The reaction mixture was concentrated under reduced pressure, filtered, and the filter cake was washed with ethyl acetate (200 mL), and dried under vacuum to obtain the hydrochloride of intermediate **B.** ¹H NMR (400 MHz, DMSO-d₆) δ 8.71 (s, 3H), 7.97 (d, *J*=7.8 Hz, 1H), 7.70 (d, *J*=7.8 Hz, 1H), 7.52 (t, *J*=7.8 Hz, 1H), 4.73-4.69 (m, 1H), 2.45 (s, 3H), 1.52 (d, *J*=6.8 Hz, 3H).

### Intermediate C

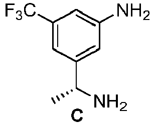

### Synthetic route:

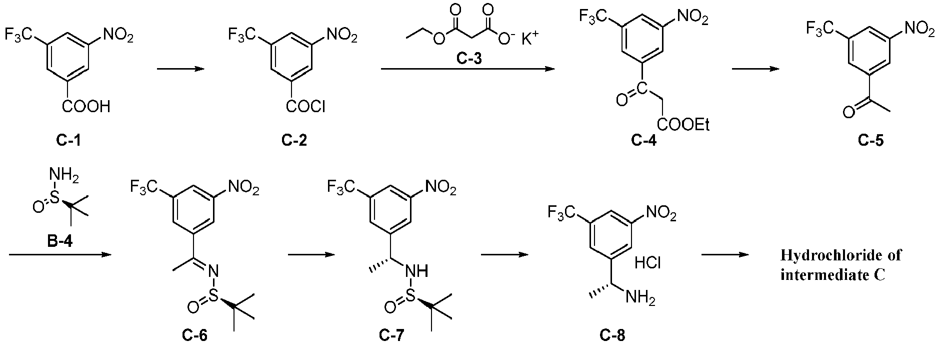

### Step 1

Compound **C-1** (10.0 g, 42.5 mmol) was dissolved in thionyl chloride (30 mL), and *N,N*-dimethylformamide (164 µL, 2.13 mmol) was added thereto. The reaction mixture was stirred and reacted at 80°C for 3 hours. The reaction mixture was added with anhydrous toluene (100 mL × 2) and concentrated under reduced pressure to obtain compound **C-2.**

### Step 2

Compound **C-3** (15.2 g, 89.5 mmol) was dissolved in acetonitrile (100 mL) under nitrogen atmosphere, and triethylamine (14.6 g, 144 mmol) and magnesium chloride (9.33 g, 98.0 mmol) were sequentially added thereto at 0°C, and the mixture was stirred at 15°C for 2 hours. The mixture was cooled to 0°C, and a solution of compound **C-2** (10.8 g, 42.6 mmol) in acetonitrile (50 mL) was added dropwise thereto, and the reaction mixture was stirred and reacted at 15°C for 12 hours. To the reaction mixture was added dilute hydrochloric acid (4 M, 100 mL) at 0°C. After the phases were separated, the mixture was concentrated to remove acetonitrile. The aqueous phase was extracted with ethyl acetate (150 mL × 2), and the organic phase was washed with saturated sodium bicarbonate aqueous solution (200 mL) and saturated brine (200 mL), dried over anhydrous sodium sulfate, filtered, and the filtrate was concentrated under reduced pressure to obtain compound **C-4.** MS-ESI calculated for [M-H]⁻ 304, found 304.

### Step 3

Compound **C-4** (12.8 g, 41.9 mmol) was dissolved in acetic acid (40 mL), then water (20 mL) and concentrated sulfuric acid (5 mL) were added thereto, and the reaction mixture was reacted at 100°C for 3 hours. The reaction mixture was concentrated to remove acetic acid, added with ice water (300 mL), extracted with ethyl acetate (100 mL × 3), washed with saturated sodium bicarbonate aqueous solution (100 mL × 2) and sodium hydroxide aqueous solution (2 mol/L, 100 mL × 2), dried over anhydrous sodium sulfate, and filtered. The filtrate was concentrated under reduced pressure, and the crude product was purified by silica gel column chromatography (petroleum ether/ethyl acetate, 10/1 to 2/1, V/V) to obtain compound **C-5.** MS-ESI calculated for [M-H]⁻ 232, found 232.

### Step 4

Compound C-5 (8.00 g, 34.3 mmol) was dissolved in tetrahydrofuran (80 mL), then **B-4** (6.24 g, 51.5 mmol) and tetraisopropyl titanate (30.5 g, 85.8 mmol, purity: 80%) were added thereto, and the reaction mixture was reacted at 80°C for 16 hours. The reaction mixture was added with water (300 mL) and filtered. The filtrate was dried over anhydrous sodium sulfate, filtered, concentrated under reduced pressure, and the residue was purified by silica gel column chromatography (petroleum ether/ethyl acetate, 10/1 to 3/1, V/V) to obtain compound **C-6.** MS-ESI calculated for [M+H]⁺ 337, found 337.

### Step 5

Compound **C-6** (8.00 g, 23.8 mmol) was dissolved in tetrahydrofuran (100 mL) and water (2 mL) at -78°C. Sodium borohydride (1.80 g, 47.5 mmol) was added thereto, and the reaction mixture was reacted at 20°C for 1 hour. The reaction mixture was added with water (300 mL), extracted with ethyl acetate (100 mL × 3), dried over anhydrous sodium sulfate, filtered, concentrated under reduced pressure, and the residue was purified by silica gel column chromatography (petroleum ether/ethyl acetate, 5/1 to 2/1, V/V) to obtain compound **C-7.** MS-ESI calculated for [M-H]⁻ 337, found 337.

### Step 6

Compound **C-7** (5.50 g, 16.3 mmol) was dissolved in ethyl acetate (10 mL), and a solution of hydrogen chloride in ethyl acetate (4 M, 50.0 mL) was added thereto. The reaction mixture was reacted at 15°C for 1 hour. The reaction mixture was concentrated under reduced pressure, then added with dichloromethane (50 mL), stirred, filtered, and dried under vacuum to obtain compound **C-8.** MS-ESI calculated for [M+H]⁺ 235, found 235.

### Step 7

Compound **C-8** (3.50 g, 12.9 mmol) was dissolved in ethyl acetate (70 mL), then palladium on carbon (0.70 g, purity: 10%) was added thereto, and the reaction mixture was reacted at 15°C for 2 hours under hydrogen (15 Psi) atmosphere. The reaction mixture was filtered to remove palladium on carbon, and concentrated under reduced pressure to obtain the hydrochloride of intermediate C. MS-ESI calculated for [M+H]⁺ 205, found 205.

### Example 1

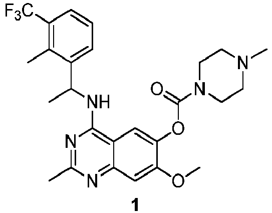

### Synthetic route:

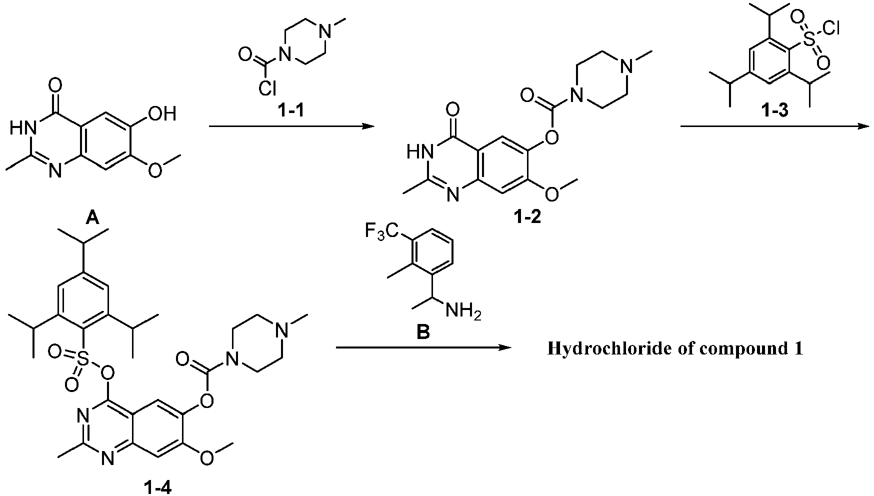

### Step 1

Intermediate **A** (200 mg, 970 µmol), 4-dimethylaminopyridine (11.9 mg, 97.0 µmol), triethylamine (393 mg, 3.88 mmol), and compound 1-1 (232 mg, 1.16 mmol) were dissolved in *N,N*-dimethylformamide (3 mL), and the reaction mixture was reacted at 20°C for 12 hours. After the reaction mixture was concentrated, the crude product was separated by silica gel column chromatography (10/1, dichloromethane/methanol, Rf = 0.2) to obtain compound **1-2.** ¹H NMR (400 MHz, DMSO-*d*₆) δ 12.14 (s, 1H), 7.66 (s, 1H), 7.17 (s, 1H), 3.88 (s, 3H), 3.63 (s, 2H), 3.49-3.40 (m, 2H), 2.48-2.40 (m, 4H), 2.33 (s, 3H), 2.29 (s, 3H). MS-ESI calculated for [M+H]⁺ 333, found 333.

### Step 2

Compound **1-2** (140 mg, 421 µmol), 4-dimethylaminopyridine (10.3 mg, 84.3 µmol), triethylamine (128 mg, 1.26 mmol), and compound **1-3** (255 mg, 842 µmol) were dissolved in dichloromethane (5 mL), and the reaction mixture was reacted at 15°C for 56 hours. After the reaction mixture was concentrated, the crude product was separated by silica gel column chromatography (10/1, dichloromethane/methanol, Rf = 0.45) to obtain compound **1-4.** ¹H NMR (400 MHz, DMSO-*d*₆) δ 7.79 (s, 1H), 7.47 (s, 1H), 7.35 (s, 2H), 4.24 - 4.14 (m, 2H), 4.03 (q, *J*=7.2 Hz, 1H), 3.97 (s, 3H), 3.69-3.58 (m, 2H), 3.47-3.41 (m, 1H), 3.02-2.91 (m, 1H), 2.45 (s, 3H), 2.43-2.34 (m, 4H), 2.25 (s, 3H), 1.23-1.17 (m, 18H). MS-ESI calculated for [M+H]⁺ 599, found 599.

### Step 3

Compound **1-4** (55 mg, 91.9 µmol), triethylamine (72.7 mg, 718 µmol), and the hydrochloride of intermediate **B** (28.0 mg, 138 µmol) were dissolved in dimethyl sulfoxide (1 mL), and the reaction mixture was reacted at 90°C for 34 hours. After the reaction mixture was concentrated, the resulting crude product was separated by preparative high performance liquid chromatography (chromatographic column: Phenomenex Luna C18 75 × 30 mm × 3 µm; mobile phase: 0.05% hydrochloric acid aqueous solution-acetonitrile; gradient: acetonitrile 19% to 39%, 7 min) to obtain the hydrochloride of compound **1.** ¹H NMR (400 MHz, DMSO-*d*₆) δ 14.91 (br s, 1H), 11.56 (br s, 1H), 10.52 (br s, 1H), 9.04 (br s, 1H), 8.01 (br d, *J*=8.0 Hz, 1H), 7.58 (br d, *J*=7.2 Hz, 1H), 7.43-7.36 (m, 2H), 5.99-5.83 (m, 1H), 4.40-4.19 (m, 1H), 4.18-4.04 (m, 2H), 3.96 (s, 3H), 3.71-3.44 (m, 1H), 3.26-3.12 (m, 3H), 2.85 (br s, 3H), 2.60 (br s, 3H), 2.57 (br s, 3H), 2.55-2.53 (m, 1H), 1.65 (br d, *J*=6.4 Hz, 3H). MS-ESI calculated for [M+H]⁺ 518, found 518.

### Example 2

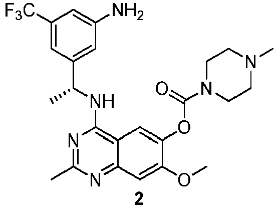

### Synthetic route:

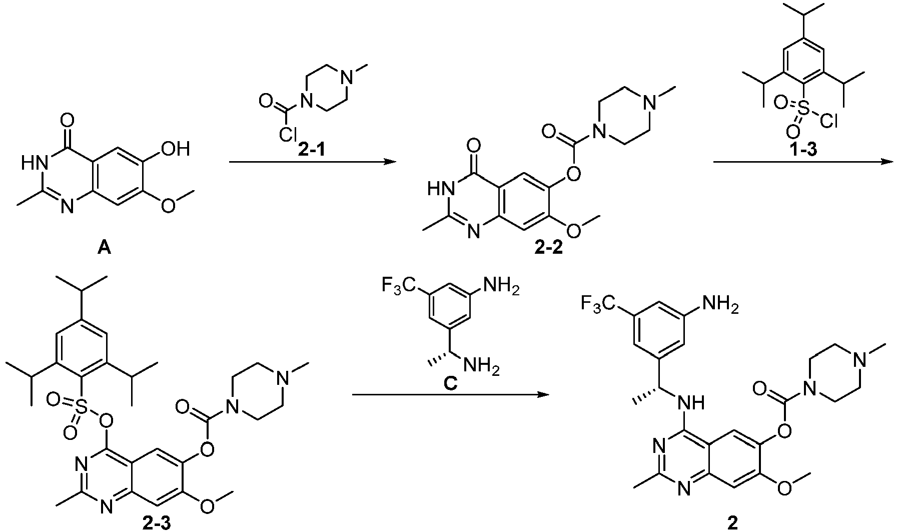

### Step 1

Intermediate **A** (300 mg, 1.45 mmol) and compound **2-1** (348 mg, 1.75 mmol) were dissolved in *N,N-*dimethylformamide (5 mL), then triethylamine (589 mg, 5.75 mmol) and 4-dimethylaminopyridine (17.8 mg, 0.145 mmol) were added to the reaction mixture. The reaction mixture was stirred at 20°C for 12 hours, and concentrated under reduced pressure. The crude product was separated by silica gel column chromatography (20/1, dichloromethane/methanol, Rf = 0.62) to obtain compound **2-2.** ¹H NMR (400 MHz, CD₃OD) δ 7.82 (s, 1H), 7.17 (s, 1H), 3.98 (s, 3H), 3.83-3.80 (br s, 2H), 3.65 (br s, 2H), 3.37 (s, 3H), 2.76 (br s 2H), 2.53 (br s, 2H), 2.47 (s, 3H).

### Step 2

Compounds **2-2** (442 mg, 1.33 mmol) and **1-3** (604 mg, 1.99 mmol) were dissolved in dichloromethane (10 mL), and 4-dimethylaminopyridine (32.5 mg, 0.266 mmol) and triethylamine (404 mg, 3.99 mmol) were added to the reaction mixture. The reaction mixture was stirred at 20°C for 12 hours, diluted with dichloromethane (30 mL), washed with saturated sodium bicarbonate aqueous solution (20 mL × 2) and brine (20 mL), dried over anhydrous sodium sulfate, filtered, and the filtrate was concentrated under reduced pressure. The crude product was separated by silica gel column chromatography (30/1, dichloromethane/methanol, Rf = 0.47) to obtain compound **2-3.** MS-ESI calculated for [M+H]⁺ 599, found 599.

### Step 3

Compound **2-3** (200 mg, 0.314 mmol) and the hydrochloride of intermediate C (77.0 mg, 0.320 mmol) were dissolved in dimethyl sulfoxide (5 mL), and triethylamine (153 mg, 1.51 mmol) was added to the reaction mixture. The reaction mixture was stirred at 100°C for 12 hours, quenched with water (10 mL), and extracted with ethyl acetate (15 mL × 3). The organic phase was washed with saturated brine (150 mL), dried over anhydrous sodium sulfate, filtered, and the filtrate was concentrated under reduced pressure. The residue was separated by preparative high performance liquid chromatography (chromatographic column: Waters Xbridge 150 × 25 mm × 5 µm; mobile phase: water (10 mmol/L ammonium bicarbonate aqueous solution-acetonitrile; gradient: acetonitrile 28% to 58%, 9 min) to obtain compound 2. ¹H NMR (400 MHz, CD₃OD) δ 8.05 (s, 1H), 7.12 (s, 1H), 7.99-6.96 (m, 2H), 6.81 (s,1H), 5.61 (q, *J*=7.2 Hz, 1H), 3.97 (s, 3H), 3.79 (br s , 2H), 3.60 (br s , 2H), 2.59-2.56 (m, 4H), 2.50 (s, 3H), 2.39 (s, 3H), 1.63 (d, *J*=7.2 Hz, 3H). MS-ESI calculated for [M+H]⁺ 519, found 519.

### Example 3

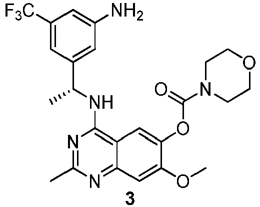

### Synthetic route:

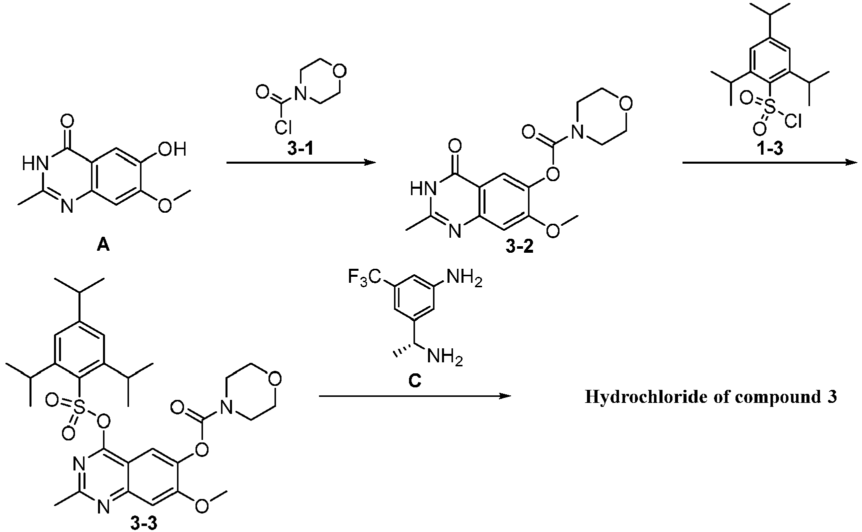

### Step 1

Intermediate **A** (300 mg, 1.45 mmol) and **3-1** (260 mg, 1.74 mmol) were dissolved in *N,N*-dimethylformamide (5 mL), then triethylamine (587 mg, 5.80 mmol) and 4-dimethylaminopyridine (17.7 mg, 0.145 mmol) were added to the reaction mixture. The reaction mixture was stirred at 25°C for 12 hours, and concentrated under reduced pressure. The crude product was separated by silica gel column chromatography (30/1, dichloromethane/methanol, Rf = 0.37) to obtain compound **3-2.** ¹H NMR (400 MHz, CDCl₃) δ 7.86 (s, 1H), 7.06 (s, 1H), 3.88 (s, 3H), 3.70-3.69 (br s, 2H), 3.66 (br s, 2H), 3.52 (br s, 2H), 2.46 (s, 3H), 1.60 (br s, 2H).

### Step 2

Compounds **3-2** (342 mg, 1.07 mmol) and **1-3** (357 mg, 1.18 mmol) were dissolved in dichloromethane (5 mL), then 4-dimethylaminopyridine (26.2 mg, 0.214 mmol) and triethylamine (325 mg, 3.21 mmol) were added to the reaction mixture. The reaction mixture was stirred at 20°C for 12 hours, concentrated under reduced pressure, and the crude product was separated by thin-layer chromatography (40/1, dichloromethane/methanol, Rf = 0.35) to obtain compound **3-3.** MS-ESI calculated for [M-tBu+H]⁺ 586, found 586.

### Step 3

Compound **3-3** (201 mg, 0.343 mmol) and the hydrochloride of intermediate C (90.8 mg, 0.377 mmol) were dissolved in dimethyl sulfoxide (3 mL), and triethylamine (977 mg, 9.66 mmol) was added to the reaction mixture. The reaction mixture was stirred at 100°C for 2 hours, quenched with water (10 mL), and extracted with ethyl acetate (15 mL × 3). The organic phase was washed with saturated brine (150 mL), dried over anhydrous sodium sulfate, filtered, and the filtrate was concentrated under reduced pressure. The residue was separated by preparative high performance liquid chromatography (chromatographic column: 3_Phenomenex Luna C18 75 × 30 mm × 3 µm; mobile phase: 0.05% hydrochloric acid aqueous solution-acetonitrile; gradient: acetonitrile 23% to 43%, 7 min) to obtain the hydrochloride of compound **3.** ¹H NMR (400MHz, CD₃OD) δ 8.28 (s, 1H), 7.72 (s, 1H), 7.65 (s, 1H), 7.43-7.42 (m, 1H), 7.10 (s, 1H),5.77 (q, *J*=7.2 Hz, 1H), 3.95 (s, 3H), 3.65 (br s, 6H), 3.45 (br s, 2H), 2.55 (s, 3H), 1.67 (d, *J*=1.2 Hz, 3H). MS-ESI calculated for [M+H]⁺ 506, found 506.

### Example 4

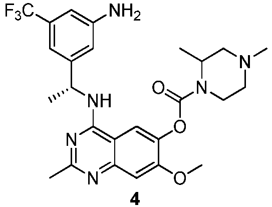

### Synthetic route:

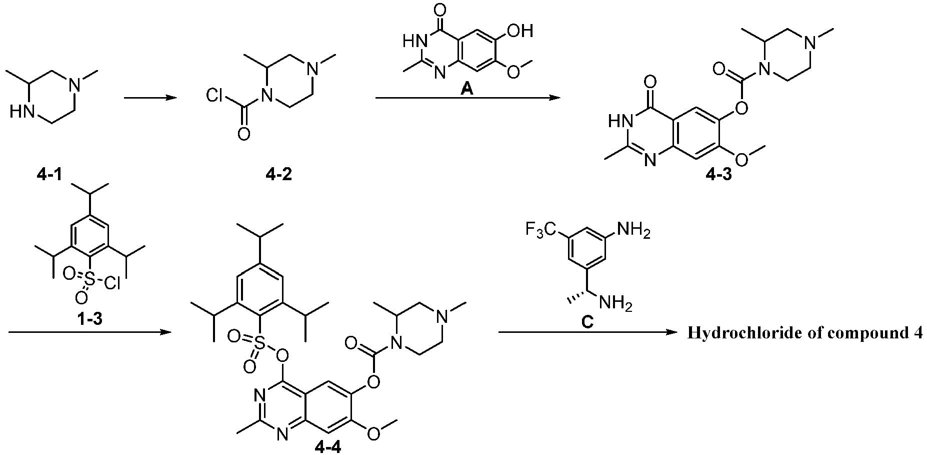

### Step 1

Compound **4-1** (200 mg, 1.75 mmol) was dissolved in dichloromethane (3 mL), and triphosgene (345 mg, 1.16 mmol) was added to the reaction mixture. The reaction mixture was cooled to -78°C, added with pyridine (383 mg, 4.84 mmol) under nitrogen atmosphere, slowly warmed to 0°C and then to 25°C. The reaction mixture was stirred at 25°C for 6 hours, and concentrated under reduced pressure to obtain compound **4-2.**

### Step 2

Compound **4-2** (201 mg, 1.14 mmol) and intermediate **A** (258 mg, 1.25 mmol) were dissolved in *N,N-*dimethylformamide (5 mL), and potassium carbonate (315 mg , 2.28 mmol) was added to the reaction mixture. The reaction mixture was stirred at 25°C for 12 hours, and concentrated under reduced pressure. The crude product was separated by thin-layer chromatography (20/1, dichloromethane/methanol, Rf = 0.63) to obtain compound **4-3.** ¹H NMR (CD₃OD, 400 MHz) δ 7.80 (s, 1H), 7.16 (s, 1H), 4.61-4.35 (m, 3H), 3.98 (s, 3H), 2.90-2.87 (m, 1H), 2.81-2.78 (m, 1H), 2.46 (s, 3H), 2.33 (s, 3H), 2.31-2.27 (m, 1H), 2.13-2.06 (m,1H), 1.43 (d, *J*=4.8 Hz, 3H).

### Step 3

Compounds **4-3** (231 mg, 0.667 mmol) and **1-3** (242 mg, 0.8 mmol) were dissolved in dichloromethane (10 mL), then 4-dimethylaminopyridine (16.3 mg, 0.133 mmol) and triethylamine (202 mg, 0.278 mmol) were added to the reaction mixture. The reaction mixture was stirred at 25°C for 12 hours, concentrated under reduced pressure, and the crude product was separated by thin-layer chromatography (40/1, dichloromethane/methanol, Rf = 0.22) to obtain compound **4-4.** MS-ESI calculated for [M-H]⁺ 613, found 613.

### Step 4

Compound **4-4** (315 mg, 0.502 mmol) and the hydrochloride of intermediate C (123 mg, 0.511 mmol) were dissolved in dimethyl sulfoxide (3 mL), and triethylamine (0.3 mL) was added to the reaction mixture. The reaction mixture was stirred at 100°C for 12 hours, quenched with water (10 mL), and extracted with a mixed solvent of dichloromethane/methanol (10:1, 20 mL × 3). The organic phase was washed with saturated brine (20 mL), dried over anhydrous sodium sulfate, filtered, and the filtrate was concentrated under reduced pressure. The crude product was separated by preparative high performance liquid chromatography (chromatographic column: 3_Phenomenex Luna C18 75 × 30 mm × 3 µm; mobile phase: 0.05% hydrochloric acid aqueous solution-acetonitrile; gradient: acetonitrile 12% to 32%, 7 min) to obtain the hydrochloride of compound **4.** ¹H NMR (400MHz, CD₃OD) δ 8.47 (s, 1H), 7.43 (s, 1H), 7.40 (s, 1H), 7.43 (s, 2H), 5.83 (q, *J*=7.2 Hz, 1H), 4.71-4.24 (m, 1H), 4.06 (s, 3H), 3.68-3.55 (m, 2H), 3.47-3.37 (m, 2H), 3.27-3.14 (m, 2H), 3.03 (s, 3H), 2.68 (s, 3H), 1.76 (d, *J*=7.2 Hz, 3H), 1.59-1.49 (m, 3H). MS-ESI calculated for [M+H]⁺ 533, found 533.

### Example 5

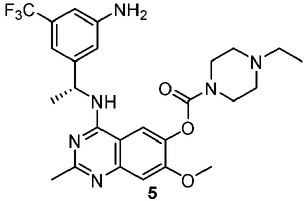

### Synthetic route:

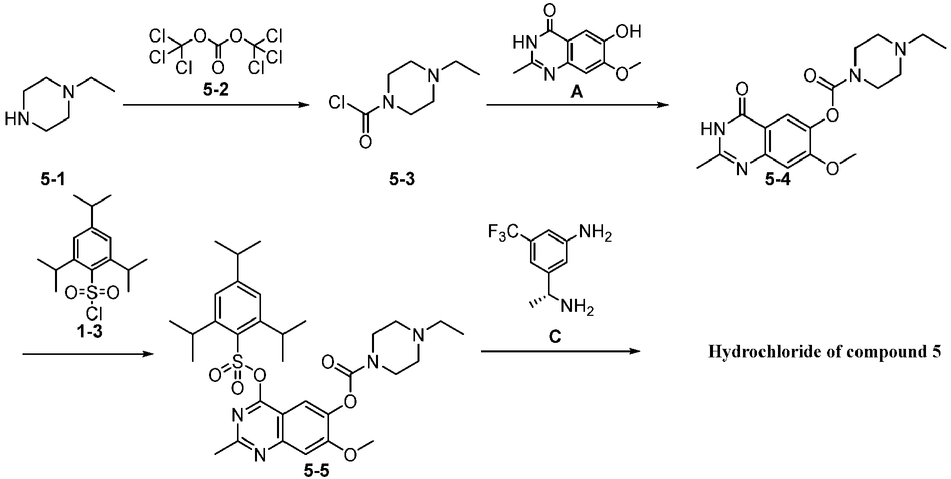

### Step 1

Compound **5-1** (100 mg, 876 µmol) was dissolved in dichloromethane (5 mL), and compound **5-2** (190 mg, 641 µmol) was added thereto at 0°C, and then pyridine (192 mg, 2.43 mmol) was added thereto. The reaction mixture was gradually warmed up from 0°C to 25°C, stirred and reacted at 25°C for 12 hours. The reaction mixture was concentrated under reduced pressure to obtain compound **5-3.**

### Step 2

Compound **5-3** (154 mg, 872 µmol) was dissolved in *N,N-*dimethylformamide (5 mL). Potassium carbonate (241 mg, 1.74 mmol) and intermediate **A** (198 mg, 959 µmol) were added thereto, and the reaction mixture was stirred and reacted at 25°C for 5 hours. After the reaction mixture was concentrated under reduced pressure, the reaction mixture was added with water (20 mL), extracted with dichloromethane (20 mL × 1). The organic phase was washed with saturated brine (20 mL × 3), dried over anhydrous sodium sulfate, filtered, concentrated under reduced pressure, and the residue was purified by silica gel column chromatography (dichloromethane/methanol, 100/1 to 10/1, V/V) to obtain compound **5-4.** ¹H NMR (400MHz, CD₃Cl) δ 7.94 (s, 1H), 7.12 (s, 1H), 3.94 (s, 3 H), 3.79 - 3.71 (m, 2H), 3.66 - 3.58 (m, 2H), 2.55 - 2.52 (m, 4H), 2.51 - 2.48 (m, 2H), 1.95 (s, 3H), 1.16 - 1.11 (m, 3H). MS-ESI calculated for [M+H]⁺ 347, found 347.

### Step 3

Compound **5-4** (140 mg, 404 µmol) was dissolved in dichloromethane (5 mL). Compound **1-3** (147 mg, 485 µmol), 4-dimethylaminopyridine (9.88 mg, 80.8 µmol), and triethylamine (123 mg, 1.21 mmol) were added thereto, and the reaction mixture was stirred and reacted at 25°C for 12 hours. The reaction mixture was filtered, concentrated under reduced pressure, and the residue was purified by silica gel column chromatography (dichloromethane/methanol, 100/1 to 10/1, V/V) to obtain compound **5-5.** ¹H NMR (400MHz, CD₃Cl) δ 7.83 (s, 1H), 7.29 - 7.27 (m, 1H), 7.22 - 7.20 (m, 2H), 4.49 - 4.10 (m, 3 H), 3.97 (s, 3H), 3.81 - 3.75 (m, 2H), 3.67 - 3.61 (m, 2H), 2.96 - 2.90 (m, 1H), 2.60 - 2.56 (m, 3H), 2.54 (s, 3H), 2.53 - 2.49 (m, 2H), 1.28 - 1.25 (m, 18H), 1.20 - 1.15 (m, 3H). MS-ESI calculated for [M+H]⁺ 613, found 613.

### Step 4

Compound **5-5** (178 mg, 290 µmol) was dissolved in dimethyl sulfoxide (5 mL). The hydrochloride of intermediate **C** (105 mg, 436 µmol) and triethylamine (88.2 mg, 871 µmol) were added thereto, and the reaction mixture was stirred and reacted at 100°C for 12 hours. The reaction mixture was added with water (20 mL), extracted with dichloromethane (20 mL × 3), and the organic phase was washed with saturated brine (20 mL × 5), dried over anhydrous sodium sulfate, filtered, and concentrated under reduced pressure. The residue was purified by preparative high performance liquid chromatography (chromatographic column: Phenomenx Synergi C18 100 × 21.2 mm× 4 µm; mobile phase: 0.05% hydrochloric acid aqueous solution-acetonitrile; gradient: acetonitrile 5% to 35%, 9 min) to obtain the hydrochloride of compound 5. ¹H NMR (400MHz, CD₃OD) δ 8.55 (s, 1H), 7.65 - 7.50 (m, 2H), 7.33 (s, 1H), 7.22 (s, 1H), 5.89 - 5.78 (m, 1H), 4.61 - 4.20 (m, 4H), 4.04 (s, 3H), 3.76 - 3.56 (m, 2H), 3.31 - 3.02 (m, 4H), 2.66 (s, 3H), 1.87 - 1.68 (m, 3H), 1.54 - 1.35 (m, 3H). MS-ESI calculated for [M+H]⁺ 533, found 533.

### Example 6

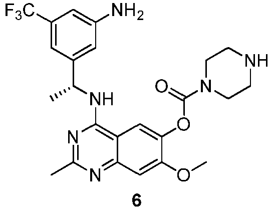

### Synthetic route:

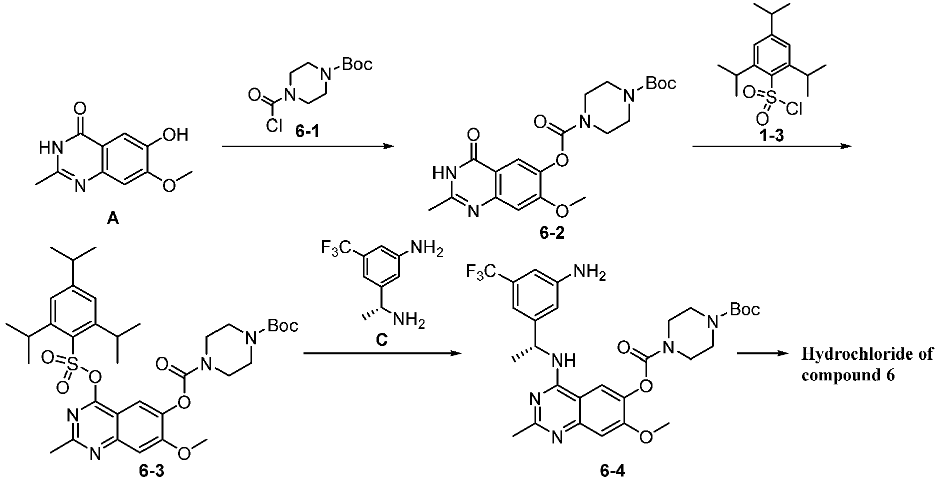

### Step 1

Intermediate **A** (200 mg, 970 µmol) and compound **6-1** (219 mg, 882 µmol) were dissolved in *N,N*-dimethylformamide (5 mL), and potassium carbonate (244 mg, 1.76 mmol) was added to the reaction mixture. The reaction mixture was stirred at 20°C for 12 hours, concentrated under reduced pressure, added with water (20 mL), and extracted with ethyl acetate (20 mL × 2). The organic phase was washed with saturated brine (20 mL), dried over anhydrous sodium sulfate, filtered, and the filtrate was concentrated under reduced pressure, and separated by silica gel column chromatography (10/1, dichloromethane/methanol, Rf = 0.74) to obtain compound **6-2.** MS-ESI calculated for [M+H]⁺ 419, found 419.

### Step 2

Compounds **6-2** (280 mg, 522 µmol) and **1-3** (190 mg, 626 µmol) were dissolved in dichloromethane (5 mL), then 4-dimethylaminopyridine (12.8 mg, 104 µmol) and triethylamine (158 mg, 1.57 mmol) were added to the reaction mixture. The reaction mixture was stirred at 20°C for 12 hours, concentrated under reduced pressure, and the crude product was separated by silica gel column chromatography (2/1, petroleum ether/ethyl acetate, Rf = 0.35) to obtain compound **6-3.** MS-ESI calculated for [M+H]⁺ 685, found 685.

### Step 3

Compound **6-3** (220 mg, 321 µmol) and the hydrochloride of intermediate **C** (98.4 mg, 482 µmol) were dissolved in dimethyl sulfoxide (5 mL), and triethylamine (97.5 mg, 964 µmol) was added to the reaction mixture. The reaction mixture was stirred at 100°C for 12 hours, quenched with water (20 mL), and extracted with ethyl acetate (20 mL × 2). The organic phase was washed with saturated brine (20 mL), dried over anhydrous sodium sulfate, filtered, and the filtrate was concentrated under reduced pressure to obtain compound **6-4.** MS-ESI calculated for [M+H]⁺ 605, found 605.

### Step 4

Compound **6-4** (192 mg, 318 µmol) was dissolved in a solution of hydrogen chloride in ethyl acetate (4 M, 10 mL). The reaction mixture was stirred at 25°C for 12 hours. The reaction mixture was concentrated under reduced pressure. The crude product was separated by preparative high performance liquid chromatography (chromatographic column: Venusil ASB Phenyl 150 × 30 mm × 5 µm; mobile phase: 0.05% hydrochloric acid aqueous solution-acetonitrile; gradient: acetonitrile 11% to 41%, 9 min) to obtain the hydrochloride of compound **6.** ¹H NMR (400 MHz, CD₃OD) δ 8.58 (s, 1H), 7.83 (s, 1H), 7.78 (s, 1H), 7.52 (s, 1H), 7.25 (s, 1H), 5.90 (q, *J*=6.8 Hz, 1H), 4.07 (s, 3H), 4.01 - 4.06 (m, 2H), 3.86 (s, 2H), 3.40 (s, 4H), 2.68 (s, 3H), 1.81 (d, *J*=6.8 Hz, 3H). MS-ESI calculated for [M+H]⁺ 505, found 505.

### Example 7

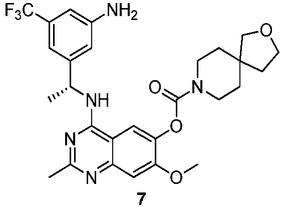

### Synthetic route:

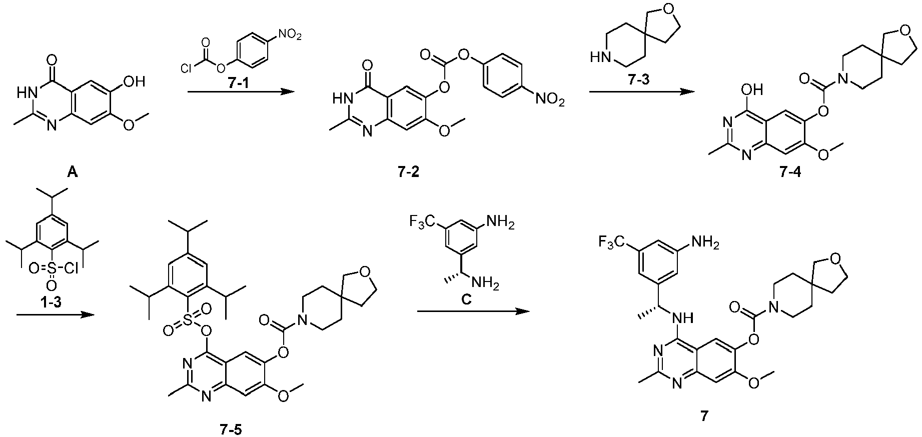

### Step 1

Intermediate **A** (300 mg, 2.91 mmol) was dissolved in tetrahydrofuran (5 mL), then compound **7-1** (674 mg, 3.35 mmol) and *N,N*-diisopropylethylamine (1.13 g, 8.73 mmol) were added thereto. The reaction mixture was stirred and reacted at 25°C for 1 hour. The reaction mixture was poured into water (20 mL), filtered, and the filtrate was extracted with ethyl acetate (20 mL × 3). The organic phase was dried over anhydrous sodium sulfate, and concentrated under reduced pressure to obtain compound **7-2.**

### Step 2

Compound **7-2** (600 mg, 1.62 mmol) and compound **7-3** (431 mg, 2.42 mmol) were dissolved in *N,N*-dimethylformamide (10 mL), and triethylamine (654 mg, 6.46 mmol) was added thereto, and the reaction mixture was reacted at 25°C for 6 hours. The reaction mixture was concentrated under reduced pressure to obtain a crude compound. The crude product was separated by silica gel column chromatography (dichloromethane/methanol, 30/1 to 10/1, V/V) to obtain compound **7-4.** MS-ESI calculated for [M+H]⁺ 374, found 374.

### Step 3

Compound **7-4** (200 mg, 0.314 mmol) was dissolved in dichloromethane (2 mL). To the reaction mixture were added 4-dimethylaminopyridine (9.82 mg, 80.3 µmol), triethylamine (243 mg, 2.41 mmol), and compound **1-3** (487 mg, 1.61 mmol), and the reaction mixture was stirred at 25°C for 16 hours. The reaction mixture was quenched with water (10 mL), extracted with ethyl acetate (10 mL × 3), dried over anhydrous sodium sulfate, and filtered. The filtrate was concentrated under reduced pressure, and the crude product was separated by silica gel column chromatography (1/1, petroleum ether/ethyl acetate, Rf = 0.3) to obtain compound **7-5.** MS-ESI calculated for [M+H]⁺ 640, found 640.

### Step 4

Compound **7-5** (130 mg, 0.203 mmol) was dissolved in dimethyl sulfoxide (1 mL). To the reaction mixture were added triethylamine (102 mg, 1.02 mmol) and the hydrochloride of intermediate C (73.4 mg, 305 µmol), and the reaction mixture was stirred at 90°C for 2 hours. The reaction mixture was quenched with water (0.5 mL), and the crude product was separated by high performance liquid chromatography (chromatographic column: Waters Xbridge 75 × 30 mm × 3 µm; mobile phase: 10 mM sodium bicarbonate aqueous solution-acetonitrile; gradient: 30% to 50%, 6 min) to obtain compound 7. ¹H NMR (400MHz, CD₃OD) δ 8.13 - 8.08 (m, 2H), 7.14 (s, 1H), 6.88 - 6.84 (m, 2H), 6.89 (s,1H), 5.54 - 5.49 (m, 3H), 3.87 (s, 3H), 3.80 - 3.77 (m, 2H), 3.52 (s, 6H), 2.38 (s, 3H), 1.81 - 1.77 (m, 2H), 1.06 - 1.50 (m, 7H). MS-ESI calculated for [M+H]⁺ 560, found 560.

### Example 8

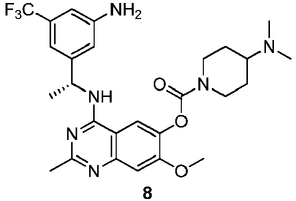

### Synthetic route:

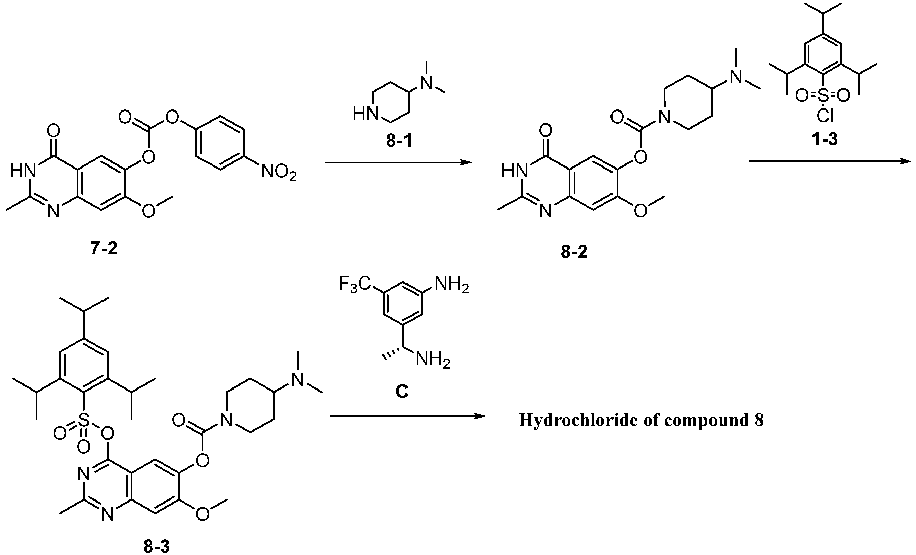

### Step 1

Compound **7-2** (165 mg, 444 µmol) was dissolved in *N,N*-dimethylformamide (10 mL). Triethylamine (180 mg, 1.78 mmol) and compound **8-1** (57 mg, 444 µmol) were added dropwise thereto, and the reaction mixture was stirred and reacted at 25°C for 1 hour. The reaction mixture was filtered, concentrated under reduced pressure, and the residue was purified by thin-layer chromatography (dichloromethane/methanol, 10/1, V/V) to obtain compound **8-2.** MS-ESI calculated for [M+H]⁺ 361, found 361.

### Step 2

Compound **8-2** (82 mg, 179 µmol) was dissolved in dichloromethane (10 mL). Compound **1-3** (64.9 mg, 214 µmol), 4-dimethylaminopyridine (4.36 mg, 35.7 µmol), and triethylamine (54.2 mg, 536 µmol) were added thereto, and the reaction mixture was stirred and reacted at 25°C for 12 hours. The reaction mixture was filtered, concentrated under reduced pressure, and the residue was purified by thin-layer chromatography (dichloromethane/methanol, 10/1, V/V) to obtain compound **8-3.** MS-ESI calculated for [M+H]⁺ 627, found 627.

### Step 3

Compound **8-3** (68.0 mg, 81.4 µmol) was dissolved in dimethyl sulfoxide (5 mL). The hydrochloride of intermediate **C** (29.4 mg, 122 µmol) and triethylamine (34.0 µL, 244 µmol) were added thereto, and the reaction mixture was stirred and reacted at 90°C for 12 hours. The reaction mixture was added with water (20 mL), extracted with dichloromethane (20 mL × 1), and the organic phase was washed with saturated brine (20 mL × 5), dried over anhydrous sodium sulfate, filtered, and concentrated under reduced pressure. The residue was purified by preparative high performance liquid chromatography (chromatographic column: Venusil ASB Phenyl 150 × 30 mm × 5 µm; mobile phase: 0.05% hydrochloric acid aqueous solution-acetonitrile; gradient: acetonitrile 20% to 50%, 9 min) to obtain the hydrochloride of compound **8.** ¹H NMR (400MHz, CD₃OD) δ 8.50 (s, 1H), 7.70 - 7.60 (m, 2H), 7.39 (s, 1H), 7.21 (s, 1H), 5.90 - 5.81 (m, 1H), 4.81 - 4.74 (m, 2H), 4.60 - 4.25 (m, 2H), 4.04 (s, 3H), 3.66 - 3.42 (m, 1H), 3.24 - 3.01 (m, 2H), 3.01 - 2.84 (m, 6H), 2.66 (m, 3H), 2.33 - 2.13 (m, 2H), 1.77 (d, *J*=6.4 Hz, 3H). MS-ESI calculated for [M+H]⁺ 547, found 547.

### Example 9

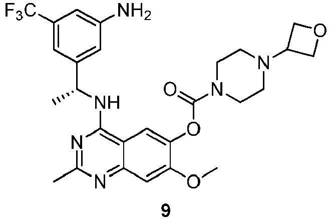

### Synthetic route:

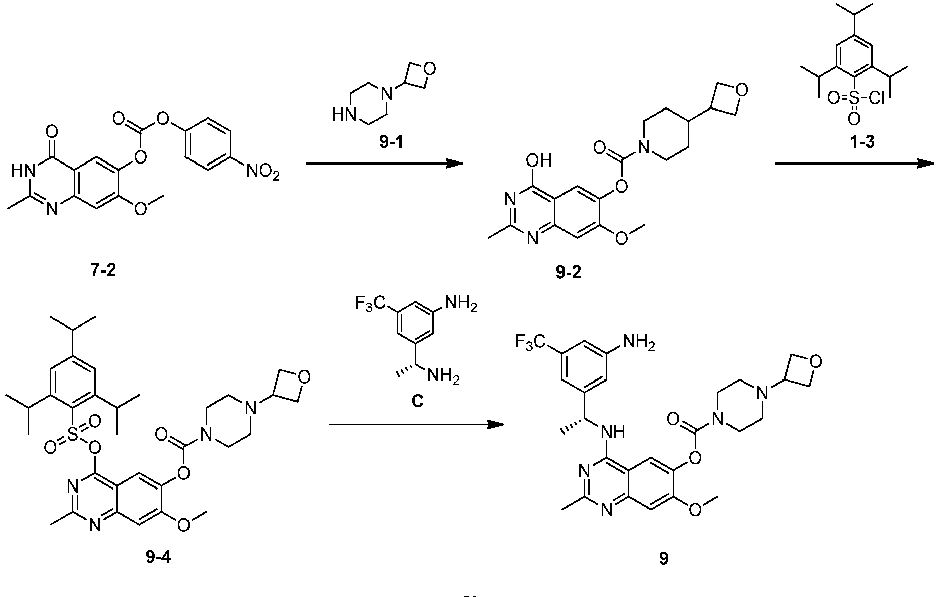

### Step 1

Compound **7-2** (160 mg, 431 µmol) and compound **9-1** (61 mg, 431 µmol) were dissolved in *N,N*-dimethylformamide (10 mL), then triethylamine (174 mg, 172 mmol) was added thereto, and the reaction mixture was reacted at 25°C for 1 hour. The reaction mixture was concentrated under reduced pressure to obtain a crude compound. The crude product was separated by thin-layer chromatography (10/1, V/V, dichloromethane/methanol, Rf = 0.08) to obtain compound **9-2.** MS-ESI calculated for [M+H]⁺ 375, found 375.

### Step 2

Compound **9-2** (60 mg, 160 µmol) was dissolved in dichloromethane (5 mL). To the reaction mixture were added 4-dimethylaminopyridine (4 mg, 32 µmol), triethylamine (49 mg, 481 µmol), and compound **1-3** (58 mg, 192 µmol), and the reaction mixture was concentrated under reduced pressure. The crude product was separated by silica gel column chromatography (2/1, V/V, petroleum ether/ethyl acetate, Rf = 0.5) to obtain compound **9-4.** MS-ESI calculated for [M+H]⁺ 641, found 641.

### Step 3

Compound **9-4** (30 mg, 46.8 µmol) was dissolved in dimethyl sulfoxide (2 mL). To the reaction mixture were added triethylamine (14.2 mg, 140 µmol) and the hydrochloride of intermediate **C** (16.9 mg, 70.2 µmol), and the reaction mixture was stirred at 100°C for 2 hours, quenched with water (20 mL), and extracted with ethyl acetate (20 mL × 2). The organic phase was washed with saturated brine (20 mL), dried over anhydrous sodium sulfate, filtered, and the filtrate was concentrated under reduced pressure. The crude product was separated by preparative high performance liquid chromatography (chromatographic column: Phenomenx Gemini-NX 80 × 30 mm× 3 µm; mobile phase: 10 mM sodium bicarbonate aqueous solution-acetonitrile; gradient: acetonitrile 10% to 80%, 9 min) to obtain compound **9.** ¹H NMR (400 MHz, DMSO-*d*₆) δ 8.02 (s, 1H), 7.12 (s, 1H), 6.97 (s, 1H), 6.81 (s, 1H), 6.81 (s, 1H), 5.59 (q, *J*=6.8 Hz, 1H), 4.72-4.78 (m, 2H), 4.65 - 4.69 (m, 2H), 3.96 (s, 3H), 3.81 (s, 2H), 3.58 - 3.67 (m, 2H), 2.48 (s, 7H), 1.62 (d, *J*=6.8 Hz, 3H). MS-ESI calculated for [M+H]⁺ 561, found 561.

### Example 10

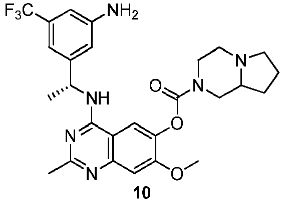

### Synthetic route:

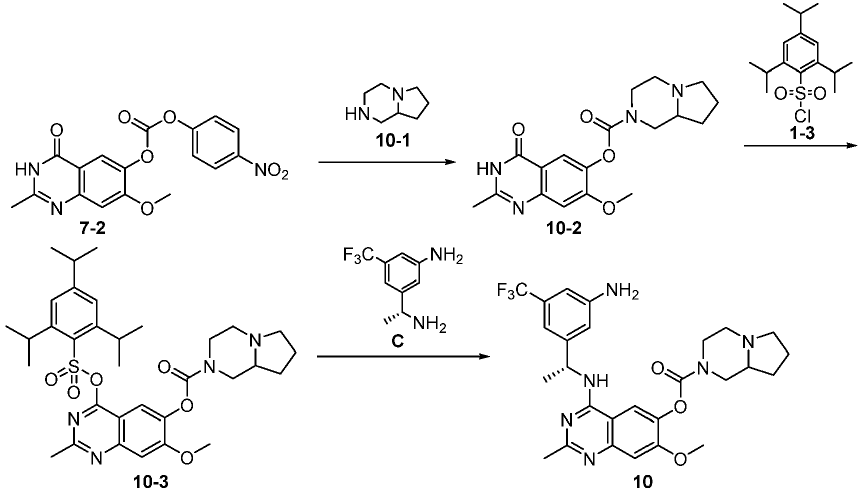

### Step 1

Compound **7-2** (183 mg, 493 µmol) was dissolved in *N,N*-dimethylformamide (10 mL). Triethylamine (199 mg, 1.97 mmol) and compound **10-1** (62.2 mg, 493 µmol) were added dropwise thereto, and the reaction mixture was stirred and reacted at 25°C for 1 hour. The reaction mixture was filtered, concentrated under reduced pressure, and the residue was purified by thin-layer chromatography (dichloromethane/methanol, 10/1, V/V) to obtain compound **10-2.** MS-ESI calculated for [M+H]⁺ 359, found 359.

### Step 2

Compound **10-2** (42 mg, 102 µmol) was dissolved in dichloromethane (10 mL). Compound **1-3** (37.1 mg, 122 µmol), 4-dimethylaminopyridine (2.49 mg, 20.4 µmol), and triethylamine (31.0 mg, 306 µmol) were added thereto, and the reaction mixture was stirred and reacted at 25°C for 12 hours. The reaction mixture was filtered, concentrated under reduced pressure, and the residue was purified by thin-layer chromatography (dichloromethane/methanol, 10/1, V/V) to obtain compound **10-3.** MS-ESI calculated for [M+H]⁺ 625, found 625.

### Step 3

Compound **10-3** (18 mg, 28.8 µmol) was dissolved in dimethyl sulfoxide (3 mL). The hydrochloride of intermediate **C** (10.4 mg, 43.2 µmol) and triethylamine (8.75 mg, 86.4 µmol) were added thereto, and the reaction mixture was stirred and reacted at 90°C for 12 hours. The reaction mixture was added with water (20 mL), extracted with dichloromethane (20 mL × 1), and the organic phase was washed with saturated brine (20 mL × 5), dried over anhydrous sodium sulfate, filtered, and concentrated under reduced pressure. The residue was purified by preparative high performance liquid chromatography (chromatographic column: Phenomenex Gemini-NX 80 × 30 mm × 3 µm; mobile phase: 10 mM ammonium bicarbonate aqueous solution-acetonitrile; gradient: acetonitrile 10% to 80%, 9 min) to obtain compound **10.** ¹H NMR (400MHz, CD₃OD) δ 8.01 (s, 1H), 7.11 (s, 1H), 7.00 - 6.93 (m, 2H), 6.79 (s, 1H), 5.61 - 5.55 (m, 1H), 4.49 - 4.10 (m, 2H), 3.94 (s, 3H), 3.28 - 3.06 (m, 3H), 2.99 - 2.66 (m, 1H), 2.46 (s, 3H), 2.37 - 2.21 (m, 2H), 2.20 - 2.03 (m, 1H), 2.00 - 1.78 (m, 3H), 1.60 (d, *J*=7.2 Hz, 3H), 1.53 - 1.41 (m, 1H). MS-ESI calculated for [M+H]⁺ 545, found 545.

### Example 11

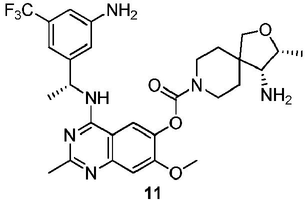

### Synthetic route:

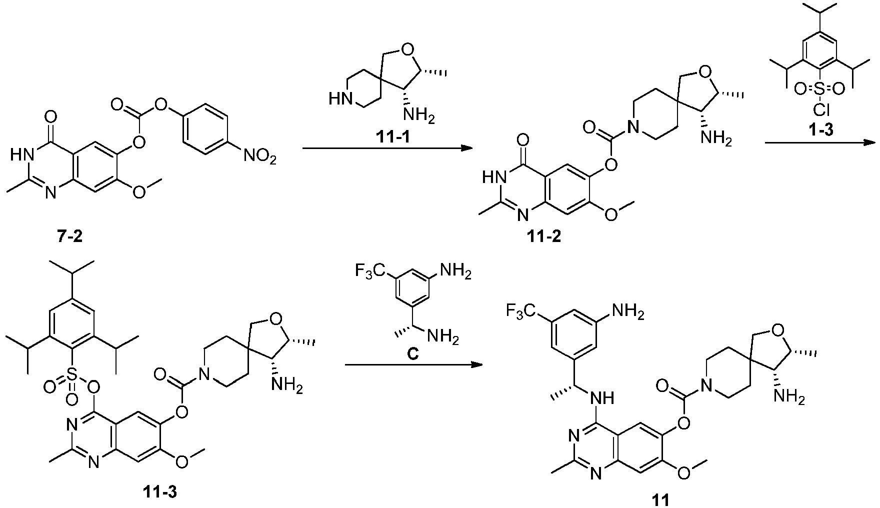

### Step 1

Compound **7-2** (293 mg, 789 µmol) was dissolved in *N,N*-dimethylformamide (5 mL). Triethylamine (319 mg, 3.16 mmol) and compound **11-1** (148 mg, 868 µmol) were added dropwise thereto, and the reaction mixture was stirred and reacted at 25°C for 1 hour. The reaction mixture was added with water (20 mL), extracted with dichloromethane (20 mL × 1). The organic phase was washed with saturated brine (20 mL × 1), dried over anhydrous sodium sulfate, filtered, concentrated under reduced pressure, and the residue was purified by thin-layer chromatography (dichloromethane/methanol, 10/1, V/V) to obtain compound **11-2.** MS-ESI calculated for [M+H]⁺ 403, found 403.

### Step 2

Compound **11-2** (50 mg, 124 µmol) was dissolved in dichloromethane (10 mL). Compound **1-3** (45.2 mg, 149 µmol), 4-dimethylaminopyridine (3.04 mg, 24.9 µmol), and triethylamine (37.7 mg, 373 µmol) were added thereto, and the reaction mixture was stirred and reacted at 25°C for 12 hours. The reaction mixture was filtered, concentrated under reduced pressure, and the residue was purified by thin-layer chromatography (dichloromethane/methanol, 10/1, V/V) to obtain compound **11-3.** MS-ESI calculated for [M+H]⁺ 669, found 669.

### Step 3

Compound **11-3** (20 mg, 29.9 µmol) was dissolved in dimethyl sulfoxide (2 mL). The hydrochloride of intermediate **C** (10.8 mg, 44.9 µmol) and triethylamine (9.08 mg, 89.7 µmol) were added thereto, and the reaction mixture was stirred and reacted at 90°C for 12 hours. The reaction mixture was added with water (20 mL), extracted with dichloromethane (20 mL × 1), and the organic phase was washed with saturated brine (20 mL × 5), dried over anhydrous sodium sulfate, filtered, and concentrated under reduced pressure. The residue was purified by preparative high performance liquid chromatography (chromatographic column: Phenomenex Gemini-NX 80 × 30 mm × 3 µm; mobile phase: 10 mM ammonium bicarbonate aqueous solution-acetonitrile; gradient: acetonitrile 37% to 67%, 9 min) to obtain compound **11.** ¹H NMR (400MHz, CD₃OD) δ 8.00 (s, 1H), 7.11(s, 1H), 7.01 - 6.93 (m, 2H), 6.80 (s, 1H), 5.61 - 5.53 (m, 1H), 4.31 - 4.19 (m, 1H), 3.95 (s, 3H), 3.89 - 3.85 (m, 1H), 3.77 - 3.70 (m, 1H), 3.11 - 3.05 (m, 1H), 2.46 (s, 3H), 1.86 - 1.77 (m, 1H), 1.60 (d, *J*=6.8 Hz, 3H), 1.36 - 1.31 (m, 2H), 1.31 - 1.26 (m, 4H), 1.24 (d, *J*=6.0 Hz, 3H), 0.92 - 0.86 (m, 1H). MS-ESI calculated for [M+H]⁺ 589, found 589.

### Example 12

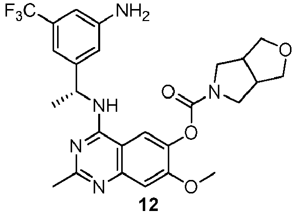

### Synthetic route:

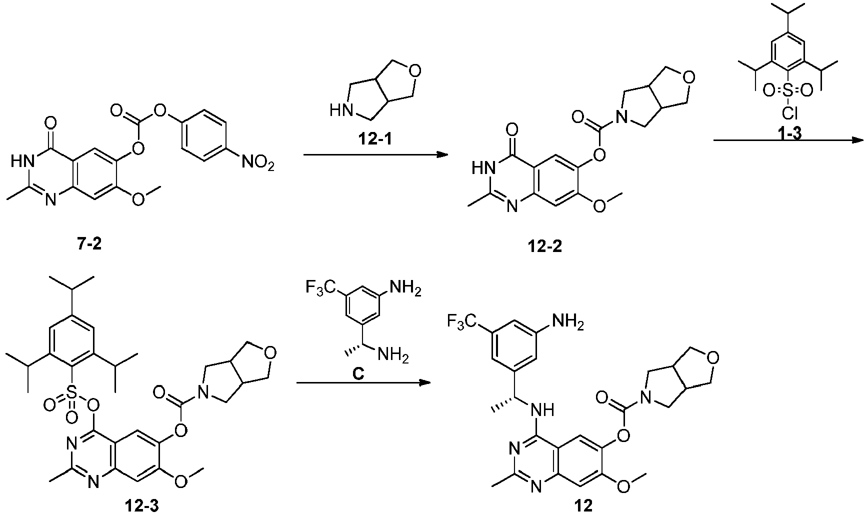

### Step 1

Compound **7-2** (390 mg, 1.05 mmol) was dissolved in *N,N*-dimethylformamide (10 mL). Triethylamine (425 mg, 4.20 mmol) and compound **12-1** (131 mg, 1.16 mmol) were added dropwise thereto, and the reaction mixture was stirred and reacted at 25°C for 1 hour. The reaction mixture was filtered, concentrated under reduced pressure, and the residue was purified by silica gel column chromatography (dichloromethane/methanol, 100/1 to 10/1, V/V) to obtain compound **12-2.** MS-ESI calculated for [M+H]⁺ 346, found 346.

### Step 2

Compound **12-2** (150 mg, 434 µmol) was dissolved in dichloromethane (10 mL). Compound **1-3** (197 mg, 652 µmol), 4-dimethylaminopyridine (10.6 mg, 86.9 µmol), and triethylamine (132 mg, 1.30 mmol) were added thereto, and the reaction mixture was stirred and reacted at 25°C for 12 hours. The reaction mixture was filtered, concentrated under reduced pressure, and the residue was purified by silica gel column chromatography (dichloromethane/methanol, 100/1 to 10/1, V/V) to obtain compound **12-3.** MS-ESI calculated for [M+H]⁺ 612, found 612.

### Step 3

Compound **12-3** (54.0 mg, 88.3 µmol) was dissolved in dimethyl sulfoxide (3 mL). The hydrochloride of intermediate **C** (31.9 mg, 132 µmol) and triethylamine (26.8 mg, 265 µmol) were added thereto, and the reaction mixture was stirred and reacted at 90°C for 12 hours. The reaction mixture was added with water (20 mL), extracted with dichloromethane (20 mL × 1), and the organic phase was washed with saturated brine (20 mL × 5), dried over anhydrous sodium sulfate, filtered, and concentrated under reduced pressure. The residue was purified by preparative high performance liquid chromatography (chromatographic column: Phenomenex Gemini-NX 80 × 30 mm × 3 µm; mobile phase: 10 mM ammonium bicarbonate aqueous solution-acetonitrile; gradient: acetonitrile 38% to 68%, 9 min) to obtain compound **12.** ¹H NMR (400MHz, CD₃OD) δ 8.01 (s, 1H), 7.11(s, 1H), 7.03 - 6.94 (m, 2H), 6.80 (s, 1H), 5.62 - 5.52 (m, 1H), 4.00 - 3.95 (m, 1H), 3.94 (s, 3H), 3.91 - 3.82 (m, 1H), 3.75 - 3.61 (m, 4H), 3.52 - 3.41 (m, 1H), 3.15 - 3.01 (m, 2H), 2.46 (s, 3H), 1.60 (d, *J*=7.2 Hz, 3H), 1.36 - 1.15 (m, 1H). MS-ESI calculated for [M+H]⁺ 532, found 532.

### Example 13

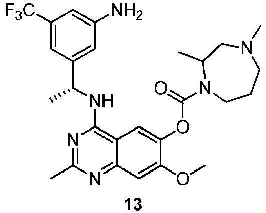

### Synthetic route:

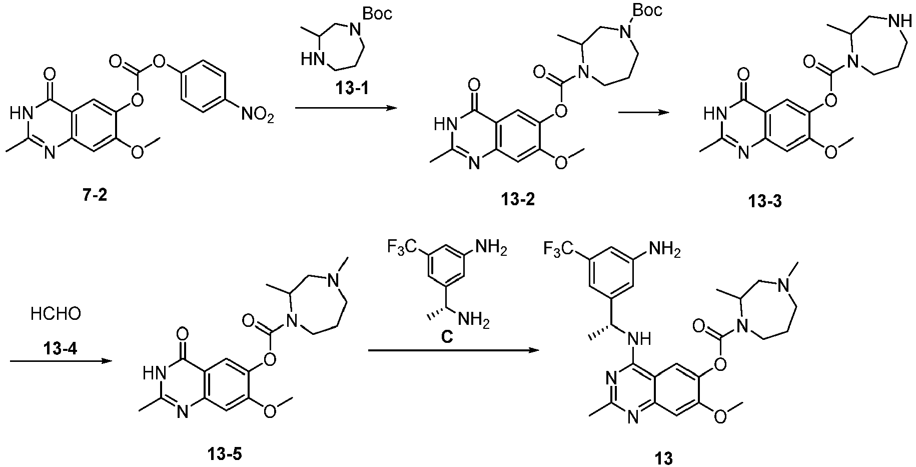

### Step 1

Compound **7-2** (700 mg, 1.89 mmol) was dissolved in *N,N-*dimethylformamide (10 mL). Triethylamine (763 mg, 7.54 mmol) and compound **13-1** (404 mg, 1.89 mmol) were added dropwise thereto, and the reaction mixture was stirred and reacted at 25°C for 1 hour. The reaction mixture was filtered, concentrated under reduced pressure, and the residue was purified by silica gel column chromatography (dichloromethane/methanol, 100/1 to 10/1, V/V) to obtain compound **13-2.** ¹H NMR (400MHz, CD₃Cl) δ 7.94 - 7.87 (m, 1H), 7.14 - 7.08 (m, 1H), 4.73 - 4.45(m, 1H), 4.24 - 3.99 (m, 2H), 3.94 (s, 3H), 3.21 - 2.94 (m, 2H), 2.89 - 2.75 (m, 2H), 2.52 (s, 3H), 2.01 - 1.78 (m, 2H), 1.68 (d, *J*=14 Hz, 3H), 1.55 - 1.46 (m, 9H). MS-ESI calculated for [M+H]⁺ 447, found 447.

### Step 2

Compound **13-2** (325 mg, 728 µmol) was dissolved in ethyl acetate (10 mL), and a solution of hydrogen chloride in ethyl acetate (4 M, 3.64 mL) was added thereto. The reaction mixture was stirred and reacted at 25°C for 12 hours. The reaction mixture was concentrated under reduced pressure, and the residue was added with ethyl acetate (20 mL) and stirred at 20°C for 30 minutes, then filtered. The upper solid was concentrated under reduced pressure to obtain the hydrochloride of compound **13-3.** MS-ESI calculated for [M+H]⁺ 347, found 347.

### Step 3

The hydrochloride of compound **13-3** (318 mg, 521 µmol) was dissolved in dichloromethane (5 mL) and methanol (5 mL), then 37% formaldehyde aqueous solution (127 mg, 1.56 mmol, purity: 37%), sodium triacetoxyborohydride (442 mg, 2.08 mmol), and acetic acid (62.6 mg, 1.04 mmol) were added thereto, and the reaction mixture was stirred and reacted at 25°C for 12 hours. The reaction mixture was filtered, concentrated under reduced pressure, and the residue was purified by silica gel column chromatography (dichloromethane/methanol, 100/1 to 10/1, V/V) to obtain compound **13-5.** MS-ESI calculated for [M+H]⁺ 361, found 361.

### Step 4

Compound **13-5** (160 mg, 444 µmol), *N,N*-diisopropylethylamine (172 mg, 1.33 mmol), and benzotriazol-1-yl-oxytripyrrolidinophosphonium hexafluorophosphate (347 mg, 666 µmol) were dissolved in *N,N*-dimethylformamide (7 mL), and the mixture was stirred and reacted at 25°C for 1 hour, then added with the hydrochloride of compound **C** (160 mg, 666 µmol) dissolved in *N,N*-dimethylformamide (3 mL), and stirred at 25°C for 12 hours. The reaction mixture was filtered and concentrated under reduced pressure, and the resulting residue was purified by preparative high performance liquid chromatography (chromatographic column: Phenomenex Gemini-NX 80 × 30 mm × 3 µm; mobile phase: 10 mM ammonium bicarbonate aqueous solution-acetonitrile; gradient: acetonitrile 41% to 71%, 9 min) to obtain compound **13.** ¹H NMR (400MHz, CD₃OD) δ 8.02 (s, 1H), 7.10 (s, 1H), 7.00 - 6.93 (m, 2H), 6.79 (s, 1H), 5.61 - 5.54 (m, 1H), 4.62 - 4.55 (m, 4H), 3.94 (s, 3H), 3.04 - 2.87 (m, 2H), 2.53 - 2.48 (m, 2H), 2.49 - 2.43 (m, 5H), 1.60 (d, *J*=7.2 Hz, 3H), 1.33 -1.27 (m, 1H), 1.27 - 1.20 (m, 2H), 1.15 - 1.06 (m, 2H). MS-ESI calculated for [M+H]⁺ 547, found 547.

### Example 14

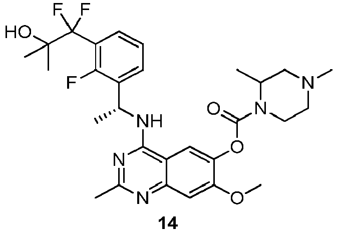

### Synthetic route:

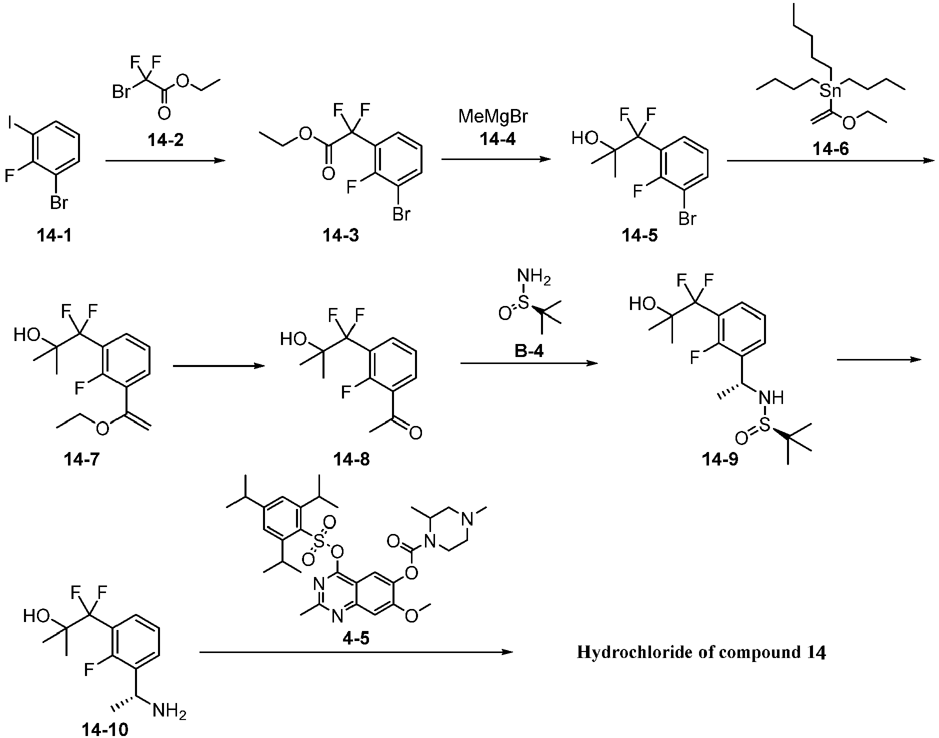

### Step 1

Ethyl bromodifluoroacetate (3.37 g, 16.6 mmol) was dissolved in dimethyl sulfoxide (20 mL), and copper powder (1.06 g, 16.6 mmol) was added thereto. The reaction mixture was stirred and reacted at 25°C for 1 hour. Then the reaction mixture was added with compound **14-1** (2.00 g, 6.65 mmol), stirred and reacted at 70°C for 12 hours. The reaction mixture was poured into 20 mL of ice water, added with ethyl acetate (20 mL), filtered, and the filtrate was extracted with ethyl acetate (20 mL × 3). The organic phase was washed with saturated brine (20 mL × 3), dried over anhydrous sodium sulfate, filtered, and concentrated under reduced pressure, and the residue was purified by silica gel column chromatography (petroleum ether/ethyl acetate, 1/0 to 20/1, V/V) to obtain compound **14-3.** ¹H NMR (400 MHz, CD₃Cl) δ 7.75 - 7.68 (m, 1H), 7.64 - 7.57 (m, 1H), 7.16 (t, *J*=8.0 Hz, 1H), 4.38 (m, 2H), 1.34 (t, *J*=8.0 Hz, 3H).

### Step 2

Compound **14-3** (677 mg, 1.82 mmol) was dissolved in toluene (10 mL) under nitrogen atmosphere, and methylmagnesium bromide solution (compound **14-4)** (3 M, 2.43 mL) was added thereto at 0°C. The reaction mixture was stirred and reacted at 25°C for 2 hours. The reaction mixture was added with saturated ammonium chloride solution (10 mL) to quench, and extracted with ethyl acetate (10 mL × 2). The organic phase was washed with saturated brine (10 mL × 2), dried over anhydrous sodium sulfate, filtered, and concentrated under reduced pressure, and the residue was purified by silica gel column chromatography (petroleum ether/ethyl acetate, 1/0 to 10/1, V/V) to obtain compound **14-5.** ¹H NMR (400 MHz, CD₃Cl) δ 7.66 (t, *J*=6.4 Hz, 1H), 7.51 - 7.34 (m, 1H), 7.16 - 6.93 (m, 1H), 2.01 (s, 1H), 1.35 (s, 6H).

### Step 3

Compound **14-5** (441 mg, 1.56 mmol) was dissolved in toluene (5 mL) under nitrogen atmosphere, and compound **14-6** (1.87 g, 5.19 mmol) and bis(triphenylphosphine)palladium(II) dichloride (109 mg, 0.16 mmol) were added thereto, and the reaction mixture was stirred and reacted at 120°C for 12 hours. The reaction mixture was added with saturated potassium fluoride solution (20 mL) to quench, and extracted with ethyl acetate (15 mL × 2). The reaction mixture was filtered, and concentrated under reduced pressure to obtain compound **14-7.**

### Step 4

Compound **14-7** (425 mg, 1.55 mmol) was dissolved in acetone (10 mL) under nitrogen atmosphere, and hydrochloric acid solution (12 M, 1.03 mL) was added dropwise thereto at 0°C. The reaction mixture was stirred and reacted at 25°C for 1 hour. The reaction mixture was neutralized to pH = 8 by adding saturated sodium bicarbonate solution, and extracted with ethyl acetate (10 mL). The organic phase was washed with saturated brine (10 mL × 1), dried over anhydrous sodium sulfate, filtered, and concentrated under reduced pressure, and the residue was purified by silica gel column chromatography (petroleum ether/ethyl acetate, 20/1 to 4/1, V/V) to obtain compound **14-8.** MS-ESI calculated for [M+H]⁺ 247, found 247.

### Step 5

Compound **14-8** (346 mg, 1.41 mmol) was dissolved in tetrahydrofuran (5 mL) at 25°C, and compound **B-4** (511 mg, 4.22 mmol) and tetraethyl titanate (2.00 g, 7.03 mmol) were added thereto. The reaction mixture was stirred and reacted at 80°C for 36 hours. Then the reaction mixture was added with sodium borohydride (64.0 mg, 1.69 mmol) at -5°C, stirred and reacted at 25°C for 1 hour. The reaction mixture was poured into 20 mL of ice water, filtered, and the filtrate was extracted with ethyl acetate (5 mL × 2). The organic phase was washed with saturated brine (5 mL × 1), dried over anhydrous sodium sulfate, filtered, and concentrated under reduced pressure, and the residue was purified by silica gel column chromatography (petroleum ether/ethyl acetate, 2/1 to 0/1, V/V) to obtain compound **14-9.** MS-ESI calculated for [M+H]⁺ 352, found 352.

### Step 6

Compound **14-9** (366 mg, 1.04 mmol) was dissolved in dioxane (2.5 mL), and a solution of hydrogen chloride in dioxane (4 M, 1.15 mL) was added thereto. The reaction mixture was stirred and reacted at 25°C for 6 hours. The reaction mixture was concentrated under reduced pressure, and the residue was purified by silica gel column chromatography (dichloromethane/methanol, 1/0 to 8/1, V/V) to obtain the hydrochloride of compound **14-10.** MS-ESI calculated for [M+H]⁺ 248, found 248.

### Step 7

The hydrochloride of compound **14-10** (374 mg, 0.61 mmol) was dissolved in dimethyl sulfoxide (3 mL) under nitrogen atmosphere, and compound **4-5** (181 mg, 0.73 mmol) and triethylamine (926 mg, 9.15 mmol) were added thereto. The reaction mixture was stirred and reacted at 90°C for 12 hours. The reaction mixture was added with water (5 mL), extracted with ethyl acetate (5 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Phenomenx Synergi C18 150 × 30 mm× 4 µm; mobile phase: 0.05% hydrochloric acid aqueous solution-acetonitrile; gradient: acetonitrile 29% to 59%, 9 min) to obtain the hydrochloride of compound **14.** ¹H NMR (400 MHz, CD₃OD) δ 8.46 (s, 1H), 7.63 (t, *J*=6.8 Hz, 1H), 7.42 (t, *J*=6.8 Hz, 1H), 7.26 - 7.19 (m, 2H), 6.01 (q, *J*=6.8 Hz, 1H), 4.82 - 4.59 (m, 1H), 4.50 - 4.19 (m, 1H), 4.04 (s, 3H), 3.76 - 3.50 (m, 3H), 3.41 (s, 1H), 3.25 (s, 1H), 3.00 (s, 3H), 2.63 (s, 3H), 1.74 (d, *J*=7.2 Hz, 3H), 1.66 - 1.43 (m, 3H), 1.29 (s, 6H). MS-ESI calculated for [M+H]⁺ 576, found 576.

### Example 15

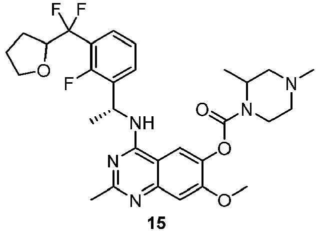

### Synthetic route:

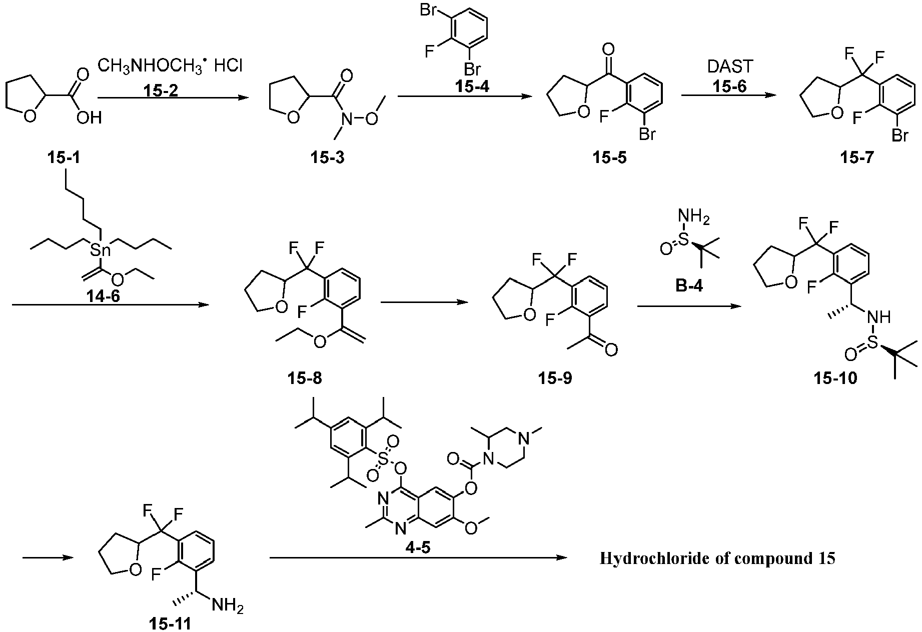

### Step 1

Compound **15-1** (5.00 g, 43.1 mmol) was dissolved in dichloromethane (50 mL), then 1-(3-dimethylaminopropyl)-3-ethylcarbodiimide hydrochloride (compound **15-2**) (12.4 g, 64.6 mmol), triethylamine (6.54 g, 64.6 mmol), and 4-dimethylaminopyridine (52.6 mg, 0.43 mmol) were added thereto at 0°C, and the reaction mixture was stirred at 25°C for 16 hours. The reaction mixture was added with 1M hydrochloric acid solution (20 mL) and extracted with dichloromethane (50 mL × 2). The organic phase was washed with saturated sodium bicarbonate solution (50 mL × 2), dried over anhydrous sodium sulfate, filtered, and concentrated under reduced pressure, and the residue was purified by silica gel column chromatography (petroleum ether/ethyl acetate, 4/1 to 0/1, V/V) to obtain compound **15-3.** MS-ESI calculated for [M+H]⁺ 160, found 160.

### Step 2

Compound **15-3** (2.85 g, 11.2 mmol) was dissolved in tetrahydrofuran (10 mL), and a solution of n-butyllithium in tetrahydrofuran (2.5 M, 5.38 mL) was added thereto at -78°C, and the reaction mixture was stirred at -78°C for 30 minutes. A solution of compound **15-4** (2.00 g, 11.2 mmol) in tetrahydrofuran (10 mL) was added thereto, and the reaction mixture was stirred and reacted at 25°C for 3 hours. The reaction mixture was added with saturated ammonium chloride solution (20 mL) to quench, and extracted with ethyl acetate (30 mL × 2). The organic phase was washed with saturated brine (30 mL × 2), dried over anhydrous sodium sulfate, filtered, and concentrated under reduced pressure, and the residue was purified by silica gel column chromatography (petroleum ether/ethyl acetate, 1/0 to 4/1, V/V) to obtain compound **15-5.** MS-ESI calculated for [M+H]⁺ 273, found 273.

### Step 3

Compound **15-5** (1.70 g, 5.23 mmol) was dissolved in dichloromethane (20 mL), and diethylaminosulfur trifluoride (1.26 g, 7.84 mmol) was added thereto at 0°C, and the reaction mixture was stirred and reacted at 25°C for 16 hours. The reaction mixture was poured into 20 mL of ice water and extracted with ethyl acetate (20 mL × 2). The organic phase was washed with saturated brine (20 mL × 2), dried over anhydrous sodium sulfate, filtered, and concentrated under reduced pressure, and the residue was purified by silica gel column chromatography (petroleum ether/ethyl acetate, 100/1 to 10/1, V/V) to obtain compound **15-7.** MS-ESI calculated for [M+H]⁺ 295, found 295.

### Step 4

Compound **15-7** (1.37 g, 4.64 mmol) was dissolved in toluene (10 mL) under nitrogen atmosphere, and compound **14-6** (5.58 g, 15.5 mmol) and bis(triphenylphosphine)palladium(II) dichloride (326 mg, 464 µmol) were added thereto, and the reaction mixture was stirred and reacted at 120°C for 12 hours. The reaction mixture was added with saturated potassium fluoride solution (20 mL) to quench, and extracted with ethyl acetate (15 mL × 2). The organic phase was filtered and concentrated under reduced pressure to obtain the crude product, which could be directly used in the next step.

### Step 5

Compound **15-8** (1.32 g, 4.61 mmol) was dissolved in acetone (30 mL) under nitrogen atmosphere, and hydrochloric acid solution (12 M, 3.07 mL) was added dropwise thereto at 0°C. The reaction mixture was stirred and reacted at 25°C for 1 hour. The reaction mixture was neutralized to pH = 8 by adding saturated sodium bicarbonate solution, and extracted with ethyl acetate (30 mL). The organic phase was washed with saturated brine (20 mL × 1), dried over anhydrous sodium sulfate, filtered, and concentrated under reduced pressure, and the residue was purified by silica gel column chromatography (petroleum ether/ethyl acetate, 50/1 to 10/1, V/V) to obtain compound **15-9.** MS-ESI calculated for [M+H]⁺ 259, found 259.

### Step 6

Compound **15-9** (936 mg, 3.62 mmol) was dissolved in tetrahydrofuran (10 mL) at 25°C, and compound **B-4** (659 mg, 5.44 mmol) and tetraethyl titanate (3.09 g, 10.9 mmol) were added thereto. The reaction mixture was stirred and reacted at 80°C for 23 hours. Then the reaction mixture was added with sodium borohydride (165 mg, 4.35 mmol) at -5°C, stirred and reacted at 25°C for 1 hour. The reaction mixture was poured into 20 mL of ice water, filtered, and the filtrate was extracted with ethyl acetate (10 mL × 2). The organic phase was washed with saturated brine (10 mL × 1), dried over anhydrous sodium sulfate, filtered, and concentrated under reduced pressure, and the residue was purified by silica gel column chromatography (petroleum ether/ethyl acetate, 2/1 to 1/2, V/V) to obtain compound **15-10.** MS-ESI calculated for [M+H]⁺ 364, found 364.

### Step 7

Compound **15-10** (366 mg, 1.04 mmol) was dissolved in dioxane (2.5 mL), and a solution of hydrogen chloride in dioxane (4 M, 1.15 mL) was added thereto. The reaction mixture was stirred and reacted at 25°C for 8 hours. The reaction mixture was concentrated under reduced pressure, and the residue was purified by silica gel column chromatography (dichloromethane/methanol, 1/0 to 8/1, V/V) to obtain the hydrochloride of compound **15-11.** MS-ESI calculated for [M+H]⁺ 260, found 260.

### Step 8

The hydrochloride of compound **15-11** (778 mg, 1.27 mmol) was dissolved in dimethyl sulfoxide (5 mL) under nitrogen atmosphere, and compound **4-5** (395 mg, 1.52 mmol) and triethylamine (1.93 g, 19.0 mmol) were added thereto. The reaction mixture was stirred and reacted at 90°C for 12 hours. The reaction mixture was added with water (5 mL), extracted with ethyl acetate (5 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Xtimate C18 150 × 40 mm× 5 µm; mobile phase: 0.05% hydrochloric acid aqueous solution-acetonitrile; gradient: acetonitrile 10% to 40%, 10 min) to obtain the hydrochloride of compound **15.** ¹H NMR (400 MHz, CD₃OD) δ 8.46 (s, 1H), 7.65 (t, *J*=6.8 Hz, 1H), 7.46 (s, 1H), 7.29 - 7.14 (m, 2H), 5.94 - 6.04 (m, 1H), 4.70 - 4.12 (m, 3H), 4.04 (s, 3H), 3.88 - 3.75 (m, 2H), 3.72 - 3.48 (m, 3H), 3.45 - 3.35 (m, 1H), 3.25 (s, 1H), 3.00 (s, 3H), 2.63 (s, 3H), 2.18 - 1.99 (m, 2H), 1.97 - 1.85 (m, 2H), 1.75 (d, *J*=7.2 Hz, 3H), 1.67 - 1.41 (m, 3H). MS-ESI calculated for [M+H]⁺ 588, found 588.

### Example 16

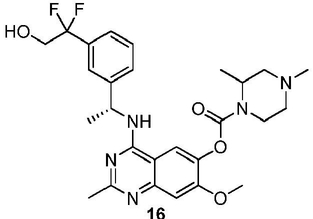

### Synthetic route:

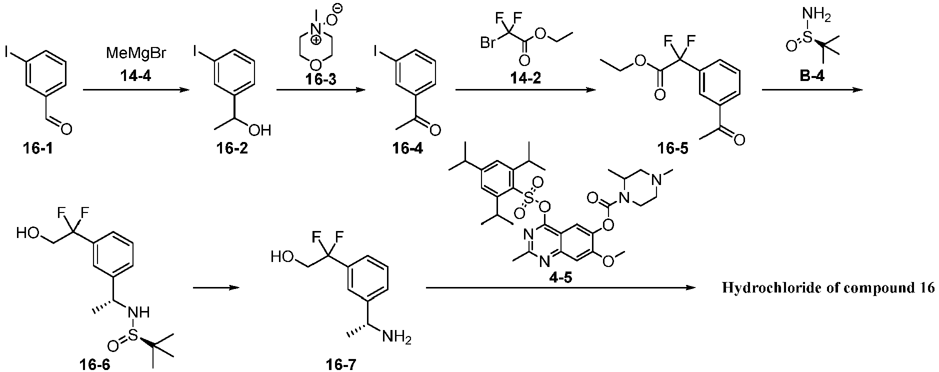

### Step 1

Compound **16-1** (2.00 g, 8.62 mmol) was dissolved in tetrahydrofuran (20 mL) under nitrogen atmosphere, and methylmagnesium bromide solution (compound **14-4**) (3 M, 4.31 mL) was added thereto at -78°C. The reaction mixture was stirred and reacted at -78°C for 3 hours. The reaction mixture was added with saturated ammonium chloride solution (10 mL) to quench, and extracted with ethyl acetate (20 mL × 2). The organic phase was washed with saturated brine (20 mL × 2), dried over anhydrous sodium sulfate, filtered, and concentrated under reduced pressure, and the residue was purified by silica gel column chromatography (petroleum ether/ethyl acetate, 1/0 to 10/1, V/V) to obtain compound **16-2.** ¹H NMR (400 MHz, CD₃OD) δ 7.73 (d, *J*=1.2 Hz, 1H), 7.60 (d, *J*=8.0 Hz, 1H), 7.32 (d, *J*=7.2 Hz, 1H), 7.08 (t, *J*=8.0 Hz, 1H), 4.86 - 4.76 (m, 1H), 1.48 - 1.43 (m, 3H).

### Step 2

Compound **16-2** (3.01 g, 12.1 mmol) was dissolved in acetonitrile (30 mL), and 4-methylmorpholine *N*-oxide (2.13 g, 18.1 mmol) and tetrabutylammonium perruthenate (427 mg, 1.21 mmol) were added thereto at 25°C. The reaction mixture was stirred and reacted at 25°C for 4 hours. The reaction mixture was filtered and concentrated under reduced pressure, and the residue was purified by silica gel column chromatography (petroleum ether/ethyl acetate, 1/0 to 10/1, V/V) to obtain compound **16-4.** ESI calculated for [M+H]⁺ 247, found 247.

### Step 3

Compound ethyl bromodifluoroacetate (6.43 g, 31.7 mmol) was dissolved in dimethyl sulfoxide (30 mL), and then copper powder (2.01 g, 31.7 mmol) and compound **16-4** (2.60 g, 10.6 mmol) were added thereto. The reaction mixture was stirred and reacted at 90°C for 16 hours. The reaction mixture was poured into 20 mL of ice water, added with 10 mL of ethyl acetate, filtered, and the filtrate was extracted with ethyl acetate (10 mL × 3). The organic phase was washed with saturated brine (15 mL × 1), dried over anhydrous sodium sulfate, filtered, and concentrated under reduced pressure, and the residue was purified by silica gel column chromatography (petroleum ether/ethyl acetate, 1/0 to 20/1, V/V) to obtain compound **16-5.** MS-ESI calculated for [M+H]⁺ 243, found 243.

### Step 4

Compound **16-5** (1.12 g, 4.62 mmol) was dissolved in tetrahydrofuran (10 mL) at 25°C, and compound **B-4** (841 mg, 6.94 mmol) and tetraethyl titanate (3.16 g, 13.9 mmol) were added thereto. The reaction mixture was stirred and reacted at 80°C for 12 hours. Then the reaction mixture was added with sodium borohydride (525 mg, 13.9 mmol) and water (200 µL) at -78°C, stirred and reacted at 25°C for 3 hours. The reaction mixture was poured into 20 mL of ice water, filtered, and the filtrate was extracted with ethyl acetate (10 mL × 2). The organic phase was washed with saturated brine (10 mL × 1), dried over anhydrous sodium sulfate, filtered, and concentrated under reduced pressure, and the residue was purified by silica gel column chromatography (petroleum ether/ethyl acetate, 4/1 to 1/2, V/V) to obtain compound **16-6.** MS-ESI calculated for [M+H]⁺ 306, found 306.

### Step 5

Compound **16-6** (1.05 mg, 3.44 mmol) was dissolved in dioxane (8 mL), and a solution of hydrogen chloride in dioxane (4 M, 3.78 mL) was added thereto. The reaction mixture was stirred and reacted at 25°C for 6 hours. The reaction mixture was concentrated under reduced pressure, and the residue was purified by silica gel column chromatography (dichloromethane/petroleum ether, 0/1 to 10/1, V/V) to obtain the hydrochloride of compound **16-7.** MS-ESI calculated for [M+H]⁺ 202, found 202.

### Step 6

The hydrochloride of compound **16-7** (777 mg, 1.27 mmol) was dissolved in dimethyl sulfoxide (10 mL) under nitrogen atmosphere, and compound **4-5** (306 mg, 1.52 mmol) and triethylamine (1.93 g, 19.0 mmol) were added thereto. The reaction mixture was stirred and reacted at 90°C for 12 hours. The reaction mixture was added with water (5 mL), extracted with ethyl acetate (5 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Xtimate C18 150 × 40 mm× 5 µm; mobile phase: 0.05% hydrochloric acid aqueous solution-acetonitrile; gradient: acetonitrile 12% to 42%, 10 min) to obtain the hydrochloride of compound **16.** ¹H NMR (400 MHz, CD₃OD) δ 8.52 - 8.41 (m, 1H), 7.73 - 7.58 (m, 2H), 7.50 - 7.35 (m, 2H), 7.27 - 7.17 (m, 1H), 5.84 (d, *J*=5.6 Hz, 1H), 4.71 - 4.16 (m, 2H), 4.04 (s, 3H), 3.90 (t, *J*=13.2 Hz, 2H), 3.76 - 3.51 (m, 3H), 3.42 (s, 1H), 3.29 - 3.13 (m, 1H), 3.00 (s, 3H), 2.66 (s, 3H), 1.76 (d, *J*=5.6 Hz, 3H), 1.67 - 1.37 (m, 3H). MS-ESI calculated for [M+H]⁺ 530, found 530.

### Example 17

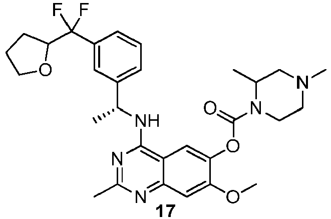

### Synthetic route:

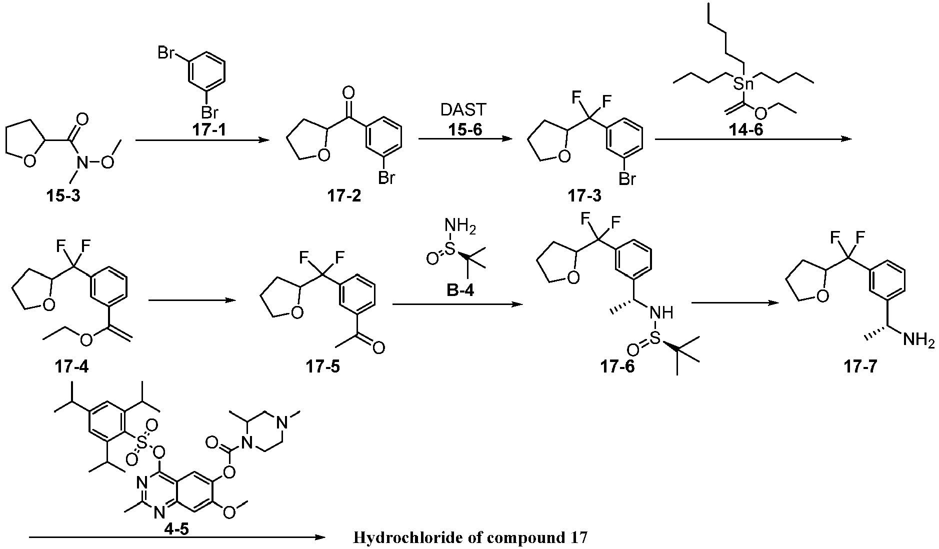

### Step 1

Compound **15-3** (2.33 g, 9.86 mmol) was dissolved in tetrahydrofuran (20 mL), and a solution of n-butyllithium in tetrahydrofuran (2.5 M, 4.74 mL) was added thereto at -78°C, and the reaction mixture was stirred and reacted at -78°C for 30 minutes. A solution of compound **17-1** (1.76 g, 9.86 mmol) in tetrahydrofuran (20 mL) was added thereto, and the reaction mixture was stirred and reacted at 25°C for 3 hours. The reaction mixture was added with saturated ammonium chloride solution (20 mL) to quench, and extracted with ethyl acetate (30 mL × 2). The organic phase was washed with saturated brine (30 mL × 2), dried over anhydrous sodium sulfate, filtered, and concentrated under reduced pressure, and the residue was purified by silica gel column chromatography (petroleum ether/ethyl acetate, 1/0 to 10/1, V/V) to obtain compound **17-2.** MS-ESI calculated for [M+H]⁺ 255, found 255.

### Step 2

Compound **17-2** (1.6 g, 6.27 mmol) was dissolved in dichloromethane (20 mL), and diethylaminosulfur trifluoride (3.03 g, 18.8 mmol) was added thereto at 0°C, and the reaction mixture was stirred and reacted at 25°C for 48 hours. The reaction mixture was poured into 20 mL of ice water and extracted with ethyl acetate (20 mL × 2). The organic phase was washed with saturated brine (20 mL × 3), dried over anhydrous sodium sulfate, filtered, and concentrated under reduced pressure, and the residue was purified by silica gel column chromatography (petroleum ether/ethyl acetate, 100/1 to 10/1, V/V) to obtain compound **17-3.** ¹H NMR (400 MHz, CD₃Cl) δ 7.69 (s, 1H), 7.58 (d, *J*=8.0 Hz, 1H), 7.47 (d, *J*=8.0 Hz, 1H), 7.34-7.28 (m, 1H), 4.31 (m, 1H), 3.84 (t, *J*=6.0 Hz, 2H), 2.05 (m, 2H), 1.90-1.81 (m, 2H), 1.82-1.81 (m, 2H).

### Step 3

Compound **17-3** (1.35 g, 4.87 mmol) was dissolved in toluene (10 mL) under nitrogen atmosphere, and compound **14-6** (586 g, 16.2 mmol) and bis(triphenylphosphine)palladium(II) dichloride (342 mg, 0.49 mmol) were added thereto, and the reaction mixture was stirred and reacted at 120°C for 12 hours. The reaction mixture was added with saturated potassium fluoride solution (20 mL) to quench, and extracted with ethyl acetate (15 mL × 2). The reaction mixture was filtered, and concentrated under reduced pressure to obtain compound **17-4.**

### Step 4

Compound **17-4** (1.3 g, 4.85 mmol) was dissolved in acetone (30 mL) under nitrogen atmosphere, and hydrochloric acid solution (12 M, 3.23 mL) was added dropwise thereto at 0°C. The reaction mixture was stirred and reacted at 25°C for 1 hour. The reaction mixture was neutralized to pH = 8 by adding saturated sodium bicarbonate solution, and extracted with ethyl acetate (30 mL). The organic phase was washed with saturated brine (30 mL × 1), dried over anhydrous sodium sulfate, filtered, and concentrated under reduced pressure, and the residue was purified by silica gel column chromatography (petroleum ether/ethyl acetate, 50/1 to 10/1, V/V) to obtain compound **17-5.** MS-ESI calculated for [M+H]⁺ 241, found 241.

### Step 5

Compound **17-5** (828 mg, 3.45 mmol) was dissolved in tetrahydrofuran (10 mL) at 25°C, and compound **B-4** (627 mg, 5.17 mmol) and tetraethyl titanate (2.94 g, 10.3 mmol) were added thereto. The reaction mixture was stirred and reacted at 80°C for 32 hours. Then the reaction mixture was added with sodium borohydride (156 mg, 4.14 mmol) at -5°C, stirred and reacted at 25°C for 1 hour. The reaction mixture was poured into ice water, filtered, and the filtrate was extracted with ethyl acetate (10 mL × 2). The organic phase was washed with saturated brine (10 mL × 1), dried over anhydrous sodium sulfate, filtered, and concentrated under reduced pressure, and the residue was purified by silica gel column chromatography (petroleum ether/ethyl acetate, 2/1 to 1/2, V/V) to obtain compound **17-6.** MS-ESI calculated for [M+H]⁺ 346, found 346.

### Step 6

Compound **17-6** (988 mg, 2.86 mmol) was dissolved in dioxane (7 mL), and a solution of hydrogen chloride in dioxane (4 M, 3.15 mL) was added thereto. The reaction mixture was stirred and reacted at 25°C for 6 hours. The reaction mixture was concentrated under reduced pressure, and the residue was purified by silica gel column chromatography (dichloromethane/methanol, 1/0 to 10/1, V/V) to obtain the hydrochloride of compound **17-7.** MS-ESI calculated for [M+H]⁺ 242, found 242.

### Step 7

The hydrochloride of compound **17-7** (798 mg, 1.30 mmol) was dissolved in dimethyl sulfoxide (5 mL) under nitrogen atmosphere, and compound **4-5** (377 mg, 1.56 mmol) and triethylamine (1.98 g, 19.5 mmol) were added thereto. The reaction mixture was stirred and reacted at 90°C for 12 hours. The reaction mixture was added with water (5 mL), extracted with ethyl acetate (5 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Xtimate C18 150 × 40 mm× 5 µm; mobile phase: 0.05% hydrochloric acid aqueous solution-acetonitrile; gradient: acetonitrile 3% to 33%, 10 min) to obtain the hydrochloride of compound **17.** ¹H NMR (400 MHz, CD₃OD) δ 8.58 - 8.37 (m, 1H), 7.71 - 7.56 (m, 2H), 7.43 (s, 2H), 7.30 - 7.15 (m, 1H), 5.82 (s, 1H), 4.83 - 4.57 (m, 1H), 4.50 - 4.15 (m, 2H), 4.04 (s, 3H), 3.80 - 3.52 (m, 5H), 3.43 (s, 1H), 3.26 (s, 1H), 3.00 (s, 3H), 2.65 (s, 3H), 1.98 (d, *J*=6.0 Hz, 2H), 1.85 - 1.67 (m, 5H), 1.63 - 1.43 (m, 3H). MS-ESI calculated for [M+H]⁺ 570, found 570.

### Example 18

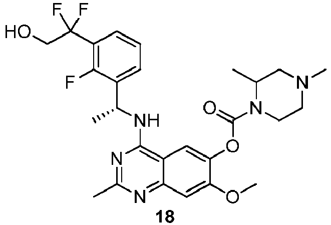

### Synthetic route:

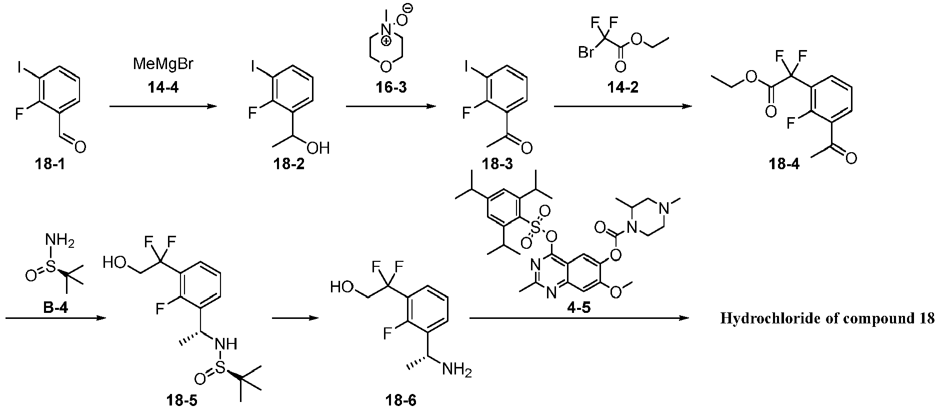

### Step 1

Compound **18-1** (3.86 g, 15.4 mmol) was dissolved in tetrahydrofuran (30 mL) under nitrogen atmosphere, and methylmagnesium bromide solution (3 M, 7.72 mL) was added thereto at -78°C. The reaction mixture was stirred and reacted at -78°C for 3 hours. The reaction mixture was added with saturated ammonium chloride solution (30 mL) to quench, and extracted with ethyl acetate (30 mL × 2). The organic phase was washed with saturated brine (30 mL × 2), dried over anhydrous sodium sulfate, filtered, and concentrated under reduced pressure, and the residue was purified by silica gel column chromatography (petroleum ether/ethyl acetate, 1/0 to 10/1, V/V) to obtain compound **18-2.** MS-ESI calculated for [M+H]⁺ 267, found 267.

### Step 2

Compound **18-2** (2.98 g, 11.2 mmol) was dissolved in acetonitrile (25 mL), and 4-methylmorpholine *N*-oxide (1.97 g, 16.8 mmol) and tetrabutylammonium perruthenate (394 mg, 1.12 mmol) were added thereto at 25°C. The reaction mixture was stirred and reacted at 25°C for 4 hours. The reaction mixture was filtered and concentrated under reduced pressure, and the residue was purified by silica gel column chromatography (petroleum ether/ethyl acetate, 1/0 to 10/1, V/V) to obtain compound **18-3.** MS-ESI calculated for [M+H]⁺ 265, found 265.

### Step 3

Compound ethyl bromodifluoroacetate (4.84 g, 23.9 mmol) was dissolved in dimethyl sulfoxide (20 mL), and copper powder (1.52 g, 23.9 mmol) and compound **18-3** (2.10 g, 7.95 mmol) were added thereto. The reaction mixture was stirred and reacted at 90°C for 16 hours. The reaction mixture was poured into 20 mL of ice water, added with 20 mL of ethyl acetate, filtered, and the filtrate was extracted with ethyl acetate (20 mL × 3). The organic phase was washed with saturated brine (20 mL × 1), dried over anhydrous sodium sulfate, filtered, and concentrated under reduced pressure, and the residue was purified by silica gel column chromatography (petroleum ether/ethyl acetate, 1/0 to 20/1, V/V) to obtain compound **18-4.** MS-ESI calculated for [M+H]⁺ 261, found 261.

### Step 4

Compound **18-4** (820 mg, 3.15 mmol) was dissolved in tetrahydrofuran (5 mL) at 25°C, and compound **B-4** (573 mg, 4.73 mmol) and tetraethyl titanate (2.16 g, 9.45 mmol) were added thereto. The reaction mixture was stirred and reacted at 80°C for 16 hours. Then the reaction mixture was added with sodium borohydride (358 mg, 9.45 mmol) and water (100 µL) at -78°C, warmed to 25°C, stirred and reacted at 25°C for 3 hours. The reaction mixture was poured into 5 mL of ice water, filtered, and the filtrate was extracted with ethyl acetate (5 mL × 2). The organic phase was washed with saturated brine (5 mL × 1), dried over anhydrous sodium sulfate, filtered, and concentrated under reduced pressure, and the residue was purified by silica gel column chromatography (petroleum ether/ethyl acetate, 4/1 to 0/1, V/V) to obtain compound **18-5.** MS-ESI calculated for [M+H]⁺ 324, found 324.

### Step 5

Compound **18-5** (295 mg, 912 µmol) was dissolved in dioxane (2 mL), and a solution of hydrogen chloride in dioxane (4 M, 1.00 mL) was added thereto. The reaction mixture was stirred and reacted at 25°C for 6 hours. The reaction mixture was concentrated under reduced pressure, and the residue was purified by silica gel column chromatography (dichloromethane/methanol, 20/1 to 10/1, V/V) to obtain the hydrochloride of compound **18-6.** MS-ESI calculated for [M+H]⁺ 220, found 220.

### Step 6

The hydrochloride of compound **18-6** (452 mg, 738 µmol) was dissolved in dimethyl sulfoxide (10 mL) under nitrogen atmosphere, and compound **4-5** (194 mg, 885 µmol) and triethylamine (1.12 g, 1.54 mmol) were added thereto. The reaction mixture was stirred and reacted at 90°C for 12 hours. The reaction mixture was added with water (5 mL), extracted with ethyl acetate (5 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Phenomenx Synergi C18 150 × 30 mm× 4 µm; mobile phase: 0.05% hydrochloric acid aqueous solution-acetonitrile; gradient: acetonitrile 10% to 80%, 9 min) to obtain the hydrochloride of compound **18.** ¹H NMR (400 MHz, CD₃OD) δ 8.53 - 8.44 (m, 1H), 7.67 (t, *J*=6.8 Hz, 1H), 7.51 (t, *J*=6.8 Hz, 1H), 7.26 (t, *J*=8.0 Hz, 1H), 7.23 - 7.19 (m, 1H), 6.00 (d, *J*=7.2 Hz, 1H), 4.83 - 4.59 (m, 1H), 4.50 - 4.18 (m, 1H), 4.07 - 3.97 (m, 5H), 3.77 - 3.50 (m, 3H), 3.46 -3.34 (m, 1H), 3.20 - 15 (m, 1H), 3.00 (s, 3H), 2.63 (s, 3H), 1.75 (d, *J*=6.8 Hz, 3H), 1.66 - 1.44 (m, 3H). MS-ESI calculated for [M+H]⁺ 548, found 548.

### Embodiment 19

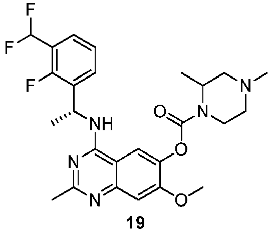

### Synthetic route:

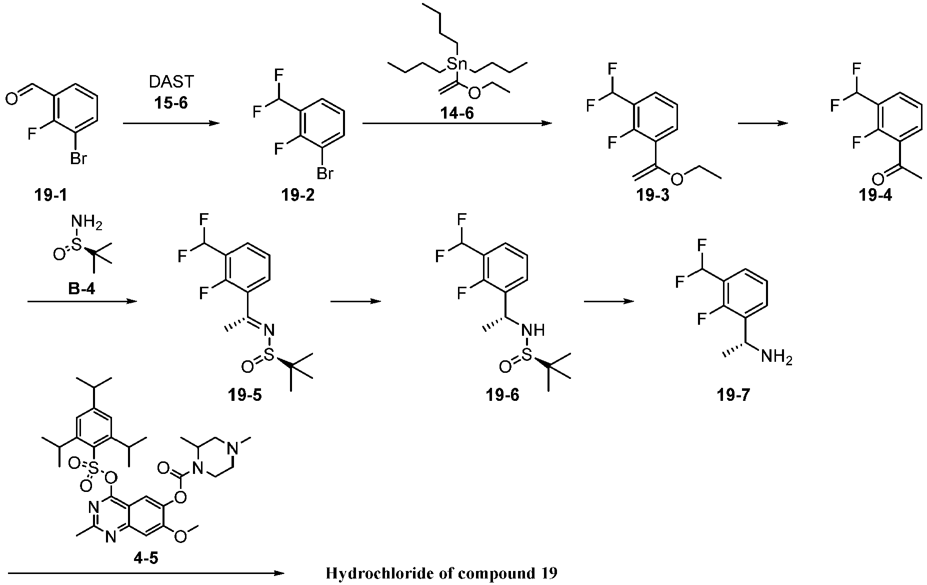

### Step 1

Compound **19-1** (3.00 g, 14.8 mmol) was dissolved in dry dichloromethane (30 mL) under nitrogen atmosphere, and diethylaminosulfur trifluoride (3.57 g, 22.2 mmol) was added dropwise thereto at 0°C. The reaction mixture was stirred at 25°C for 12 hours, added with ice water (20 mL), and extracted with dichloromethane (20 mL × 1). The organic phase was washed with saturated brine (20 mL × 1), dried over anhydrous sodium sulfate, filtered, concentrated under reduced pressure, and the residue was separated by silica gel column chromatography (petroleum ether/ethyl acetate, 100/1 to 10/1, V/V) to obtain compound **19-2.** ¹H NMR (400MHz, CD₃Cl) δ 7.72 - 7.67 (m, 1H), 7.52 - 7.59 (m, 1H), 7.16 (t, *J*=7.6 Hz, 1H), 7.05 - 6.75 (m, 1H).

### Step 2

Compound **19-2** (3.10 g, 13.8 mmol) was dissolved in dry toluene (50 mL) under nitrogen atmosphere. Tributyl(1-ethoxyvinyl)tin (compound **14-6**) (9.95 g, 27.6 mmol) was added thereto, and then bis(triphenylphosphine)palladium(II) dichloride (967 mg, 1.38 mmol) was added thereto. The reaction mixture was stirred at 110°C for 12 hours, added with saturated potassium fluoride aqueous solution (200 mL), and extracted with ethyl acetate (200 mL × 1). The organic phase was dried over anhydrous sodium sulfate, filtered, and concentrated under reduced pressure to obtain crude compound **19-3,** which was directly used in the next step.

### Step 3

Compound **19-3** (2.98 g, 13.8 mmol) was dissolved in acetone (90 mL) under nitrogen atmosphere, then concentrated hydrochloric acid (12 M, 9.19 mL) was added dropwise thereto at 0°C, and the reaction mixture was stirred at 25°C for 1 hour. The pH of the reaction mixture was alkalized to 8 with saturated sodium bicarbonate aqueous solution, and the reaction mixture was extracted with ethyl acetate (200 mL × 2). The organic phase was washed with saturated brine (200 mL × 1), dried over anhydrous sodium sulfate, filtered, concentrated under reduced pressure, and the residue was separated by silica gel column chromatography (petroleum ether/ethyl acetate, 100/1 to 10/1, V/V) to obtain compound **19-4.** ¹H NMR (400MHz, CD₃Cl) δ 8.06 - 7.97 (m, 1H), 7.84 - 7.76 (m, 1H), 7.38 - 7.32 (m, 1H), 7.10 - 6.80 (m, 1H), 2.68 (d, *J*=4.8 Hz, 3H).

### Step 4

Compound **19-4** (1.85 g, 9.83 mmol) was dissolved in dry tetrahydrofuran (50 mL) under nitrogen atmosphere, then tetraethyl titanate (8.97 g, 39.3 mmol) and (*R*)-(+)-tert-butylsulfinamide (compound **B-4**) (2.38 g, 19.7 mmol) were added thereto. The reaction mixture was stirred at 72°C for 36 hours, cooled to 0°C and added dropwise with saturated ammonium chloride aqueous solution (20 mL), then added with ethyl acetate ( 20 mL), and filtered. The filter cake was washed with ethyl acetate (10 mL), and the organic phase was washed with saturated brine (20 mL × 1), dried over anhydrous sodium sulfate, filtered, concentrated under reduced pressure, and the residue was separated by silica gel column chromatography (petroleum ether/ethyl acetate, 100/1 to 4/1, V/V) to obtain compound **19-5.** MS-ESI calculated for [M+H]⁺ 292, found 292.

### Step 5

Compound **19-5** (2.33 g, 8.00 mmol) was dissolved in anhydrous tetrahydrofuran (20 mL), then sodium borohydride (303 mg, 8.00 mmol) was added thereto. The reaction mixture was stirred at 25°C for 1 hour, cooled to 0°C and added dropwise with saturated ammonium chloride aqueous solution (50 mL), then added with ethyl acetate (50 mL). The organic phase was washed with saturated brine (50 mL × 1), dried over anhydrous sodium sulfate, filtered, and concentrated under reduced pressure to obtain compound **19-6.** MS-ESI calculated for [M+H]⁺ 294, found 294.

### Step 6

Compound **19-6** (200 mg, 0.682 mmol) was dissolved in ethyl acetate (5 mL), then a solution of hydrogen chloride in ethyl acetate (4 M, 5.00 mL) was added dropwise thereto at 25°C. The reaction mixture was stirred at 25°C for 2 hours and concentrated under reduced pressure to obtain the hydrochloride of crude compound **19-7,** which was directly used in the next step.

### Step 7

The hydrochloride of compound **19-7** (200 mg, 0.326 mmol) was dissolved in dimethyl sulfoxide (10 mL) under nitrogen atmosphere, then compound 7 (110 mg, 0.490 mmol) and triethylamine (165 mg, 1.63 mmol) were added thereto. The reaction mixture was stirred at 100°C for 12 hours, and separated by preparative high performance liquid chromatography (chromatographic column: Phenomenex Synergi C18 150 × 30 mm × 4 µm; mobile phase: 0.05% hydrochloric acid aqueous solution-acetonitrile; gradient: acetonitrile 25% to 55%, 9 min) to obtain the hydrochloride of compound **19.** ¹H NMR (400MHz, CD₃OD) δ 8.46 (s, 1H), 7.74 - 7.65 (m, 1H), 7.58 - 7.49 (m, 1H), 7.30 (t, *J*=8.0 Hz, 1H), 7.19 (s, 1H), 7.15 - 6.85 (m, 1H), 6.00 (q, *J*=7.2 Hz, 1H), 4.84 - 4.58 (m, 1H), 4.51 - 4.20 (m, 1H), 4.04 (s, 3H), 3.74 - 3.49 (m, 3H), 3.48 - 3.35 (m, 1H), 3.29 - 3.15 (m, 1H), 3.00 (s, 3H), 2.63 (s, 3H), 1.76 (d, *J*=7.2 Hz, 3H), 1.65 - 1.44 (m, 3H). MS-ESI calculated for [M+H]⁺ 518, found 518.

### Example 20

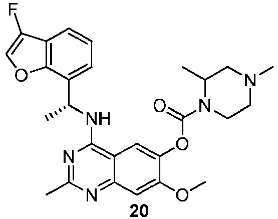

### Synthetic route:

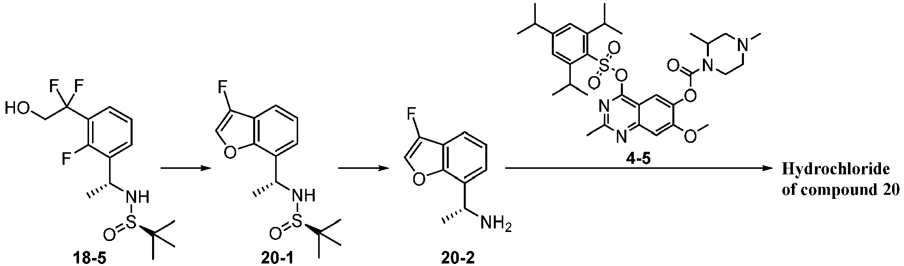

### Step 1

Compound **18-5** (121 mg, 374 µmol) was dissolved in dioxane (2 mL), and cesium carbonate (366 mg, 1.12 mmol) and 18-crown-6 (50 mg, 187 µmol) were added thereto. The reaction mixture was stirred and reacted at 80°C for 36 hours. The reaction mixture was concentrated under reduced pressure, and the residue was purified by silica gel column chromatography (petroleum ether/ethyl acetate, 15/1 to 3/1, V/V) to obtain the hydrochloride of compound **20-1.** MS-ESI calculated for [M+H]⁺ 284, found 284.

### Step 2

Compound **20-1** (104 mg, 367 µmol) was dissolved in dioxane (1 mL), and a solution of hydrogen chloride in dioxane (4 M, 404 µL) was added thereto. The reaction mixture was stirred and reacted at 25°C for 6 hours. The reaction mixture was concentrated under reduced pressure, and the residue was purified by silica gel column chromatography (dichloromethane/methanol, 20/1 to 10/1, V/V) to obtain the hydrochloride of compound **20-2.** MS-ESI calculated for [M+H]⁺ 180, found 180.

### Step 3

The hydrochloride of compound **20-2** (177 mg, 288 µmol) was dissolved in dimethyl sulfoxide (2 mL) under nitrogen atmosphere, and compound **4-5** (62 mg, 346 µmol) and triethylamine (437 mg, 4.33 mmol) were added thereto. The reaction mixture was stirred and reacted at 90°C for 12 hours. The reaction mixture was added with water (3 mL), extracted with ethyl acetate (3 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Phenomenx Synergi C18 150 × 30 mm× 4 µm; mobile phase: 0.05% hydrochloric acid aqueous solution-acetonitrile; gradient: acetonitrile 20% to 50%, 9 min) to obtain the hydrochloride of compound **20.** ¹H NMR (400 MHz, CD₃OD) δ 8.45 (s, 1H), 7.93 - 7.87 (m, 1H), 7.54 (d, *J*=8.0 Hz, 1H), 7.47 (d, *J*=7.2 Hz, 1H), 7.33 - 7.27 (m, 1H), 7.19 (s, 1H), 6.23 (d, *J*=6.8 Hz, 1H), 4.74 -4.57 (s, 1H), 4.46 - 4.22 (m, 1H), 4.04 (s, 3H), 3.59 (t, *J*=12.4 Hz, 3H), 3.46 - 3.35 (s, 1H), 3.27 -3.15 (s, 1H), 3.00 (s, 3H), 2.59 (s, 3H), 1.83 (d, *J*=7.2 Hz, 3H), 1.62 - 1.45 (m, 3H). MS-ESI calculated for [M+H]⁺ 508, found 508.

### Example 21

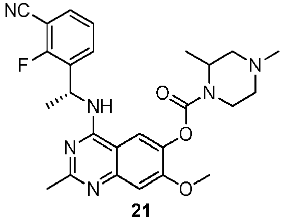

### Synthetic route:

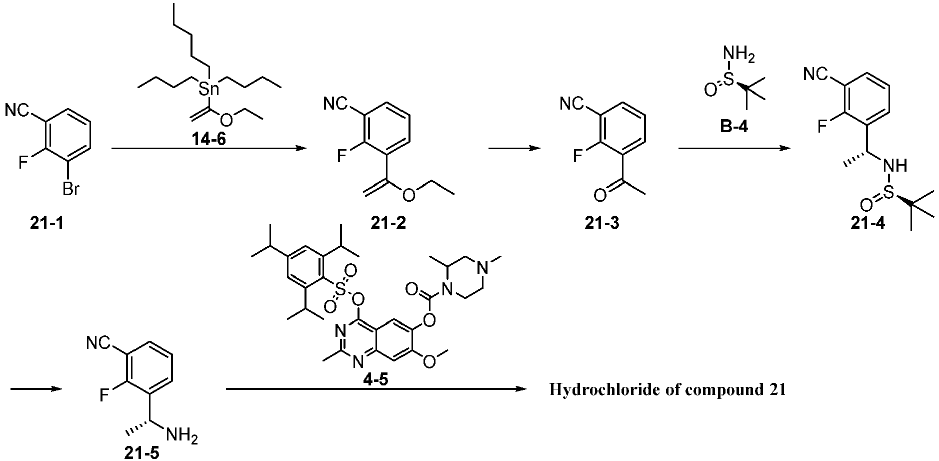

### Step 1

Compound **21-1** (1.00 g, 5.00 mmol) was dissolved in toluene (2 mL) under nitrogen atmosphere, then compound **14-6** (3.61 g, 10.0 mmol) and bis(triphenylphosphine)palladium(II) dichloride (351 mg, 0.50 mmol) were added thereto, and the reaction mixture was stirred and reacted at 120°C for 12 hours. The reaction mixture was added with saturated potassium fluoride solution (10 mL), extracted with ethyl acetate (5 mL × 2), filtered, and the filtrate was concentrated under reduced pressure to obtain compound **21-2.**

### Step 2

Compound **21-2** (950 mg, 4.97 mmol) was dissolved in acetone (30 mL) under nitrogen atmosphere, then hydrochloric acid solution (12 M, 3.31 mL) was added dropwise thereto at 0°C. The reaction mixture was stirred and reacted at 25°C for 1 hour. The pH of the reaction mixture was alkalized to 8 with saturated sodium bicarbonate solution, and the reaction mixture was extracted with ethyl acetate (30 mL × 2). The organic phase was washed with saturated brine (20 mL), dried over anhydrous sodium sulfate, filtered, and concentrated under reduced pressure, and the residue was purified by silica gel column chromatography (petroleum ether/ethyl acetate, 50/1 to 10/1, V/V) to obtain compound **21-3.**

### Step 3

Compound **21-3** (736 mg, 4.51 mmol) was dissolved in tetrahydrofuran (15 mL), then compound **B-4** (820 mg, 6.77 mmol) and tetraethyl titanate (3.09 g, 13.5 mmol) were added thereto, and the reaction mixture was stirred at 80°C for 14 hours. The reaction mixture was added with sodium borohydride (171 mg, 4.51 mmol) at -5°C, then stirred and reacted at 25°C for 1 hour. The reaction mixture was poured into ice water, filtered, and the filtrate was extracted with ethyl acetate (10 mL × 2). The organic phase was washed with saturated brine (10 mL), dried over anhydrous sodium sulfate, filtered, and concentrated under reduced pressure, and the residue was purified by silica gel column chromatography (petroleum ether/ethyl acetate, 2/1 to 1/1, V/V) to obtain compound **21-4.** MS-ESI calculated for [M+H]⁺ 269, found 269.

### Step 4

Compound **21-4** (910 mg, 3.39 mmol) was dissolved in dioxane (8 mL), and a solution of hydrogen chloride in dioxane (4 M, 3.73 mL) was added thereto. The reaction mixture was stirred and reacted at 25°C for 6 hours. The reaction mixture was concentrated under reduced pressure, and the residue was stirred with ethyl acetate (2 mL), filtered, and the filter cake was collected to obtain the hydrochloride of compound **21-5.** MS-ESI calculated for [M+H]⁺ 165, found 165.

### Step 5

The hydrochloride of compound **21-5** (198 mg, 291 µmol) was dissolved in dimethyl sulfoxide (2 mL) under nitrogen atmosphere, and compound **4-5** (70 mg, 249 µmol) and triethylamine (441 mg, 4.36 mmol) were added thereto. The reaction mixture was stirred and reacted at 90°C for 12 hours. The reaction mixture was added with water (3 mL), extracted with ethyl acetate (3 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Phenomenx Synergi C18 150 × 30 mm× 4 µm; mobile phase: 0.05% hydrochloric acid aqueous solution-acetonitrile; gradient: acetonitrile 20% to 50%, 9 min) to obtain the hydrochloride of compound **21.** ¹H NMR (400 MHz, CD₃OD) δ 8.45 (s, 1H), 7.93 - 7.83 (m, 1H), 7.75 - 7.65 (m, 1H), 7.41 - 7.32 (m, 1H), 7.18 (s, 1H), 5.96 (d, *J*=7.2 Hz, 1H), 4.80 - 4.62 (m, 1H), 4.58 - 4.15 (m, 1H), 4.04 (s, 3H), 3.78 - 3.44 (m, 3H), 3.44 - 3.34 (m, 1H), 3.25 - 3.10 (m, 1H), 3.00 (s, 3H), 2.63 (s, 3H), 1.76 (d, *J*=7.2 Hz, 3H), 1.61 - 1.43 (m, 3H). MS-ESI calculated for [M+H]⁺ 493, found 493.

### Example 22

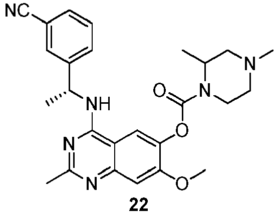

### Synthetic route:

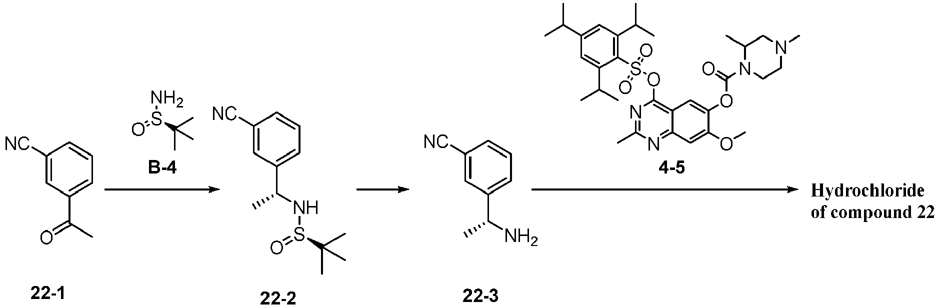

### Step 1

Compound **22-1** (600 mg, 4.13 mmol) was dissolved in tetrahydrofuran (10 mL), then compound **B-4** (751 mg, 6.20 mmol) and tetraethyl titanate (2.83 g, 12.4 mmol) were added thereto, and the reaction mixture was stirred and reacted at 80°C for 16 hours. The reaction mixture was added with sodium borohydride (156 mg, 4.13 mmol) at -5°C, then stirred and reacted at 25°C for 1 hour. The reaction mixture was poured into ice water, filtered, and the filtrate was extracted with ethyl acetate (5 mL × 2). The organic phase was washed with saturated brine (5 mL), dried over anhydrous sodium sulfate, filtered, and concentrated under reduced pressure, and the residue was purified by silica gel column chromatography (petroleum ether/ethyl acetate, 6/1 to 1/1, V/V) to obtain compound **22-2.** MS-ESI calculated for [M+H]⁺ 251, found 251.

### Step 2

Compound **22-2** (500 mg, 2.00 mmol) was dissolved in dioxane (8 mL), and a solution of hydrogen chloride in dioxane (4 M, 2 mL) was added thereto. The reaction mixture was stirred and reacted at 25°C for 6 hours. The reaction mixture was concentrated under reduced pressure, and the residue was stirred with ethyl acetate (2 mL), filtered, and the filter cake was collected to obtain the hydrochloride of compound **22-3.** MS-ESI calculated for [M+H]⁺ 147, found 147.

### Step 3

The hydrochloride of compound **22-3** (218 mg, 319 µmol) was dissolved in dimethyl sulfoxide (2 mL) under nitrogen atmosphere, and compound **4-5** (70 mg, 383 µmol) and triethylamine (441 mg, 4.36 mmol) were added thereto. The reaction mixture was stirred and reacted at 90°C for 12 hours. The reaction mixture was added with water (3 mL), extracted with ethyl acetate (3 mL × 3) and dichloromethane/methanol solution (8:1, 5 mL), and the organic phase was dried over anhydrous sodium sulfate, filtered, and concentrated under reduced pressure. The residue was purified by preparative high performance liquid chromatography (chromatographic column: Phenomenex Genimi NX C18 150 × 40 mm× 5 µm; mobile phase: 0.05% hydrochloric acid aqueous solution-acetonitrile; gradient: acetonitrile 1% to 30%, 10 min) to obtain the hydrochloride of compound **22.** ¹H NMR (400 MHz, CD₃OD) δ 8.47 (s, 1H), 7.91 - 7.75 (m, 2H), 7.67 - 7.61 (s, 1H), 7.57 - 7.49 (s, 1H), 7.22 (s, 1H), 5.90 - 5.76 (s, 1H), 4.65 - 4.18 (m, 2H), 4.04 (s, 3H), 3.77 - 3.51 (m, 2H), 3.49 - 3.32 (m, 2H), 3.26 - 3.14 (m, 1H), 3.00 (s, 3H), 2.65 (s, 3H), 1.74 (s, 3H), 1.65 - 1.32 (m, 3H). MS-ESI calculated for [M+H]⁺ 475, found 475.

### Example 23

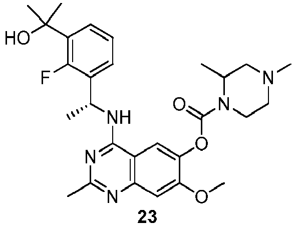

### Synthetic route:

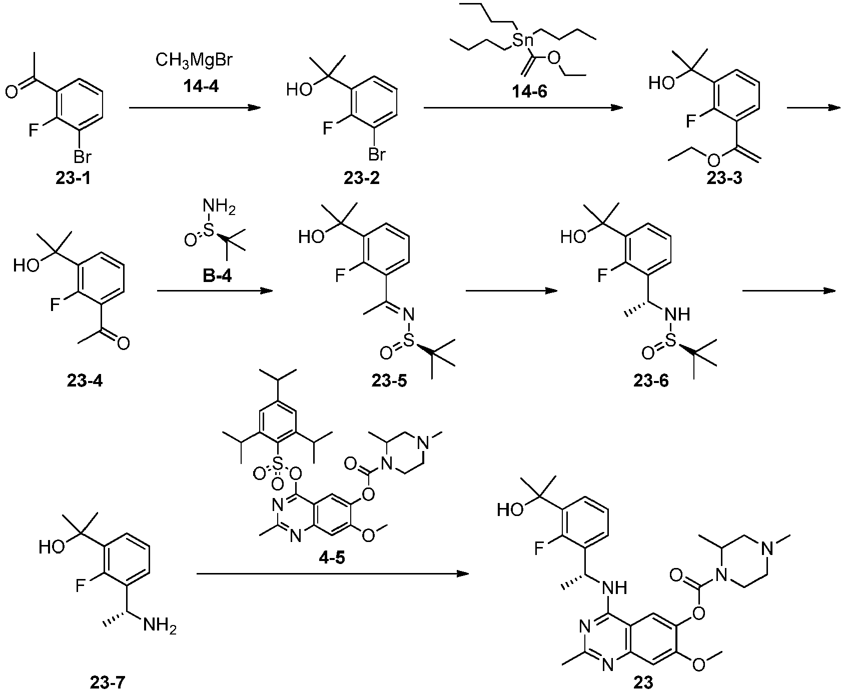

### Step 1

Compound **23-1** (1.00 g, 4.61 mmol) was dissolved in tetrahydrofuran (15 mL) under nitrogen atmosphere, then compound **14-4** (3 M tetrahydrofuran solution, 7.68 mL) was added dropwise thereto at 0°C, and the reaction mixture was stirred and reacted at 25°C for 1 hour. The reaction mixture was added with saturated ammonium chloride solution and extracted with ethyl acetate (50 mL × 2). The organic phase was washed with saturated brine (50 mL × 2), dried over anhydrous sodium sulfate, filtered, and concentrated under reduced pressure, and the residue was purified by silica gel column chromatography (petroleum ether/ethyl acetate, 10/1 to 2/1, V/V) to obtain compound **23-2.** ¹H NMR (400MHz, CD₃Cl) δ 7.59 - 7.50(m, 1H), 7.50 - 7.37(m, 1H), 7.06 - 6.97(m, 1H), 1.67 - 1.64(m, 6H).

### Step 2

Compound **23-2** (606 mg, 2.60 mmol) was dissolved in toluene (10 mL) under nitrogen atmosphere, then compound **14-6** (1.13 g, 3.12 mmol) and bis(triphenylphosphine)palladium(II) dichloride (182 mg, 260 µmol) were added thereto, and the reaction mixture was stirred and reacted at 120°C for 12 hours. The reaction mixture was added with saturated potassium fluoride solution (20 mL) and extracted with ethyl acetate (50 mL × 2), and the organic phase was dried over anhydrous sodium sulfate, filtered, and concentrated under reduced pressure to obtain compound **23-3.** MS-ESI calculated for [M+H]⁺ 225, found 225.

### Step 3

Compound **23-3** (583 mg, 2.60 mmol) was dissolved in acetone (6 mL) under nitrogen atmosphere, then hydrochloric acid solution (12 M, 0.60 mL) was added dropwise thereto, and the reaction mixture was stirred and reacted at 20°C for 1 hour. The pH of the reaction mixture was alkalized to 8 with saturated sodium bicarbonate solution, and the reaction mixture was extracted with ethyl acetate (20 mL × 1). The organic phase was washed with saturated brine (20 mL × 1), dried over anhydrous sodium sulfate, filtered, and concentrated under reduced pressure, and the residue was purified by silica gel column chromatography (petroleum ether/ethyl acetate, 10/1 to 2/1, V/V) to obtain compound **23-4.** ¹H NMR (400MHz, CD₃Cl) δ 7.85 - 7.77 (m, 1H), 7.78 - 7.67 (m, 1H), 7.20 (t, *J*=7.6 Hz, 1H), 2.68 - 2.63 (m, 3H), 1.70 - 1.67(m, 6H).

### Step 4

Compound **23-4** (430 mg, 2.19 mmol) was dissolved in tetrahydrofuran (10 mL), and then compound **B-4** (398 mg, 3.29 mmol) and tetraethyl titanate (1.00 g, 4.38 mmol) were added thereto. The reaction mixture was stirred and reacted at 80°C for 12 hours, then added with water (50 mL), and extracted with ethyl acetate (50 mL × 1). The organic phase was washed with saturated brine (50 mL × 1), dried over anhydrous sodium sulfate, filtered, concentrated under reduced pressure, and the residue was purified by silica gel column chromatography (petroleum ether/ethyl acetate, 10/1 to 1/1, V/V) to obtain compound **23-5.** ¹H NMR (400MHz, CD₃Cl) δ 7.75 - 7.65 (m, 1H), 7.58 - 7.41 (m, 1H), 7.16 (t, *J*=7.6 Hz, 1H), 1.68 - 1.66 (m, 6H), 1.32 (s, 9H), 1.24 (s, 3H). MS-ESI calculated for [M+H]⁺ 300, found 300.

### Step 5

Compound **23-5** (650 mg, 1.85 mmol) was dissolved in tetrahydrofuran (10 mL), then sodium borohydride (77.1 mg, 2.04 mmol) was added thereto at 0°C, and the reaction mixture was stirred and reacted at 20°C for 1 hour. The reaction mixture was added with water (50 mL) and extracted with ethyl acetate (50 mL × 1). The organic phase was washed with saturated brine (50 mL × 1), dried over anhydrous sodium sulfate, filtered, and concentrated under reduced pressure, and the residue was purified by silica gel column chromatography (petroleum ether/ethyl acetate, 10/1 to 1/1, V/V) to obtain compound **23-6.** ¹H NMR (400MHz, CD₃Cl) δ 7.54 - 7.44 (m, 1H), 7.34 - 7.25 (m, 1H), 7.12 (t, *J*=7.6 Hz, 1H), 4.99 - 4.69 (m, 1H), 1.68 - 1.60 (m, 6H), 1.61- 1.50 (m, 3H), 1.27 - 1.15 (m, 9H). MS-ESI calculated for [M+H]⁺ 302, found 302.

### Step 6

Compound **23-6** (116 mg, 385 µmol) was dissolved in dioxane (5 mL), and a solution of hydrogen chloride in dioxane (4 M, 96.2 µL) was added thereto. The reaction mixture was stirred and reacted at 25°C for 1 hour. The reaction mixture was concentrated under reduced pressure, and the residue was stirred with dichloromethane (5 mL), filtered, and the filter cake was collected to obtain the hydrochloride of compound **23-7.** MS-ESI calculated for [M+H]⁺ 198, found 198.

### Step 7

The hydrochloride of compound **23-7** (69.0 mg, 295 µmol) was dissolved in dimethyl sulfoxide (3 mL), then compound **4-5** (199 mg, 325 µmol) was added thereto, and triethylamine (149 mg, 1.48 mmol) was added dropwise thereto. The reaction mixture was stirred and reacted at 100°C for 12 hours, filtered, and concentrated under reduced pressure. The residue was purified by preparative high performance liquid chromatography (chromatographic column: Phenomenex Genimi NX C18 150 × 40 mm × 5 µm; mobile phase: (0.04% ammonia aqueous solution +10 mM ammonium bicarbonate aqueous solution)-acetonitrile; gradient: acetonitrile 30% to 60%, 10 min) to obtain compound **23.** ¹H NMR (400MHz, CD₃OD) δ 8.04 (s, 1H), 7.53 - 7.42 (m, 1H), 7.35 - 7.24 (m, 1H), 7.09 (s, 1H), 7.08 - 6.98 (m, 1H), 5.91 - 5.77 (m, 1H), 4.68 - 4.55 (m, 1H), 4.56 - 4.17 (m, 1H), 3.94 (s, 3H), 2.96 - 2.74 (m, 2H), 2.42 (s, 3H), 2.31 (s, 3H), 2.26 - 2.01 (m, 2H), 1.80 - 1.49 (m, 9H), 1.48 - 1.37 (m, 3H), 1.37 - 1.10 (m, 2H). MS-ESI calculated for [M+H]⁺ 526, found 526.

### Example 24

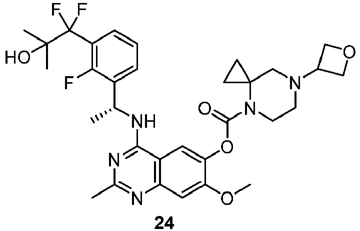

### Synthetic route:

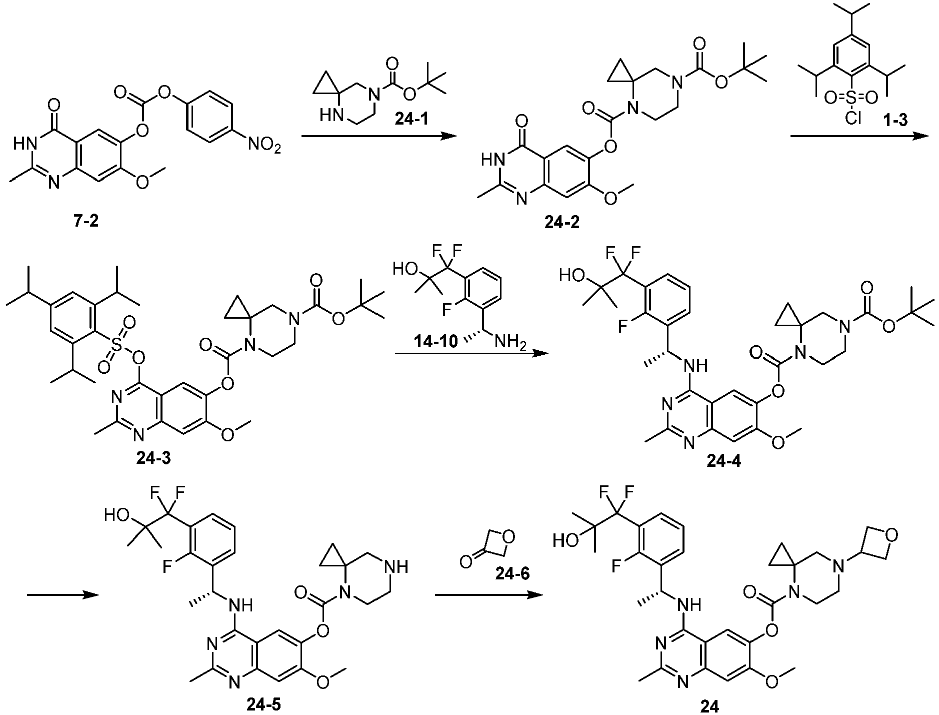

### Step 1

Compound **7-2** (200 mg, 1.62 mmol) was dissolved in *N,N*-dimethylformamide (5 mL), then triethylamine (1.12 mL, 8.08 mmol) and **24-1** (686 mg, 3.23 mmol) were added dropwise thereto, and the reaction mixture was stirred and reacted at 25°C for 12 hours. The reaction mixture was slowly added with water (20 mL) and extracted with ethyl acetate (20 mL × 3). The organic phase was washed with saturated brine (20 mL × 3), dried over anhydrous sodium sulfate, filtered, concentrated under reduced pressure, and the residue was subjected to silica gel column chromatography (dichloromethane/methanol, 100/1 to 10/1, V/V) to obtain compound **24-2.** MS-ESI calculated for [M+H]⁺ 445, found 445.

### Step 2

Compound **24-2** (425 mg, 839 µmol) was dissolved in dichloromethane (10 mL). Compound **1-3** (305 mg, 1.01 mmol), 4-dimethylaminopyridine (10.3 mg, 83.9 µmol), and triethylamine (350 µL, 2.52 mmol) were added thereto, and the reaction mixture was stirred and reacted at 25°C for 12 hours. The reaction mixture was filtered, concentrated under reduced pressure, and the residue was purified by silica gel column chromatography (dichloromethane/methanol, 100/1 to 10/1, V/V) to obtain compound **24-3.** MS-ESI calculated for [M+H]⁺ 711, found 711.

### Step 3

Compound **24-3** (220 mg, 309 µmol) was dissolved in dimethyl sulfoxide (5 mL). The hydrochloride of **14-10** (116 mg, 402 µmol) and triethylamine (129 µL, 928 µmol) were added thereto, and the reaction mixture was stirred and reacted at 90°C for 12 hours. The reaction mixture was slowly added with water (20 mL) and extracted with ethyl acetate (20 mL × 3). The organic phase was washed with saturated brine (20 mL × 5), dried over anhydrous sodium sulfate, filtered, concentrated under reduced pressure, and the residue was subjected to silica gel column chromatography (dichloromethane/methanol, 100/1 to 10/1, V/V) to obtain compound **24-4.** MS-ESI calculated for [M+H]⁺ 674, found 674.

### Step 4

Compound **24-4** (224 mg, 236 µmol) was dissolved in ethyl acetate (5 mL), then a solution of hydrogen chloride in ethyl acetate (4 mol/L, 3.53 mL) was added thereto, and the reaction mixture was stirred and reacted at 20°C for 12 hours. The reaction mixture was concentrated under reduced pressure to obtain the hydrochloride of compound **24-5.** MS-ESI calculated for [M+H]⁺ 574, found 574.

### Step 5

The hydrochloride of compound **24-5** (130 mg, 201 µmol) was dissolved in tetrahydrofuran (5 mL), then **24-6** (145 mg, 2.01 mmol) was added thereto. The reaction mixture was stirred and reacted at 70°C for 1 hour, then added with sodium triacetoxyborohydride (213 mg, 1.01 mmol), stirred and reacted at 70°C for 1 hour. The reaction mixture was cooled to room temperature and filtered, and the filtrate was concentrated under reduced pressure. The residue was purified by preparative high performance liquid chromatography (chromatographic column: Phenomenex C18 80 mm × 40 mm × 3 µm; mobile phase: 0.05% ammonia aqueous solution-acetonitrile; gradient: acetonitrile 40% to 70%, 8 min) to obtain compound **24.** ¹H NMR (400 MHz, CD₃OD) δ 8.05 (s, 1H), 7.57 - 7.46 (m, 1H), 7.40 - 7.31 (m, 1H), 7.18 - 7.11 (m, 1H), 7.12 - 7.08 (m, 1H), 5.87 - 5.78 (m, 1H), 4.75 - 4.67 (m, 2H), 4.64 - 4.59 (m, 6H), 3.97 - 3.90 (m, 3H), 3.91 - 3.63 (m, 2H), 3.64 - 3.52 (m, 1H), 2.41 (s, 3H), 1.63 (d, *J*=7.2 Hz, 3H), 1.33 - 1.27 (m, 6H), 1.23 - 0.86 (m, 4H). MS-ESI calculated for [M+H]⁺ 630, found 630.

### Example 25

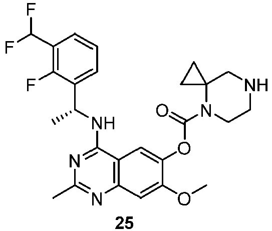

### Synthetic route:

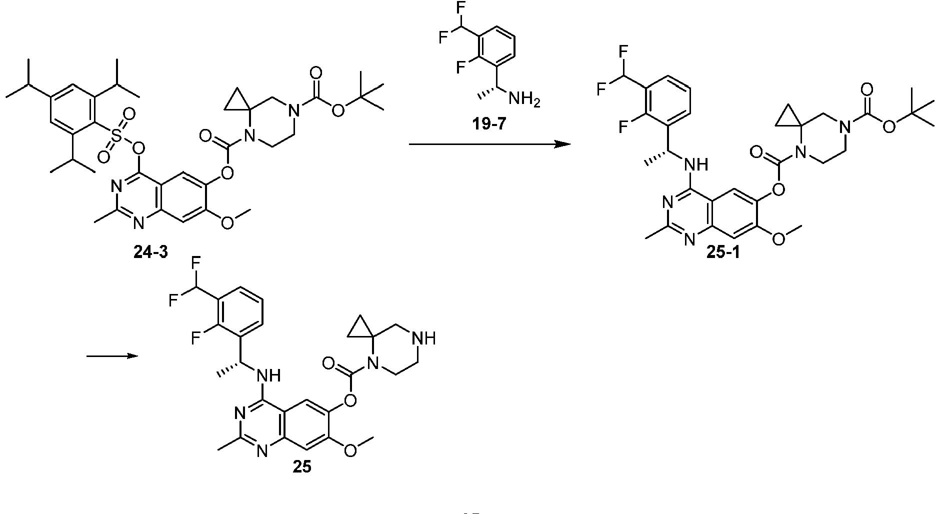

### Step 1

Compound **24-3** (1.01 g, 1.18 mmol) was dissolved in dimethyl sulfoxide (10 mL). The hydrochloride of **19-7** (245 mg, 1.30 mmol) and triethylamine (492 µL, 3.54 mmol) were added thereto, and the reaction mixture was stirred and reacted at 90°C for 12 hours. The reaction mixture was slowly added with water (20 mL) and extracted with ethyl acetate (20 mL × 3). The organic phase was washed with saturated brine (20 mL × 5), dried over anhydrous sodium sulfate, filtered, concentrated under reduced pressure, and the residue was subjected to silica gel column chromatography (dichloromethane/methanol, 100/1 to 10/1, V/V) to obtain compound **25-1.** MS-ESI calculated for [M+H]⁺ 616, found 616.

### Step 2

Compound **25-1** (675 mg, 900 µmol) was dissolved in ethyl acetate (10 mL), and a solution of hydrogen chloride in ethyl acetate (4 mol/L, 13.5 mL) was added thereto. The reaction mixture was stirred and reacted at 20°C for 12 hours. The reaction mixture was concentrated under reduced pressure, and the residue was purified by preparative high performance liquid chromatography (chromatographic column: Xtimate C18 150 mm × 40 mm × 5 µm; mobile phase: 0.05% ammonia aqueous solution-acetonitrile; gradient: acetonitrile 35% to 65%, 10 min) to obtain compound **25.** ¹H NMR (400MHz, CD₃OD) δ 8.05 (s, 1H), 7.61 - 7.48 (m, 1H), 7.43 (t, *J*=6.4 Hz, 1H), 7.29 - 7.09 (m, 2H), 7.10 - 6.81 (m, 1H), 5.87 - 5.73 (m, 1H), 3.96 (s, 3H), 3.90 - 3.50 (m, 2H), 3.15 - 2.70 (m, 4H), 2.40 (s, 3H), 1.62 (d, *J*=6.8 Hz, 3H), 1.40 - 1.05 (m, 2H), 1.01 - 0.84 (m, 2H). MS-ESI calculated for [M+H]⁺ 516, found 516.

### Example 26

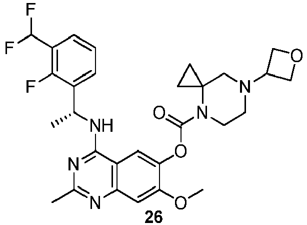

### Synthetic route:

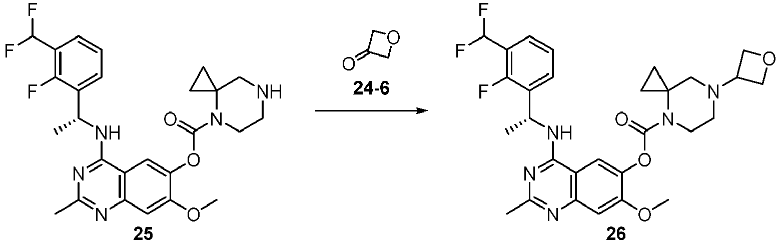

Compound **25** (280 mg, 476 µmol) was dissolved in tetrahydrofuran (5 mL), then **24-6** (343 mg, 4.76 mmol) was added thereto. The reaction mixture was stirred and reacted at 70°C for 1 hour, then added with sodium triacetoxyborohydride (504 mg, 2.38 mmol), stirred and reacted at 70°C for 1 hour. The reaction mixture was cooled to room temperature, filtered, and concentrated under reduced pressure. Then the resulting residue was purified by preparative high performance liquid chromatography (chromatographic column: Xtimate C18 150 mm × 40 mm × 5 µm; mobile phase: ammonia aqueous solution-acetonitrile; gradient: acetonitrile 40% to 60%, 10 min) to obtain compound **26.** ¹H NMR (400 MHz, CD₃OD) δ 8.04 (s, 1H), 7.57 - 7.47 (m, 1H), 7.47 - 7.33 (m, 1H), 7.28 - 7.08 (m, 2H), 7.08 - 6.75 (m, 1H), 5.86 - 5.70 (m, 1H), 4.74 - 4.54 (m, 5H), 3.93 (s, 3H), 3.89 - 3.63 (m, 2H), 3.62 - 3.46 (m, 1H), 2.69 - 2.43 (m, 2H), 2.40 (s, 3H), 2.37 - 2.30 (m, 1H), 1.62 (d, *J*=6.8 Hz, 3H), 1.39 - 1.08 (m, 2H), 1.04 - 0.86 (m, 2H). MS-ESI calculated for [M+H]⁺ 572, found 572.

### Example 27

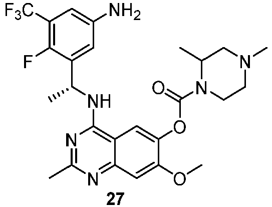

### Synthetic route:

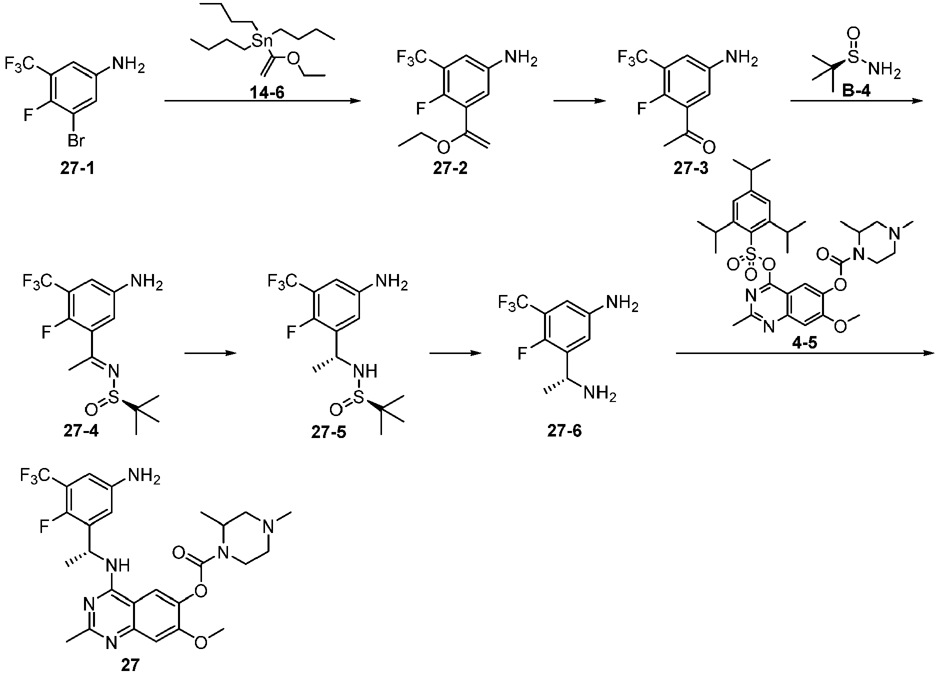

### Step 1

Compound **27-1** (6.00 g, 23.3 mmol) was dissolved in dry toluene (100 mL) under nitrogen atmosphere. Compound **14-6** (12.6 g, 34.9 mmol) was added thereto, and then bis(triphenylphosphine)palladium(II) dichloride (1.63 g, 2.33 mmol) was added thereto. The reaction mixture was stirred at 120°C for 12 hours, cooled to room temperature, then added with saturated potassium fluoride aqueous solution (100 mL), and extracted with ethyl acetate (100 mL × 2). The organic phase was dried over anhydrous sodium sulfate, filtered, and concentrated under reduced pressure to obtain crude compound **27-2,** which was directly used in the next step.

### Step 2

Compound **27-2** (5.83 g, 23.4 mmol) was dissolved in acetone (80 mL), then concentrated hydrochloric acid (12 M, 9.96 mL) was added dropwise thereto at 0°C, and the reaction mixture was stirred at 25°C for 1 hour. The pH of the reaction mixture was alkalized to 8 with saturated sodium bicarbonate aqueous solution, and the reaction mixture was extracted with ethyl acetate (50 mL × 3). The organic phase was washed with saturated brine (50 mL × 1), dried over anhydrous sodium sulfate, filtered, concentrated under reduced pressure, and the residue was separated by silica gel column chromatography (petroleum ether/ethyl acetate, 100/1 to 4/1, V/V) to obtain compound **27-3.** ¹HNMR (400 MHz, CDCl₃) δ 7.30 - 7.26 (m, 1H), 7.08 -7.00 (m, 1H), 2.66 - 2.63 (m, 3H). MS-ESI calculated for [M+H]⁺ 222, found 222.

### Step 3

Compound **27-3** (4.84 g, 16.7 mmol) was dissolved in dry tetrahydrofuran (50 mL), then tetraethyl titanate (7.62 g, 33.4 mmol) and compound **B-4** (3.04 g, 25.1 mmol) were added thereto. The reaction mixture was stirred at 80°C for 24 hours, cooled to 0°C. Water (50 mL) was poured into the reaction mixture, and then the mixture was washed with ethyl acetate (50 mL × 1). The organic phase was washed with saturated brine (50 mL × 1), dried over anhydrous sodium sulfate, filtered, concentrated under reduced pressure, and the residue was separated by silica gel column chromatography (petroleum ether/ethyl acetate, 100/1 to 2/1, V/V) to obtain compound **27-4.** MS-ESI calculated for [M+H]⁺ 325, found 325.

### Step 4

Compound **27-4** (4.68 g, 11.8 mmol) was dissolved in anhydrous tetrahydrofuran (60 mL), then sodium borohydride (535 mg, 14.2 mmol) was added thereto at 0°C. The reaction mixture was stirred at 25°C for 1 hour, cooled to 0°C, added dropwise with water (100 mL), and then added with ethyl acetate (100 mL × 1). The organic phase was washed with saturated brine (200 mL × 1), dried over anhydrous sodium sulfate, filtered, concentrated under reduced pressure, and the residue was separated by silica gel column chromatography (petroleum ether/ethyl acetate, 1/1 to 1/2, V/V) to obtain compound **27-5.** MS-ESI calculated for [M+H]⁺ 327, found 327.

### Step 5

Compound **27-5** (2.16 g, 6.62 mmol) was dissolved in dioxane (30 mL), then a solution of hydrogen chloride in dioxane (4 M, 16.6 mL) was added dropwise thereto, and the reaction mixture was stirred at 20°C for 2 hours. After the reaction mixture was concentrated under reduced pressure, dichloromethane (5 mL) was added thereto at 20°C, and the resulting reaction mixture was stirred for 10 minutes and filtered. The filter cake was collected and dried to obtain the hydrochloride of intermediate **27-6.** MS-ESI calculated for [M+H]⁺ 223, found 223.

### Step 6

Compound **4-5** (100 mg, 163 µmol) was dissolved in dimethyl sulfoxide (3 mL). The hydrochloride of **27-6** (43.5 mg, 196 µmol) and triethylamine (68.1 µL, 490 µmol) were added thereto, and the reaction mixture was stirred and reacted at 90°C for 12 hours. The reaction mixture was cooled to room temperature, filtered, and concentrated under reduced pressure. The residue was purified by high performance liquid chromatography (chromatographic column: Xtimate C18 150 mm × 25 mm × 5 µm; mobile phase: 0.05% hydrochloric acid aqueous solution-acetonitrile; gradient: acetonitrile 5% to 35%, 10 min), and then separated by preparative SFC (separation column: DAICEL CHIRALCEL OD 250 mm × 30 mm × 10 µm; mobile phase: supercritical CO₂-0.1% ammonia water in ethanol; gradient: 0.1% ammonia water in ethanol: 35% to 35%) to obtain compound **27.** ¹H NMR (400 MHz, CD₃OD) δ 8.36 (s, 1H), 7.16 (s, 1H), 6.96 (s, 1H), 6.83 (s, 1H), 5.92 - 5.81 (m, 1H), 4.29 - 4.07 (m, 1H), 4.03 (s, 3H), 3.55 - 3.39 (m, 1H), 3.31 - 3.03 (m, 4H), 2.94 - 2.72 (m, 1H), 2.67 (s, 3H), 2.61 (s, 3H), 1.70 (d, *J*=7.2 Hz, 3H), 1.56 - 1.43 (m, 3H). MS-ESI calculated for [M+H]⁺ 551, found 551.

### Example 28

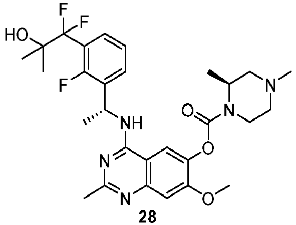

### Synthetic route:

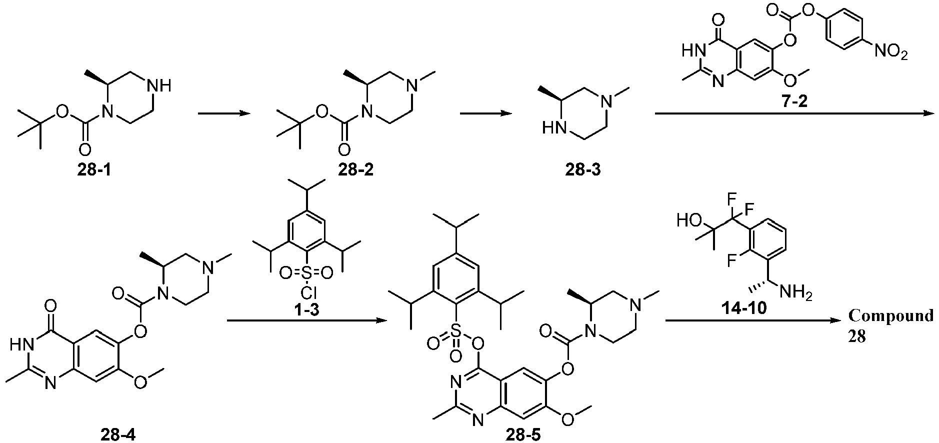

### Step 1

Compound **28-1** (10.0 g, 49.9 mmol) was dissolved in methanol (100 mL), then formaldehyde aqueous solution (20.3 g, 250 mmol, purity: 37%), sodium cyanoborohydride (9.41 g, 150 mmol), and acetic acid (15.0 g, 250 mmol) were added thereto, and the reaction mixture was stirred and reacted at 25°C for 12 hours. The reaction mixture was added with water (200 mL), extracted with dichloromethane (200 mL × 3). The organic phase was washed with saturated brine (200 mL × 1), dried over anhydrous sodium sulfate, filtered, concentrated under reduced pressure, and the residue was purified by silica gel column chromatography (dichloromethane/methanol, 100/1 to 10/1, V/V) to obtain compound **28-2.** ¹H NMR (400MHz, CDCl₃) δ 4.27 - 4.13 (m, 1H), 3.87 - 3.75 (m, 1H), 3.17 - 3.04 (m, 1H), 2.77 - 2.69 (m, 1H), 2.63 - 2.55 (m, 1H), 2.24 (s, 3H), 2.14 - 2.06 (m, 1H), 1.95 - 1.86 (m, 1H), 1.46 (s, 9H), 1.24 (d, *J*=6.8 Hz, 3H). MS-ESI calculated for [M+H]⁺ 215, found 215.

### Step 2

Compound **28-2** (6.00 g, 28.0 mmol) was dissolved in dioxane (50 mL), then a solution of hydrogen chloride in dioxane (4 M, 20.0 mL) was added dropwise thereto. The reaction mixture was stirred at 25°C for 12 hours, and concentrated under reduced pressure to obtain the hydrochloride of crude compound **28-3.** ¹H NMR (400 MHz, CD₃OD) δ 3.89 - 3.74 (m, 3H), 3.74 - 3.66 (m, 1H), 3.66 - 3.31 (m, 3H), 2.99 (s, 3H), 1.43 (d, *J*=6.4 Hz, 3H).

### Step 3

The hydrochloride of compound **28-3** (2.65 g, 14.1 mmol) was dissolved in *N,N-*dimethylformamide (20 mL). Triethylamine (4.77 g, 47.1 mmol) and compound **7-2** (3.5 g, 9.43 mmol) were added dropwise thereto, and the reaction mixture was stirred and reacted at 25°C for 12 hours. The reaction mixture was filtered, concentrated under reduced pressure, and the residue was purified by silica gel column chromatography (dichloromethane/methanol, 100/1 to 10/1, V/V) to obtain compound **28-4.** MS-ESI calculated for [M+H]⁺ 347, found 347.

### Step 4

Compound **28-4** (500 mg, 1.44 mmol) was dissolved in dichloromethane (15 mL). Compound **1-3** (525 mg, 1.73 mmol), 4-dimethylaminopyridine (17.6 mg, 144 µmol), and triethylamine (438 mg, 4.33 mmol) were added thereto, and the reaction mixture was stirred and reacted at 25°C for 12 hours. The reaction mixture was added with water (50 mL), extracted with dichloromethane (50 mL × 3). The organic phase was washed with saturated brine (50 mL × 1), dried over anhydrous sodium sulfate, filtered, concentrated under reduced pressure, and the residue was purified by silica gel column chromatography (dichloromethane/methanol, 100/1 to 10/1, V/V) to obtain compound **28-5.** MS-ESI calculated for [M+H]⁺ 613, found 613.

### Step 5

Compound **28-5** (360 mg, 523 µmol) was dissolved in dimethyl sulfoxide (10 mL), then **14-10** (194 mg, 784 µmol) and triethylamine (159 mg, 1.57 mmol) were added thereto, and the reaction mixture was stirred and reacted at 90°C for 12 hours. The reaction mixture was added with water (50 mL), extracted with ethyl acetate (50 mL × 3), and the organic phase was washed with saturated brine (50 mL × 5), dried over anhydrous sodium sulfate, filtered, and concentrated under reduced pressure. The residue was purified by preparative high performance liquid chromatography (chromatographic column: Phenomenx C18 150 × 40 mm× 5 µm; mobile phase: 0.05% hydrochloric acid aqueous solution-acetonitrile; gradient: acetonitrile 5% to 35%, 10 min) to obtain the hydrochloride of compound **28.** ¹H NMR (400MHz, MeOD) δ 8.41 (s, 1H), 7.61 (t, *J*=7.2 Hz, 1H), 7.42 (t, *J*=7.2 Hz, 1H), 7.23 (t, *J*=7.2 Hz, 1H), 7.17 (s, 1H), 6.01 - 5.99 (m, 1H), 4.73 - 4.58 (m, 1H) 4.51 - 4.20 (m, 1H), 4.04 (s, 3H), 3.63 - 3.56 (m, 3H), 3.43 - 3.37 (m, 1H), 3.25 - 3.18 (m, 1H), 3.00 (s, 3H), 2.62 (s, 3H), 1.74 (d, *J*=7.2 Hz, 3H), 1.57 - 1.47 (m, 3H), 1.29 (s, 6H). MS-ESI calculated for [M+H]⁺ 576, found 576.

### Example 29

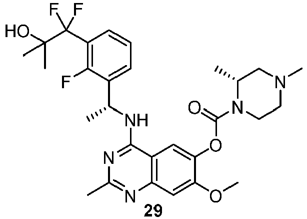

### Synthetic route:

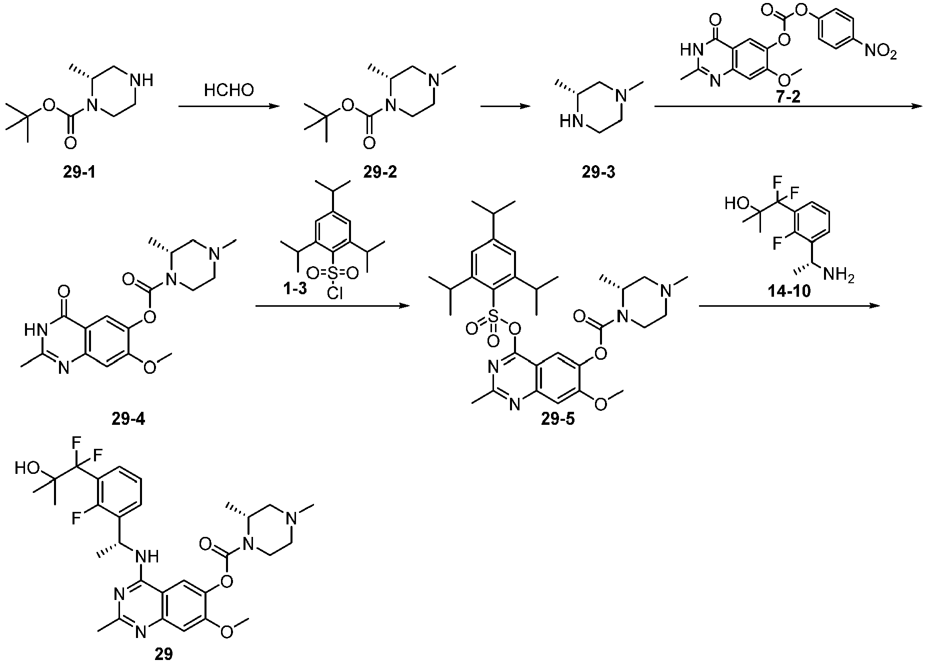

### Step 1

Compound **29-1** (10.0 g, 49.9 mmol) was dissolved in methanol (100 mL), then formaldehyde aqueous solution (20.3 g, 250 mmol, purity: 37%), sodium cyanoborohydride (9.41 g, 150 mmol), and acetic acid (15.0 g, 250 mmol) were added thereto, and the reaction mixture was stirred and reacted at 25°C for 12 hours. The reaction mixture was added with water (300 mL), extracted with ethyl acetate (200 mL × 3). The organic phase was washed with saturated brine (100 mL × 3), dried over anhydrous sodium sulfate, filtered, concentrated under reduced pressure, and the residue was purified by silica gel column chromatography (dichloromethane/methanol, 1/0 to 10/1, V/V) to obtain compound **29-2.** ¹H NMR (400MHz, CDCl₃) δ 4.21 - 4.18 (m, 1H), 3.82 - 3.79 (m, 1H), 3.13 - 3.06 (m, 1H), 2.74 - 2.70 (m, 1H), 2.60 - 2.52 (m, 1H), 2.24 (s, 3H), 2.11 - 2.07 (m, 1H), 1.93 - 1.86 (m, 1H), 1.45 (s, 9H), 1.29 (d, *J*=7.2 Hz, 3H). MS-ESI calculated for [M+H]⁺ 215, found 215.

### Step 2

Compound **29-2** (7.25 g, 33.8 mmol) was dissolved in dioxane (50 mL), then a solution of hydrogen chloride in dioxane (4 M, 67.6 mL) was added dropwise thereto. The reaction mixture was stirred at 25°C for 12 hours, and concentrated under reduced pressure to obtain the hydrochloride of crude compound **29-3.** ¹H NMR (400 MHz, CD₃OD) δ 3.82 - 3.73 (m, 3H), 3.74 - 3.66 (m, 1H), 3.66 - 3.31 (m, 3H), 3.02 (s, 3H), 1.47 (d, *J*=7.2 Hz, 3H).

### Step 3

The hydrochloride of compound **29-3** (811 mg, 5.39 mmol) was dissolved in *N,N-*dimethylformamide (15 mL). Triethylamine (1.64 g, 16.2 mmol) and compound **7-2** (1.00 g, 2.69 mmol) were added dropwise thereto, and the reaction mixture was stirred and reacted at 25°C for 12 hours. The reaction mixture was filtered, concentrated under reduced pressure, and the residue was purified by silica gel column chromatography (dichloromethane/methanol, 20/1 to 1/1, V/V) to obtain compound **29-4.** MS-ESI calculated for [M+H]⁺ 347, found 347.

### Step 4

Compound **29-4** (254 mg, 733 µmol) was dissolved in dichloromethane (5 mL). Compound **1-3** (333 mg, 1.10 mmol), 4-dimethylaminopyridine (8.96 mg, 73.3 µmol), and *N,N*-diisopropylethylamine (284 mg, 2.20 mmol) were added thereto, and the reaction mixture was stirred and reacted at 25°C for 12 hours. The reaction mixture was added with water (50 mL), extracted with ethyl acetate (40 mL × 5). The organic phase was washed with saturated brine (100 mL × 2), dried over anhydrous sodium sulfate, filtered, concentrated under reduced pressure, and the residue was purified by silica gel column chromatography (dichloromethane/methanol, 1/0 to 10/1, V/V) to obtain compound **29-5.** MS-ESI calculated for [M+H]⁺ 613, found 613.

### Step 5

Compound **29-5** (154 mg, 251 µmol) was dissolved in dimethyl sulfoxide (5 mL), then **14-10** (93.2 mg, 377 µmol) and triethylamine (76.3 mg, 754 µmol) were added thereto, and the reaction mixture was stirred and reacted at 90°C for 12 hours. The reaction mixture was added with water (50 mL), extracted with ethyl acetate (50 mL × 3), and the organic phase was washed with saturated brine (50 mL × 5), dried over anhydrous sodium sulfate, filtered, and concentrated under reduced pressure. The residue was purified by preparative high performance liquid chromatography (chromatographic column: Xtimate C18 150 × 40 mm × 5 µm; mobile phase: 0.05% hydrochloric acid aqueous solution-acetonitrile; gradient: acetonitrile 5% to 30%, 10 min) to obtain the hydrochloride of compound **29.** ¹H NMR (400MHz, MeOD) δ 8.40 (s, 1H), 7.61 (t, *J*=7.2 Hz, 1H), 7.42 (t, *J*=6.8 Hz, 1H), 7.23 (t, *J*=7.2 Hz, 1H), 7.16 (s, 1H), 6.01 - 5.99 (m, 1H), 4.93 - 4.86 (m, 1H) 4.68 - 4.22 (m, 1H), 4.04 (s, 3H), 3.62 - 3.55 (m, 4H), 3.23 - 3.19 (m, 1H), 2.99 (s, 3H), 2.62 (s, 3H), 1.74 (d, *J*=6.8 Hz, 3H), 1.57 - 1.48 (m, 3H), 1.29 (s, 6H). MS-ESI calculated for [M+H]⁺ 576, found 576.

### Biological activity:

### Experimental example 1: KRAS(G12C) and SOS1 binding assay

### Experimental principle:

The small molecular compound binds to the catalytic site of SOS1, thus inhibiting the binding of SOS 1 to KRAS (G12C). When the binding of fluorescently labeled SOS1 protein to fluorescently labeled KRAS (G12C) protein is inhibited, the emitted fluorescence changes. The ability of small molecules to prevent the binding of SOS1 to KRAS (G12C) can be tested by detecting changes in fluorescence. A homogeneous time-resolved fluorescence (HTRF) binding assay was used to detect the ability of the compounds of the present disclosure to inhibit the binding of SOS1 to KRAS (G12C).

### Experimental materials:

KRAS (G12C) protein was expressed and purified by Wuhan AtaGenix Biotechnology Co., Ltd., SOS1 exchange domin (564-1049) protein (Human recombinant) was purchased from Cytoskeleton, Mab Anti 6HIS-XL665 and Mab Anti GST-Eu cryptate were purchased from Cisbio. Multimode microplate reader Nivo5 was purchased from PerkinElmer.

### Experimental methods:

1X buffer preparation (ready-to-use): Hepes: 5 mM; NaCl: 150 mM; EDTA:10 mM; Igepal: 0.0025%; KF: 100 mM; DTT:1 mM; BSA:005%;

The test compounds were 5-fold diluted with DMSO using a multi-channel pipette to the 8th concentration, i.e., diluted from 1 mM to 0.064 µM.

The test compound was diluted by gradient with 1X buffer to a working solution with 2% DMSO. 5 µL/well of the working solution was added to corresponding wells, and the corresponding concentration gradient was 20 µM to 0.00128 nM. Duplicate experiment was set. The working solution was centrifuged at 1000 rpm for 1 minute.

A mixed working solution of KRAS(G12C) (200 nM) and Mab Anti GST-Eu cryptate (1 ng/µL) was prepared with 1X buffer, and the mixed working solution was incubated at 25°C for 5 minutes, and 2.5 µL/well of the mixed working solution was added to the corresponding wells.

A mixed working solution of SOS1 (80 nM) and Mab Anti 6HIS-XL665 (8 g/µL) was prepared with 1X buffer, and 2.5 µL/well of the mixed working solution was added to the corresponding wells. 2.5 µL of Mab Anti 6HIS-XL665 (8 g/µL) diluent was added to blank wells. At this time, the final concentration gradient of the compound was diluted from 10 µM to 0.64 nM, KRAS(G12C) (500 nM), MAb Anti GST-Eu cryptate (0.25 ng/µL), SOS1 (20 nM), Mab Anti 6HIS-XL665 (2 g/µL), the reaction system was placed at 25°C for 60 minutes. After the reaction was completed, the multimode microplate reader was used to read HTRF.

### Data analysis:

Using the equation (Sample - Min)/(Max - Min) × 100 % to convert the raw data into inhibition rate, the IC₅₀ value might be obtained by curve fitting with four parameters (obtained by "log (inhibitor) vs. response -- Variable slope" mode in GraphPad Prism). Inhibitory activity of the compounds of the present disclosure on the binding of KRAS(G12C) to SOS1 is provided in Table 1.

**Table 1 Test results of IC₅₀ values of compounds of the present disclosure for the binding of KRAS(G12C) to SOS1**

| **Test compound** | **IC₅₀ (nM)** |
|---|---|
| Compound **2** | 109.8 |
| Hydrochloride of compound **4** | 71.1 |
| Hydrochloride of compound **5** | 77.3 |
| Hydrochloride of compound **6** | 87.6 |
| Compound **7** | 92.8 |
| Hydrochloride of compound **8** | 73.7 |
| Compound **9** | 83.6 |
| Compound **11** | 78.6 |
| Compound **13** | 70.4 |
| Hydrochloride of compound **14** | 40.4 |
| Hydrochloride of compound **15** | 149.4 |
| Hydrochloride of compound **16** | 59.6 |
| Hydrochloride of compound **18** | 23.4 |
| Hydrochloride of compound **19** | 104.9 |
| Compound **24** | 47.9 |
| Compound **25** | 86.1 |
| Compound **26** | 65.9 |
| Compound **27** | 40.64 |
| Hydrochloride of compound **28** | 14.49 |
| Hydrochloride of compound **29** | 14.66 |

**Experimental conclusion:** The compounds of the present disclosure have significant inhibitory effect on the binding of KRAS (G12C) to SOS1.

### Experimental example 2: 3D proliferation inhibitory activity test of H358 cells

### Experimental principle:

In H358 cells with KRAS (G12C) mutation, KRAS signaling pathway was abnormally activated. By inhibiting the binding of SOS1 to RAS protein, the small molecule SOS1 inhibitor reduced its GEF activity and the proportion of activated RAS-GTP. The phosphorylation level of MEK/ERK pathway downstream of RAS was further down-regulated to achieve the effect of inhibiting cell proliferation. Small molecules were co-cultured with H358 cells in a 3D space, and then the number of cells was read to indirectly reflect the inhibitory activity of SOS1 inhibitors on the proliferation of H358 cells.

### Experimental materials:

RPMI1640 medium, fetal bovine serum, penicillin/streptomycin antibiotics were purchased from WISENT, and low melting point agarose was purchased from Sigma. Almar blue reagent was purchased from Invitrogen. NCI-H358 cell line was purchased from Nanjing Cobioer Biosciences Co. Ltd. Nivo multimode microplate reader (PerkinElmer).

### Experimental methods:

H358 cells were seeded in a 96-well U-shaped plate. First, low melting point agarose was prepared into 2% stock solution, and the agarose stock solution was first heated in a microwave oven to melt completely, and then transferred to a 42°C water bath to keep the agarose in a liquid state. The gel was added to a serum-containing medium to prepare a gel concentration of 0.6% as the bottom layer gel, and spread into the 96-well U-shaped plate at 50 µL per well. After the bottom layer gel was solidified, 2% gel was then added to the cell-containing medium to prepare a cell-containing upper gel with a gel concentration of 0.4% and a cell density of 4 × 10⁴ cells/mL. The cell-containing upper gel was added to the 96-well U-shaped plate covered with the bottom layer gel at 75 µL per well, with a cell density of 3,000 cells per well. After the upper gel was solidified, the cell plate was cultured overnight in a carbon dioxide incubator.

On the day of adding the compound, 85 µL of liquid medium was added to the 96-well U-shaped plate with cells already seeded. The test compounds were 3-fold diluted with a multi-channel pipette to the 9th concentration, i.e., diluted from 6 mM to 0.9 µM, and duplicate experiment was set. 97 µL of culture medium was added to an intermediate plate, and then 2.5 µL of gradient diluted compound per well was transferred to the intermediate plate according to the corresponding position. After mixing well, the compound was transferred to the cell plate at 40 µL per well. The concentration of the compound transferred to the cell plate ranged from 30 µM to 4.5 nM. The cell plate was cultured in a carbon dioxide incubator for 7 days. On the 8th day, the test compounds were 3-fold diluted with a multi-channel pipette to the 9th concentration, i.e., diluted from 6 mM to 0.9 µM, and duplicate experiment was set. 198 µL of culture medium was added to the intermediate plate, and then 2 µL of gradient diluted compound per well was transferred to the first intermediate plate according to the corresponding position. Then 100 µL of culture medium was added to the second intermediate plate, and 100 µL of mixed compound from the first intermediate plate was added thereto. After mixing well, the compound was transferred to the cell plate at 40 µL per well. The concentration of the compound transferred to the cell plate ranged from 30 µM to 4.5 nM. The cell plate was placed in a carbon dioxide incubator and cultured for another 7 days. The compounds were co-incubated with the cells for 14 days, and 20 µL of Almar blue detection reagent per well was added to the cell plate. The plate added with the dye was placed on a horizontal shaker and shaken for 15 minutes, and then the plate was incubated at room temperature for 5 hours to stabilize the luminescence signal. A multimode microplate reader was used for reading.

### Data analysis:

Using the equation (Sample - Min)/(Max - Min) × 100 % to convert the raw data into inhibition rate, the IC₅₀ value might be obtained by curve fitting with four parameters (obtained by "log (inhibitor) vs. response -- Variable slope" mode in GraphPad Prism).

**Experimental conclusion:** The compounds of the present disclosure can inhibit the proliferation of H358 cells under 3D conditions.

### Experimental example 3: p-ERK proliferation inhibitory activity test of DLD-1 cells

### Experimental materials:

DLD-1 cells were purchased from Nanjing Cobioer; 1640 medium was purchased from Biological Industries; fetal bovine serum was purchased from Biosera; Advanced Phospho-ERK1/2 (THR202/TYR204) KIT was purchased from Cisbio.

**Table 2 Composition list of Advanced Phospho-ERK1/2(THR202/TYR204) KIT**

| **Component name** | **Storage temperature** |
|---|---|
| Advanced PhosphoERK1/2 Eu Cryptate antibody | ≤ --16°C |
| Advanced PhosphoERK1/2 d2 antibody | ≤ --16°C |
| Blocking reagent (stock solution 100X) | ≤ --16°C |
| Lysis buffer # 1 (stock solution 4X) | ≤ --16°C |
| Detection buffer (ready-to-use) | ≤ --16°C |

### Experimental methods:

DLD-1 cells were seeded in a transparent 96-well cell culture plate, with 80 µL of cell suspension per well and 8000 DLD-1 cells per well. The cell plate was incubated in a carbon dioxide incubator at 37°C overnight;
the test compound was diluted to 2 mM with 100% DMSO as the first concentration, and then 5-fold diluted to the 8th concentration with a pipette, that is, diluted from 2 mM to 0.026 µM. 2 µL of compound was added to 78 µL of cell starvation medium. After mixing well, 20 µL of compound solution was taken and added to the corresponding cell plate wells, and the cell plate was put back to the carbon dioxide incubator to continue to incubate for 1 hour; at this time, the concentration of the compound was from 10 µM to 0.128 nM, and the concentration of DMSO was 0.5%;
after the incubation was completed, the cell supernatant was discarded, and 50 µL of cell lysate was added to each well, and incubated at room temperature for 30 minutes with shaking;
Phospho-ERK1/2 Eu Cryptate antibody and Phospho-ERK1/2 d2 antibody were 20-fold diluted using Detection buffer;
16 µL of cell lysate supernatant per well was put into a new 384 white microplate, then 2 µL of Phospho-ERK1/2 Eu Cryptate antibody diluent and 2 µL of Phospho-ERK1/2 d2 antibody diluent were added thereto and incubated at room temperature for 4 hours;
after the incubation was completed, a multimode microplate reader was used to read HTRF excitation: 320 nm, emission: 615 nm, 665 nm.

### Data analysis:

Using the equation (Sample - Min) / (Max - Min) * 100 % to convert the raw data into inhibition rate, the IC₅₀ value might be obtained by curve fitting with four parameters (obtained by "log (inhibitor) vs. response -- Variable slope" mode in GraphPad Prism).
Max well: Positive control well reading is 1X lysate
Min well: Negative control well reading is 0.5% DMSO cell well cell lysate

Inhibitory activity of the compounds of the present disclosure on p-ERK in DLD-1 cells is provided in Table 3.

**Table 3 Test results of IC₅₀ values of compounds of the present disclosure on p-ERK proliferation in DLD-1 cells**

| **Test compound** | **IC₅₀ (nM)** |
|---|---|
| Compound **2** | 121.7 |
| Hydrochloride of compound **4** | 113.2 |
| Hydrochloride of compound **5** | 219.4 |
| Compound **10** | 86.2 |
| Compound **13** | 281.9 |
| Compound **14** | 78.9 |
| Hydrochloride of compound **16** | 222 |
| Hydrochloride of compound **18** | 242.9 |
| Compound **26** | 283.1 |
| Compound **27** | 111.9 |
| Hydrochloride of compound **28** | 91.55 |
| Hydrochloride of compound **29** | 135.4 |

**Experimental conclusion:** The compounds of the present disclosure have significant inhibitory effect on the proliferation of p-ERK in DLD-1 cells.

### Experimental example 4A: Pharmacokinetic evaluation of compounds

### Experimental materials:

CD-1 mice (male, 7 to 9 weeks old, Shanghai SLAC)

### Experimental operation:

The pharmacokinetic characteristics of the compound in rodents after intravenous injection and oral administration were tested according to the standard protocol. In the experiment, the candidate compound was formulated into clear solutions, and a single intravenous injection and oral administration were given to mice. The vehicle for intravenous injection and oral administration was a mixed vehicle composed of 10% dimethyl sulfoxide and 90% hydroxypropyl β-cyclodextrin with a content of 10%. In this project, four female CD-1 mice were used, and two mice were administered intravenously at a dose of 1 mg/kg. Plasma samples were collected at 0.033, 0.083, 0.25, 0.5, 1, 2, 4, 8, and 12 hours after administration. The other two mice were administered orally by gavage at a dose of 2 mg/kg. Plasma samples were collected at 0.083, 0.25, 0.5, 1, 2, 4, 8, and 12 hours after administration, stirred at 4°C for 10 minutes at 3,200 × g, and the supernatants were separated from the plasma samples. The protein was precipitated by adding 20 times the volume of methanol solution containing internal standard, stirred at 12000 × g for 15 minutes, centrifuged at 4°C. 50 µL of the supernatant was transferred to a 96-well plate and centrifuged for a second time to take the supernatant for injection. The plasma concentration was quantitatively analyzed by LC-MS/MS analysis method, and the pharmacokinetic parameters were calculated, such as peak concentration (Cₘₐₓ), clearance rate (CL), half-life (T_{1/2}), volume of distribution at steady state (Vdss), area under drug-time curve (AUC₀₋ₗₐₛₜ), bioavailability (F).

The experimental results are shown in Table 4:

**Table 4 Pharmacokinetic test results of compounds of the present disclosure**

| **Compound** | Peak concentration **Cₘₐₓ (nM)** | Clearance rate **CL (mL/min/kg)** | Volume of distribution at steady state **Vdss (L/kg)** | Half life **T_{1/2} (IV, h)** | Area under drug-time curve **AUC**_{**0**-}**ₗₐₛₜ PO (nM.hr)** | Bioavailability **F (%)** |
|---|---|---|---|---|---|---|
| Hydrochloride of compound **14** | 92.3 | 29.2 | 4.95 | 2.96 | 606 | 33.9 |

**Experimental conclusion:** The compounds of the present disclosure have good pharmacokinetic properties, including good oral bioavailability, oral exposure, half-life, clearance rate, etc.

### Experimental example 4B: Pharmacokinetic evaluation of compounds

### Experimental materials:

Balb/c mice (male, Beijing Vital River Laboratory Animal Technology Co., Ltd.)

### Experimental operation:

The pharmacokinetic characteristics of the compound in rodents after intravenous injection and oral administration were tested according to the standard protocol. In the experiment, the candidate compound was formulated into clear solutions, and a single intravenous injection and oral administration were given to mice. The vehicle for intravenous injection and oral administration was a mixed vehicle composed of 5% dimethyl sulfoxide, 5% solutol, and 90% water. In this project, four male Balb/c mice were used, and two mice were administered intravenously at a dose of 10 mg/kg. Plasma samples were collected at 0.083, 0.25, 0.5, 1, 2, 4, 8, and 24 hours after administration. The other two mice were administered orally by gavage at a dose of 50 mg/kg. Plasma samples were collected at 0.25, 0.5, 1, 2, 4, 6, 8, 12, and 24 hours after administration. Blood samples were placed on ice after collection and centrifuged to separate plasma within 1 hour (centrifugation conditions: 6000 g, 3 minutes, 2 to 8°C). Plasma samples were stored in a -80°C refrigerator before analysis. The plasma concentration was quantitatively analyzed by LC-MS/MS analysis method, and the pharmacokinetic parameters were calculated, such as peak concentration (Cₘₐₓ), clearance rate (CL), half-life (T_{1/2}), volume of distribution at steady state (Vdss), area under drug-time curve (AUC₀₋ₗₐₛₜ), bioavailability (F).

The experimental results are shown in Table 5:

**Table 5 Pharmacokinetic test results of compounds of the present disclosure**

| **Compound** | Peak concentration **Cₘₐₓ (nM)** | Clearance rate **CL (mL/min/kg)** | Volume of distribution at steady state **Vdss (L/kg)** | Half life **T_{1/2} (IV, h)** | Area under drug-time curve **AUC₀₋ₗₐₛₜ PO (nM.hr)** | Bioavailability **F (%)** |
|---|---|---|---|---|---|---|
| Hydrochloride of compound **28** | 4595 | 36.7 | 11.5 | 6.01 | 37469 | 98.8 |
| Hydrochloride of compound **29** | 10867 | 18.7 | 4.15 | 3.46 | 74594 | 91.15 |

**Experimental conclusion:** The compounds of the present disclosure have good pharmacokinetic properties, including good oral bioavailability, oral exposure, half-life, clearance rate, etc.

### Experimental example 5: In vivo drug efficacy evaluation of the compound in the Miapaca2 nude mouse xenograft tumor model

### Cell culture:

Human pancreatic cancer cells (Miapaca2) were cultured *in vitro* as a monolayer on an adherent surface, and the culture conditions were DMEM medium plus 10% fetal bovine serum, cultured in a 37°C, 5% COz incubator. The cells were treated with trypsin-EDTA for routine digestion and passaged two to three times a week. When the cell saturation was 80% to 90% and the number reached the requirements, the cells were collected, counted, and inoculated.

### Experimental animals:

Balb/c nude mice, female, 6 to 7 weeks old, were purchased from Shanghai SIPPR-BK Lab Animal Co., Ltd.

### Model preparation:

0.2 mL (5×10⁶) Miapaca-2 cells (plus Matrigel, volume ratio of 1:1) were subcutaneously inoculated on the right back of each mouse, and the administration in groups began when the average tumor volume reached 118 mm³.

The administration regimen is shown in Table 6.

**Table 6 Grouping and administration regimen of experimental animals**

| **Group** | **Number of mice** | **Compound** | **Dose (mg/kg )** | **Administration volume parameter (µL/g)²** | **Route of administration** | **Frequency of administration** |
|---|---|---|---|---|---|---|
| 1 | 6 | Vehicle group 5% dimethyl sulfoxide + 95% (10% hydroxypropyl-β-cyclodextrin aqueous solution) | NA | 10 | Intragastric administration | Once a day |
| 2 | 6 | AMG-510 | 5 | 10 | Intragastric administration | Once a day |
| 3 | 6 | AMG-510+ Hydrochloride of compound **14** | 5 + 50 | 10 | Intragastric administration | Once a day + twice a day |

### Tumor measurements and experimental indicators:

The tumor diameter was measured twice a week with a vernier caliper, and the tumor volume was measured in cubic millimeters, calculated by the following formula: V = 0.5a × b², wherein a and b are the long and short diameters of the tumor, respectively. The tumor inhibitory efficacy of the test compound was evaluated by using TGI (%). TGI (%) reflects the rate of tumor growth inhibition. TGI (%) = [1- (average tumor volume at the end of administration in a certain treatment group - average tumor volume at the beginning of administration in this treatment group) / (average tumor volume at the end of treatment in vehicle control group - average tumor volume at the beginning of treatment in vehicle control group)] × 100%.

The experimental results are shown in Table 7.

**Table 7 Evaluation of tumor inhibitory efficacy of compounds of the present disclosure in Miapaca-2 nude mouse xenograft tumor model (calculated based on tumor volume on day 22 after administration)**

| **Group** | **Mean tumor volume** ± **SEM (mm³) (day 22)** | **TGI (%)** |
|---|---|---|
| Vehicle group | 2016±173 | / |
| AMG-510 | 819±407 | 59.4 |
| AMG-510 + Hydrochloride of compound **14** | 219±74 | 89.1 |

**Experimental conclusion:** The combination of the compounds of the present disclosure and AMG-510 exhibits an excellent tumor inhibitory effect in the Miapaca-2 nude mouse xenograft tumor model.

## Claims

1. A compound of formula (II) or a pharmaceutically acceptable salt thereof, 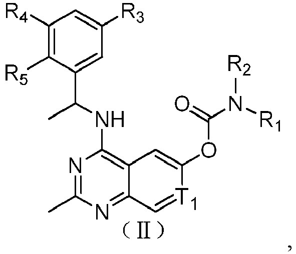 wherein
R₁ and R₂ are each independently selected from H, C₁₋₆ alkyl, C₃₋₁₂ cycloalkyl, 3- to 12-membered heterocycloalkyl, C₆₋₁₀ aryl, and 5- to 10-membered heteroaryl, wherein the C₁₋₆ alkyl, C₃₋₁₂ cycloalkyl, 3- to 12-membered heterocycloalkyl, C₆₋₁₀ aryl, and 5- to 10-membered heteroaryl are each independently and optionally substituted by 1, 2, 3, or 4 Rₐ;
or, R₁, R₂ together with the nitrogen atom to which they are attached form a 3- to 12-membered heterocycloalkyl ring, wherein the 3- to 12-membered heterocycloalkyl ring is optionally substituted by 1, 2, 3, or 4 R_{b};
R₃ is selected from H, F, Cl, Br, I, -OH, -NH₂, -CN, C₁₋₆ alkyl, C₁₋₆ alkoxy, and C₁₋₆ alkylamino, wherein the C₁₋₆ alkyl, C₁₋₆ alkoxy, and C₁₋₆ alkylamino are each independently and optionally substituted by 1, 2, 3, or 4 R_{c};
R₄ is selected from H, F, Cl, Br, I, -OH, -NH₂, -CN, C₁₋₆ alkyl, C₁₋₆ alkoxy, C₁₋₆ alkylamino, -C(=O)-O-C₁₋₆ alkyl, C₃₋₁₀ cycloalkyl, and 3- to 10-membered heterocycloalkyl, wherein the C₁₋₆ alkyl, C₁₋₆ alkoxy, C₁₋₆ alkylamino, -C(=O)-O-C₁₋₆ alkyl, C₃₋₁₀ cycloalkyl, and 3- to 10-membered heterocycloalkyl are each independently and optionally substituted by 1, 2, 3, or 4 R_{c};
R₅ is selected from H, F, Cl, Br, I, -OH, -NH₂, -CN, C₁₋₆ alkyl, C₁₋₆ alkoxy, and C₁₋₆ alkylamino, wherein the C₁₋₆ alkyl, C₁₋₆ alkoxy, and C₁₋₆ alkylamino are each independently and optionally substituted by 1, 2, 3, or 4 R_{c};
or, R₄, R₅ together with the carbon atom to which they are attached form 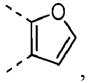 wherein 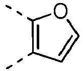 is optionally substituted by 1, 2, 3, or 4 R_{d};
T₁ is selected from CR₆ and N;
R₆ is selected from -OCH₃, -CN, and -S(=O)₂-CH₃;
each Rₐ is independently selected from F, Cl, Br, I, -OH, -NH₂, -CN, =O, C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl, wherein the C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl are each independently and optionally substituted by 1, 2, 3, or 4 R;
each R_{b} is independently selected from F, Cl, Br, I, -OH, -NH₂, -CN, =O, C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl, wherein the C₁₋₆ alkyl, C₁₋₆ alkoxy, C₁₋₆ alkylamino, -C(=O)-O-C₁₋₆ alkyl, C₃₋₁₀ cycloalkyl, 3- to 10-membered heterocycloalkyl, C₆₋₁₀ aryl, 5- to 10-membered heteroaryl, -O-C₆₋₁₀ aryl, and -O-5- to 10-membered heteroaryl are each independently and optionally substituted by 1, 2, 3, or 4 R;
each R_{c} is independently selected from F, Cl, Br, I, -OH, -NH₂, -CN, -COOH, =O, - C(=O)H, -C(=O)-NHz, C₁₋₃ alkyl, C₁₋₃ alkoxy, and 5- to 6-membered heterocycloalkyl, wherein the C₁₋₃ alkyl, C₁₋₃ alkoxy, and 5- to 6-membered heterocycloalkyl are each independently and optionally substituted by 1, 2, 3, or 4 R;
each R_{d} is independently selected from F, Cl, Br, I, -OH, -NH₂, -CN, -COOH, =O, - C(=O)H, -C(=O)-NHz, and C₁₋₃ alkyl;
each R is independently selected from F, Cl, Br, I, -OH, -NH₂, -CN, -COOH, =O, -C(=O)H, -C(=O)-NHz, and C₁₋₃ alkyl;
"hetero" in the 5- to 6-membered heterocycloalkyl, 3- to 10-membered heterocycloalkyl, 3- to 12-membered heterocycloalkyl, 5- to 10-membered heteroaryl, and -O-5- to 10-membered heteroaryl represents 1, 2, 3, or 4 heteroatoms or heteroatom groups each independently selected from -O-, -NH-, -S-, and -N-.

2. The compound or the pharmaceutically acceptable salt thereof according to claim 1, wherein the compound has a structure of formula (II-1): 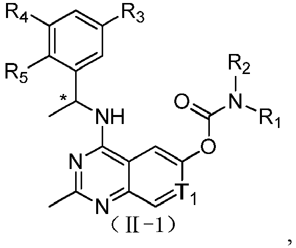
wherein T₁, R₁, R₂, R₃, R₄, and R₅ are as defined in claim 1;
the carbon atom with "*" is a chiral carbon atom, which exists in an (*R*) or (*S*) single enantiomer form or an (*R*) or (*S*) single enantiomer-rich form.

3. The compound or the pharmaceutically acceptable salt thereof according to claim 1 or 2, wherein each R is independently selected from F, Cl, Br, and =O.

4. The compound or the pharmaceutically acceptable salt thereof according to claim 1 or 2, wherein each R_{b} is independently selected from F, Cl, Br, I, -OH, -NH₂, -CN, =O, C₁₋₃ alkyl, C₁₋₃ alkylamino, and 3- to 6-membered heterocycloalkyl, wherein the C₁₋₃ alkyl, C₁₋₃ alkylamino, and 3- to 6-membered heterocycloalkyl are each independently and optionally substituted by 1, 2, 3, or 4 R.

5. The compound or the pharmaceutically acceptable salt thereof according to claim 4, wherein each R_{b} is independently selected from F, Cl, Br, -NH₂, -CH₃, -CH₂-CH₃, 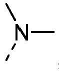 , and 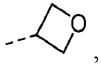 wherein the -CH₃, -CH₂-CH₃, 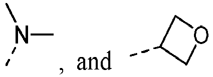 are each independently and optionally substituted by 1, 2, 3, or 4 R.

6. The compound or the pharmaceutically acceptable salt thereof according to claim 5, wherein each R_{b} is independently selected from F, Cl, Br, -NH₂, -CH₃, -CH₂-CH₃,

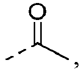

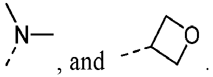

7. The compound or the pharmaceutically acceptable salt thereof according to claim 1 or 2, wherein each R_{c} is independently selected from F, Cl, Br, -OH, -OCH₃, and

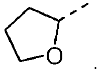

8. The compound or the pharmaceutically acceptable salt thereof according to claim 1 or 2, wherein each R_{d} is independently selected from F, Cl, and Br.

9. The compound or the pharmaceutically acceptable salt thereof according to claim 1 or 2, wherein R₃ is selected from H, F, Cl, Br, and -NH₂.

10. The compound or the pharmaceutically acceptable salt thereof according to claim 1 or 2, wherein R₄ is selected from H, F, Cl, Br, -CN, -CH₃, -CH₂CH₃, -CH(CH₃)₂, and - CH₂CH(CH₃)₂, wherein the -CH₃, -CH₂CH₃, -CH(CH₃)₂, and -CH₂CH(CH₃)₂ are each independently and optionally substituted by 1, 2, 3, or 4 R_{c}.

11. The compound or the pharmaceutically acceptable salt thereof according to claim 10, wherein R₄ is selected from H, F, Cl, Br, -CN, 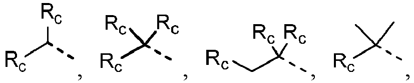 and

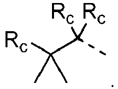

12. The compound or the pharmaceutically acceptable salt thereof according to claim 11, wherein R₄ is selected from H, F, Cl, Br, -CN,

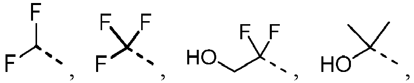

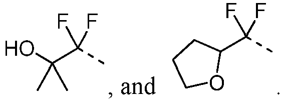

13. The compound or the pharmaceutically acceptable salt thereof according to claim 1 or 2, wherein R₅ is selected from H, F, Cl, Br, and -CH₃.

14. The compound or the pharmaceutically acceptable salt thereof according to claim 1 or 2, wherein R₁, R₂ together with the nitrogen atom to which they are attached form a 5- to 11-membered heterocycloalkyl ring, wherein the 5- to 11-membered heterocycloalkyl ring is optionally substituted by 1, 2, 3, or 4 R_{b}.

15. The compound or the pharmaceutically acceptable salt thereof according to claim 14, wherein the structural moiety 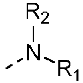 is selected from 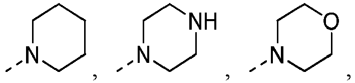 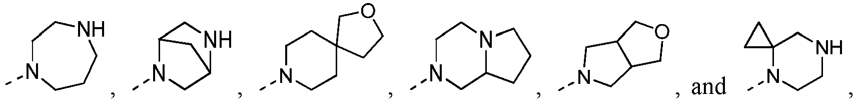 wherein the 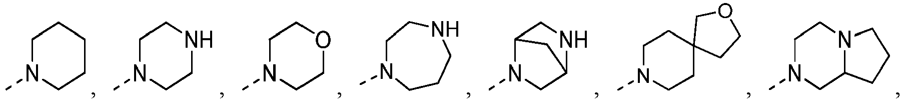 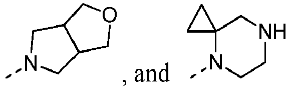 are each independently and optionally substituted by 1, 2, 3, or 4 R_{b}.

16. The compound or the pharmaceutically acceptable salt thereof according to claim 15, wherein the structural moiety 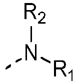 is selected from

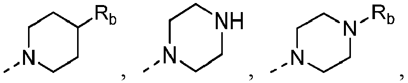

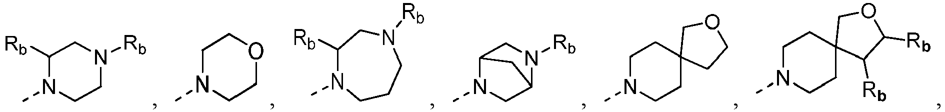

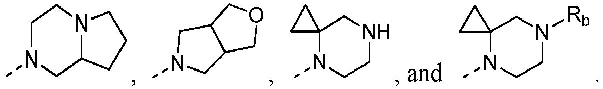

17. The compound or the pharmaceutically acceptable salt thereof according to claim 6 or 16, wherein the structural moiety 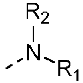 is selected from

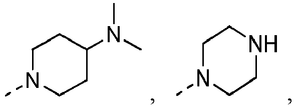

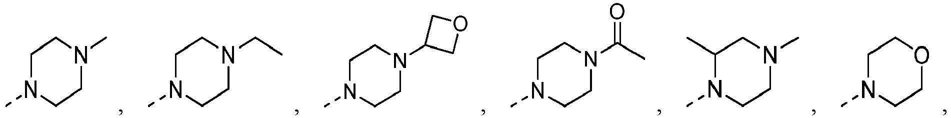

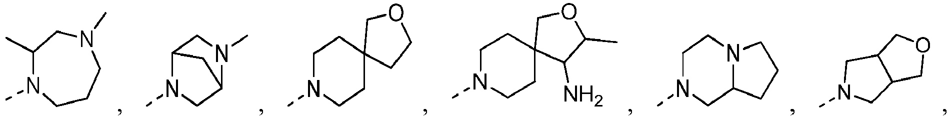

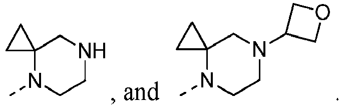

18. The compound or the pharmaceutically acceptable salt thereof according to claim 1 or 2, wherein the structural moiety 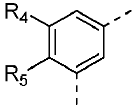 is selected from

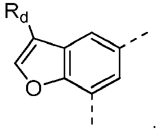

19. The compound or the pharmaceutically acceptable salt thereof according to claim 18, wherein the structural moiety 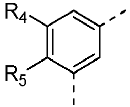 is selected from

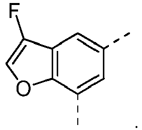

20. The compound or the pharmaceutically acceptable salt thereof according to claim 1, wherein the compound has a structure of formula (II-2) or (II-3): 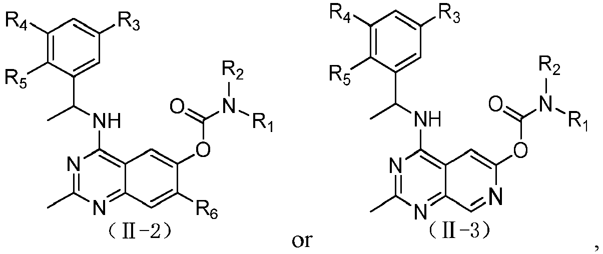 wherein R₁, R₂, R₃, R₄, R₅, and R₆ are as defined in claim 1.

21. The compound or the pharmaceutically acceptable salt thereof according to claim 20, wherein the compound has a structure of formula (I-2): 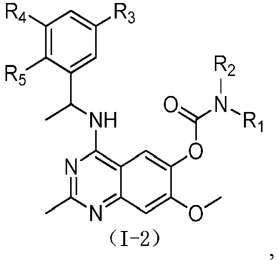 wherein R₁, R₂, R₃, R₄, and R₅ are as defined in claim 20.

22. The compound or the pharmaceutically acceptable salt thereof according to claim 21, wherein the compound has a structure of formula (I-3): 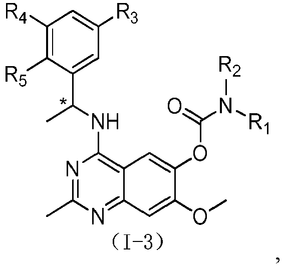
wherein R₁, R₂, R₃, R₄, and R₅ are as defined in claim 21;
the carbon atom with "*" is a chiral carbon atom, which exists in an (*R*) or (*S*) single enantiomer form or an (*R*) or (*S*) single enantiomer-rich form.

23. The compound or the pharmaceutically acceptable salt thereof according to claim 1, wherein the compound has a structure of formula (I-4), (I-5), (I-6), or (III-1): 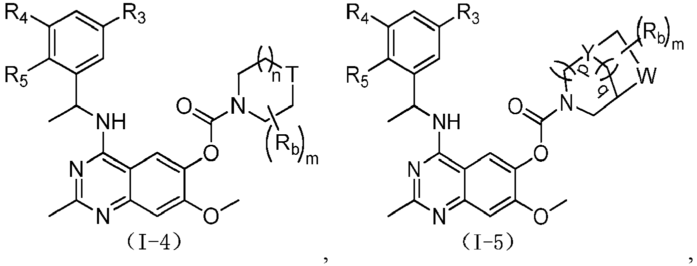 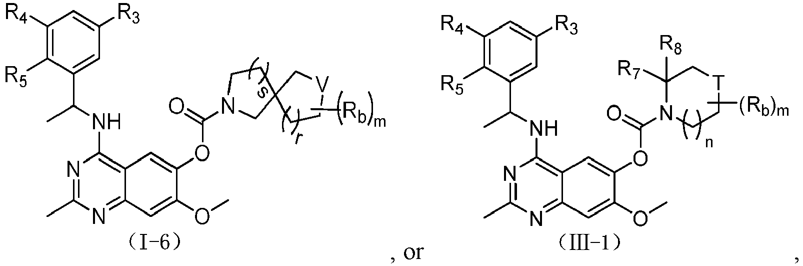 wherein
T and V are each independently selected from CH₂, NH, and O;
m is selected from 0, 1, 2, 3, and 4;
n, p, q, r, and s are each independently selected from 0, 1, and 2;
and p + q ≤ 3;
W is selected from NH, -CH₂-CH₂-, and -O-CH₂-;
Y is selected from N and CH;
R₇ and R₈ are each independently selected from H, F, Cl, Br, I, -OH, -NH₂, -CN, =O, and C₁₋₃ alkyl;
or, R₇, R₈ together with the carbon atom to which they are attached form a C₃₋₄ cycloalkyl ring;
R₃, R₄, R₅, and R_{b} are as defined in claim 1.

24. The compound or the pharmaceutically acceptable salt thereof according to claim 23, wherein R₇ and R₈ are each independently selected from H and -CH₃.

25. The compound or the pharmaceutically acceptable salt thereof according to claim 23, wherein R₇, R₈ together with the carbon atom to which they are attached form a cyclopropyl ring.

26. The compound or the pharmaceutically acceptable salt thereof according to claim 23, wherein the compound has a structure of formula (I-7), (I-8), (I-9), or (III-1A): 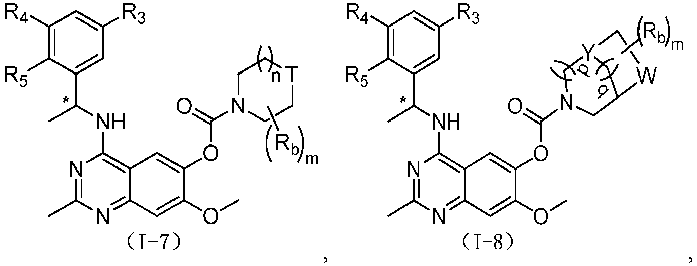 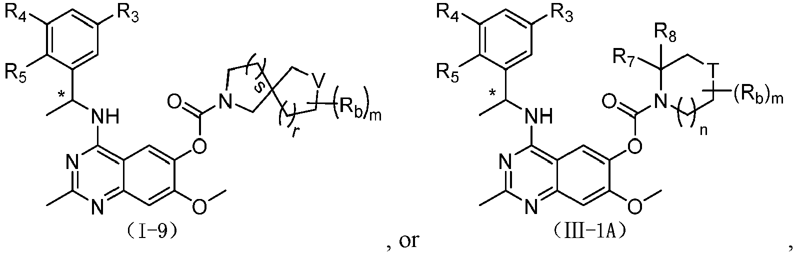
wherein T, V, W, Y, m, n, p, q, r, s, R₃, R₄, R₅, R_{b}, R₇, and R₈ are as defined in claim 23;
the carbon atom with "*" is a chiral carbon atom, which exists in an (*R*) or (*S*) single enantiomer form or an (*R*) or (*S*) single enantiomer-rich form.

27. The compound or the pharmaceutically acceptable salt thereof according to claim 26, wherein the compound has a structure of formula (I-10), (I-11), (I-12), or (III-2): 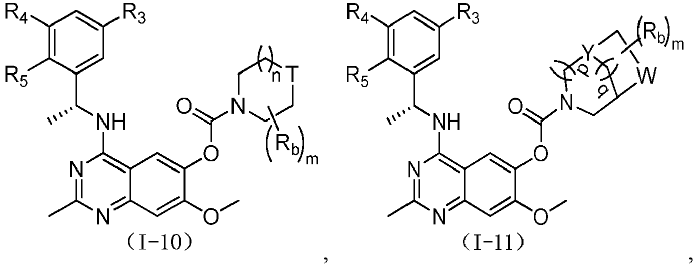 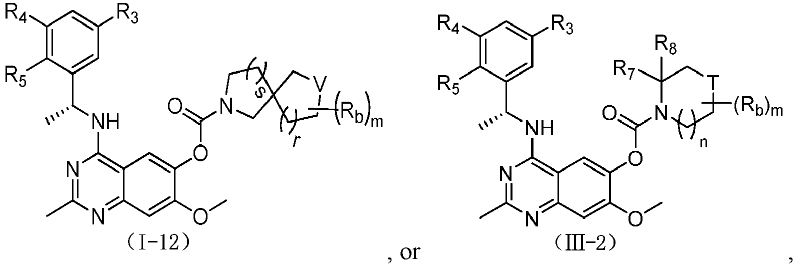 wherein T, V, W, Y, m, n, p, q, r, s, R₃, R₄, R₅, R_{b}, R₇, and R₈ are as defined in claim 26.

28. A compound of the following formula or a pharmaceutically acceptable salt thereof,

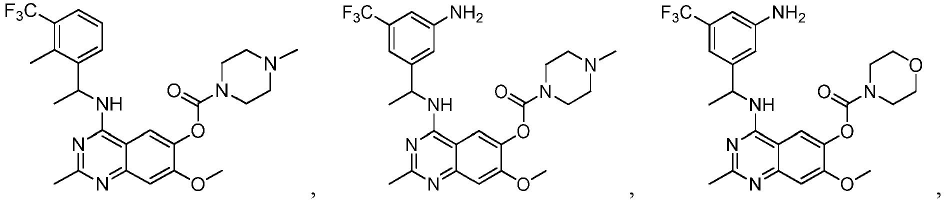

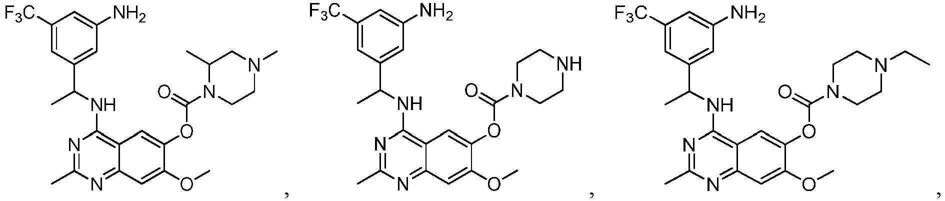

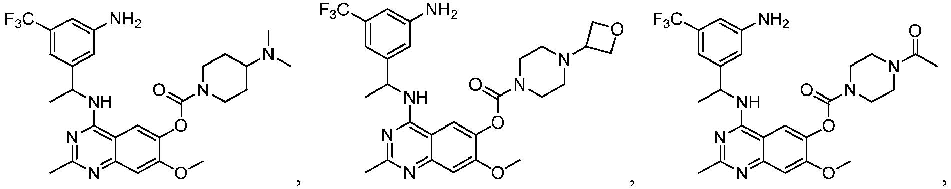

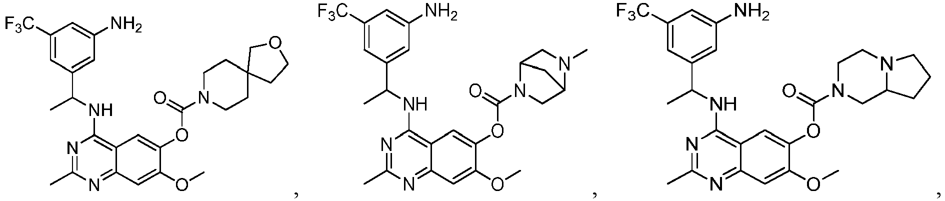

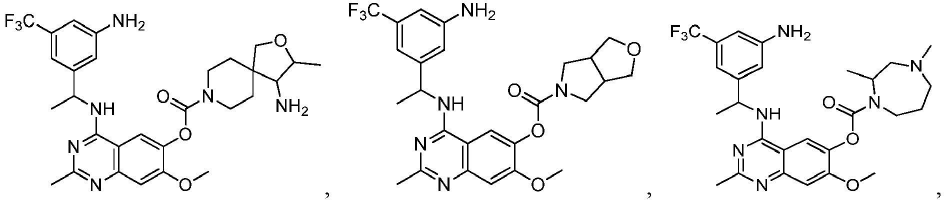

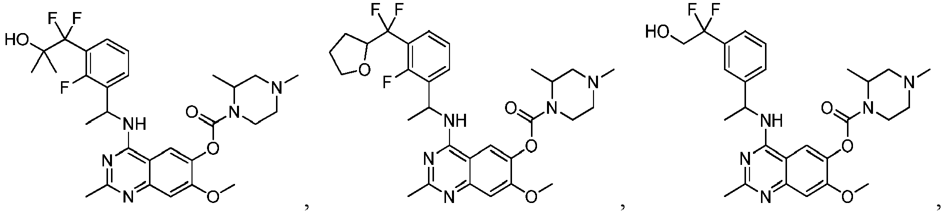

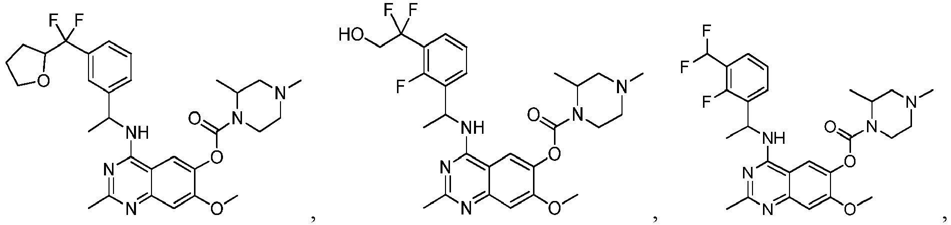

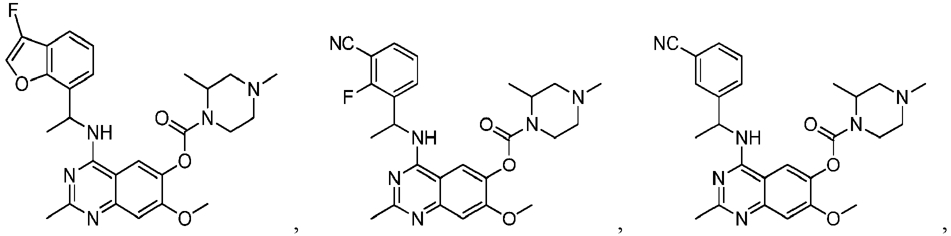

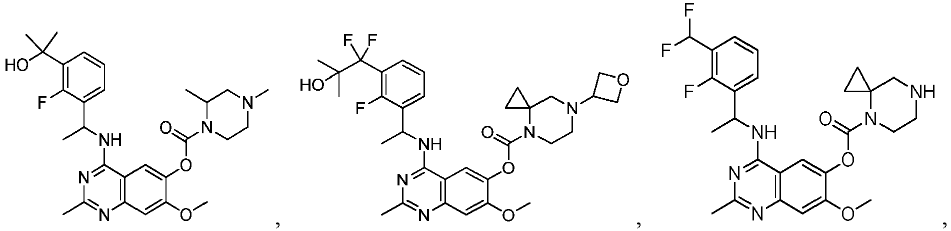

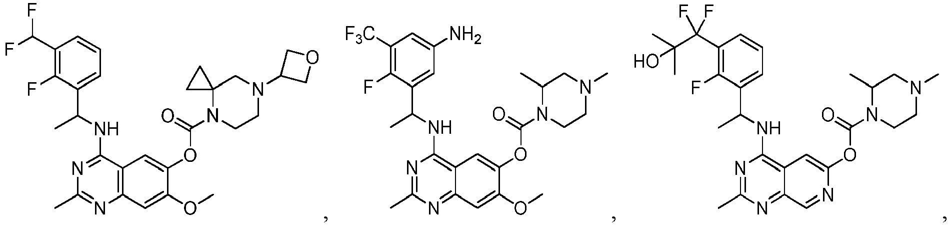

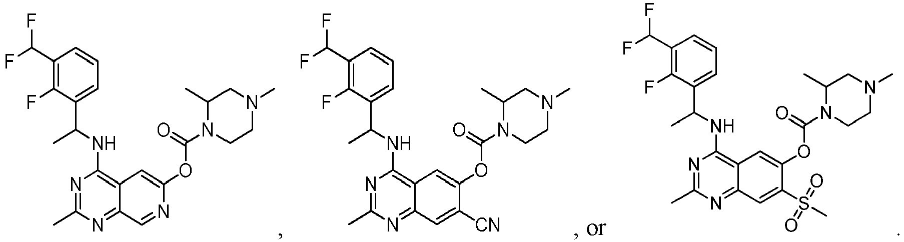

29. The compound or the pharmaceutically acceptable salt thereof according to claim 28, wherein the compound is:

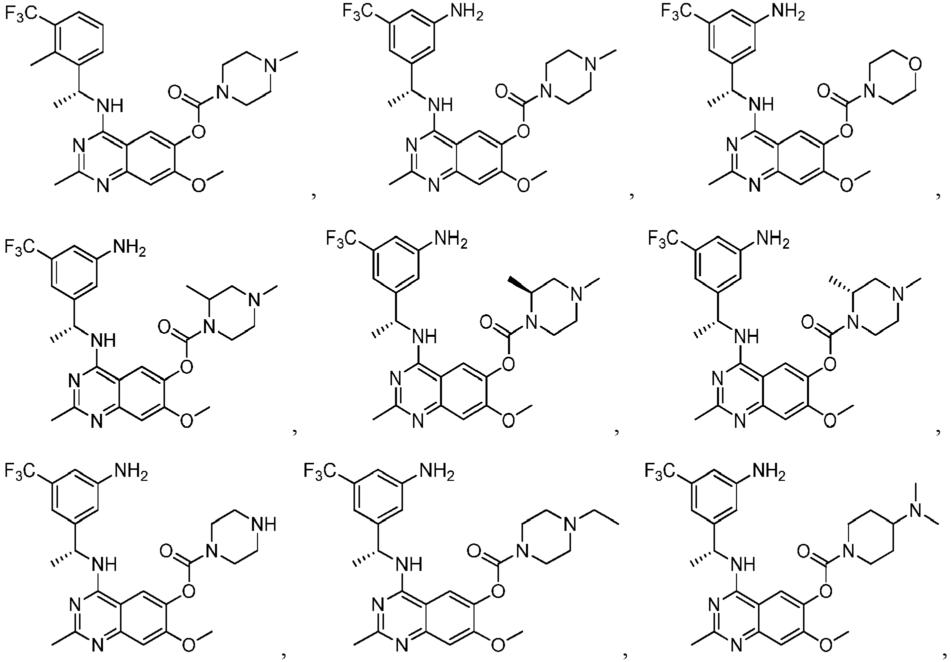

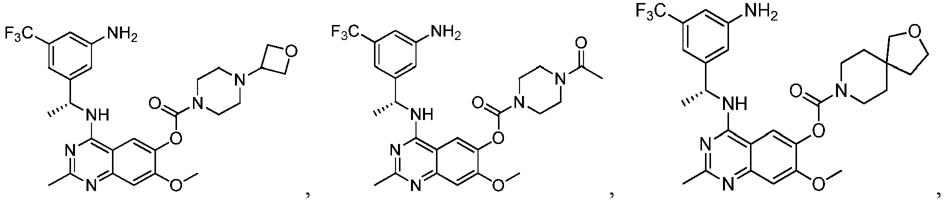

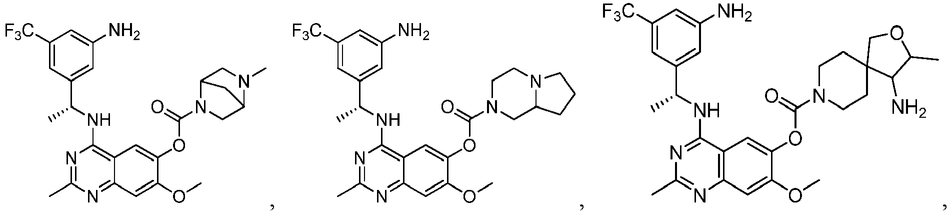

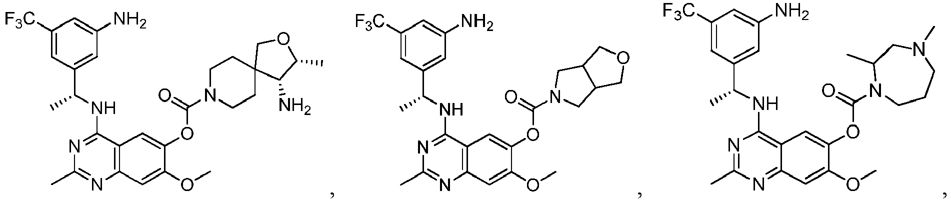

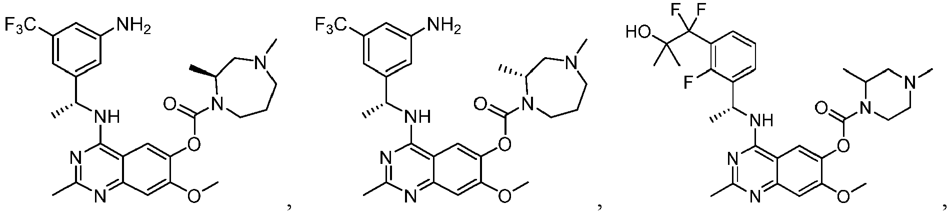

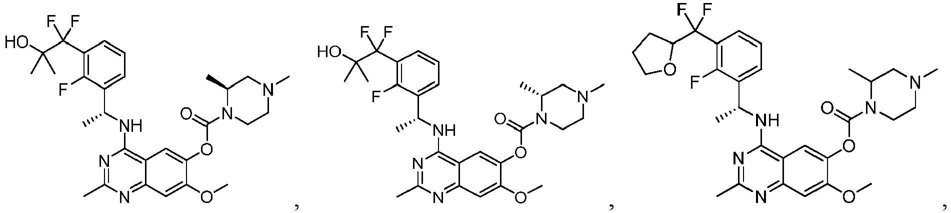

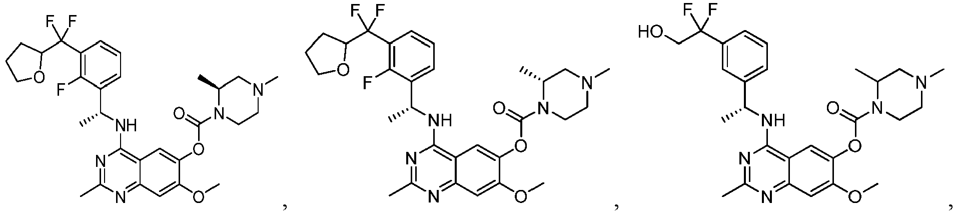

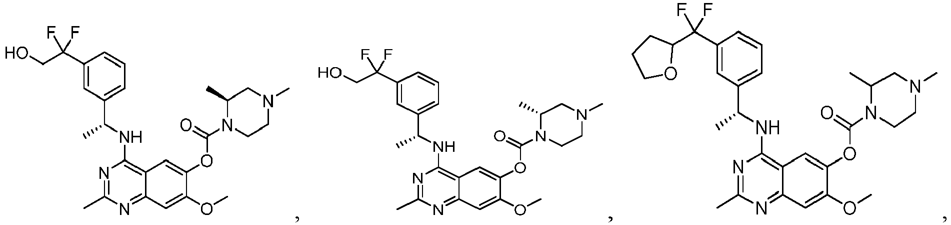

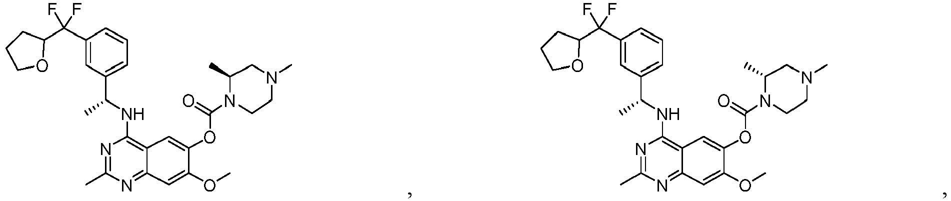

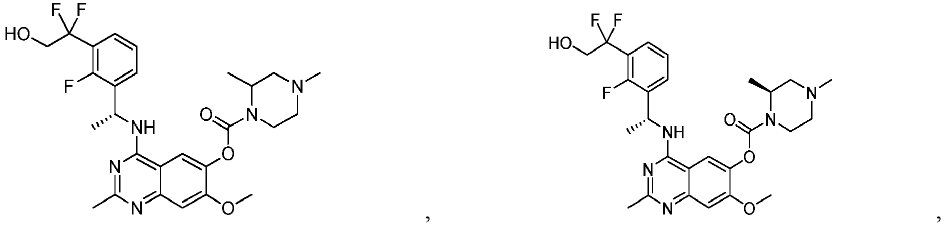

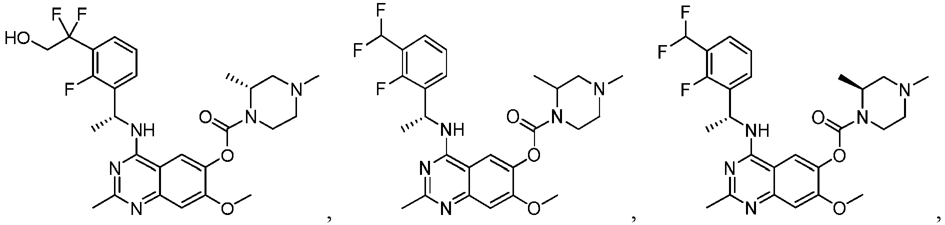

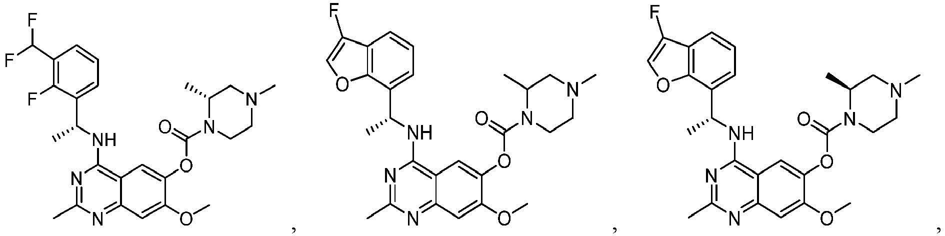

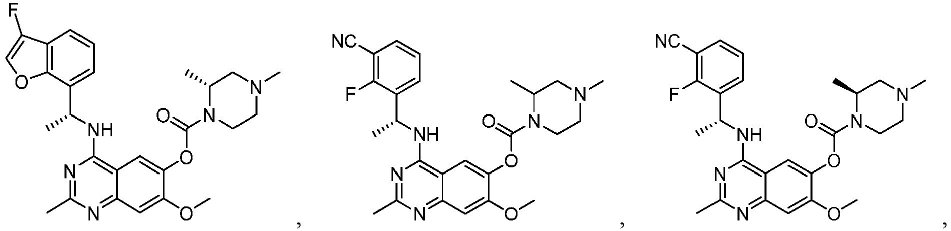

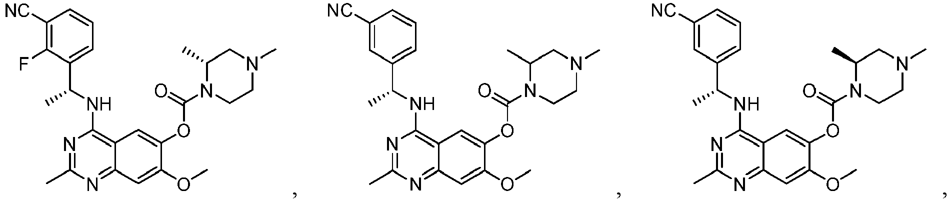

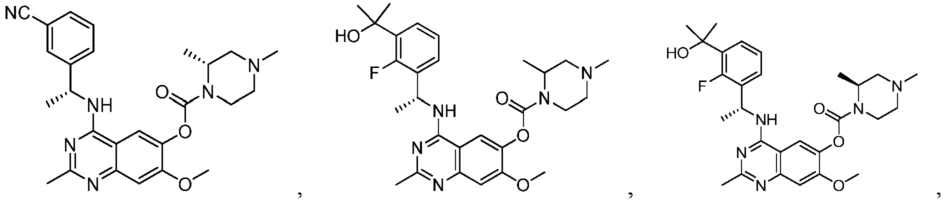

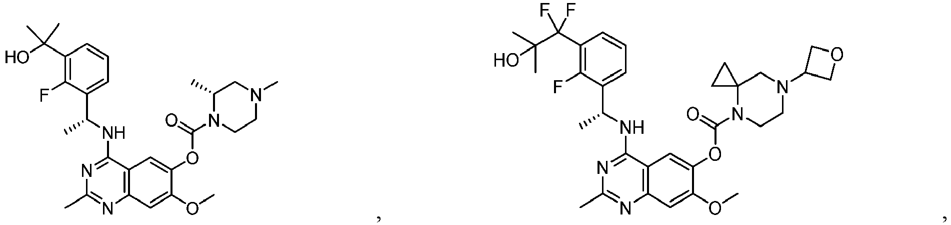

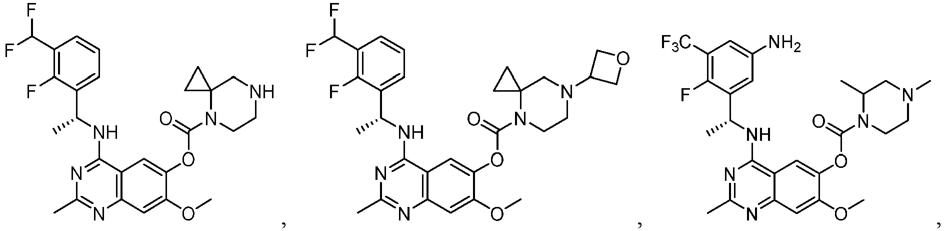

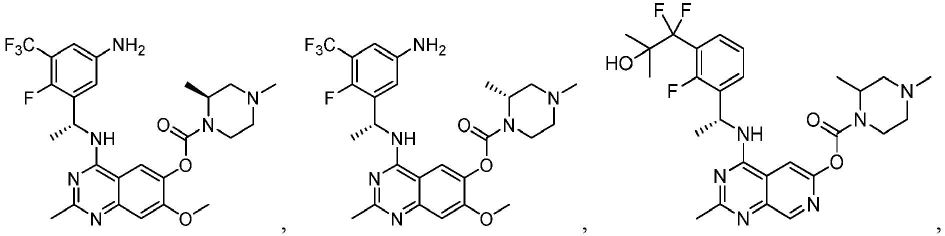

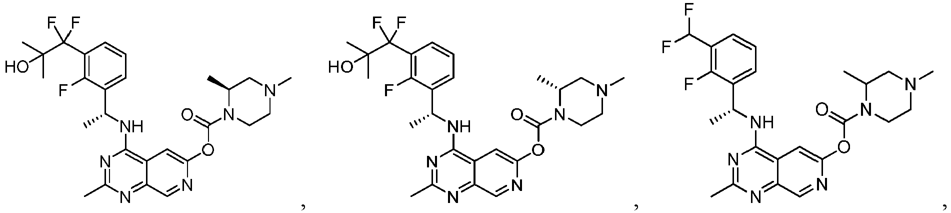

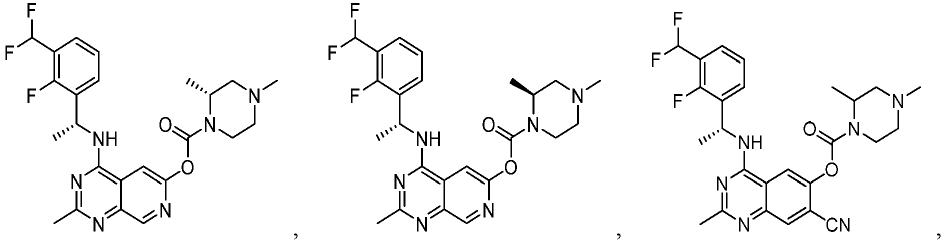

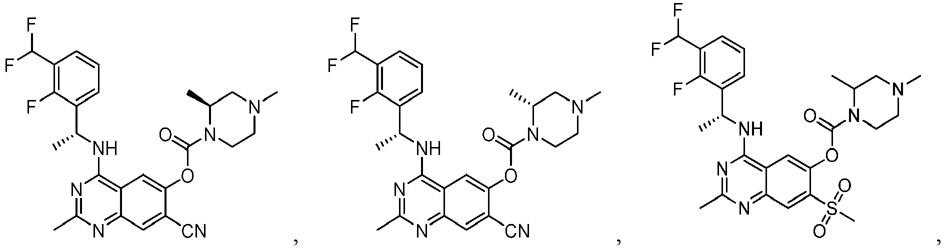

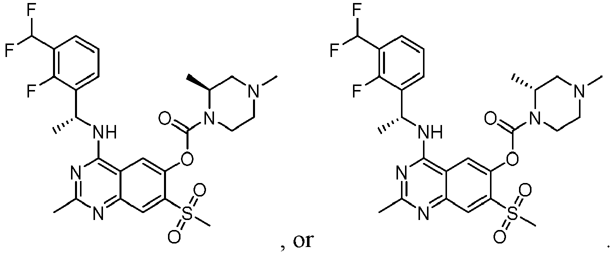

30. The compound or the pharmaceutically acceptable salt thereof according to any one of claims 1 to 29, wherein the pharmaceutically acceptable salt is hydrochloride.

31. A use of the compound or the pharmaceutically acceptable salt thereof according to any one of claims 1 to 29 in the manufacture of a medicament for treating KRAS mutant solid tumor diseases.
